# EUROPEAN PATENT APPLICATION

(11) **EP 1 911 765 A2**
(43) Date of publication of application: **16.04.2008**
(21) Application number: 07124051.9
(22) Date of filing: 18.07.2003
(51) Int. Cl.: C07K 14/47, A61K 39/00, C07K 16/44, G01N 33/50

(54) **Prevention, treatment and diagnosis of diseases associated with Beta-Amyloid formation and/or aggregation**

(30) Priority: 24.07.2002 EP 02447147; 06.08.2002 US 401497 P
(62) Divisional of application: 03766218.6
(71) Applicant: Innogenetics N.V., 9052 Gent (BE)
(72) Inventor: Delacourte, André, F-59155 Faches Thumesnil (FR); Sergeant, Nicolas, F-59790 Ronchin (FR)
(74) Representative: Grosset-Fournier, Chantal Catherine

(57) **Abstract**

The invention provides compositions and methods for prevention and treatment of diseases associated with β-amyloid formation and/or aggregation. Such methods encompass the induction of an immune response against N-terminal truncated and/or post-translationally modified Aβ peptides. These peptides are further used in compositions and methods for the diagnosis of diseases associated with β-amyloid formation and/or aggregation.

## Description

### FIELD OF THE INVENTION

The present invention relates to the prevention, treatment and diagnosis of diseases associated with β-amyloid formation and/or aggregation. More particularly, the present invention provides new β-amyloid (Aβ) and amyloid precursor protein (APP) peptides and new antibodies recognizing said Aβ and APP peptides for use in the prevention, treatment and diagnosis of diseases associated with β-amyloid formation and/or aggregation.

### BACKGROUND ART

Amyloidosis refers to a pathological condition in a mammal characterized by the presence of amyloid fibers. Amyloid is a generic term referring to a group of diverse but specific protein deposits. All amyloid deposits have common morphologic properties, stain with specific dyes (e.g. Congo red), and have a characteristic red-green birefringent appearance in polarized light after staining. Different amyloids are also characterized by the type of protein present in the deposit. For example, neurodegenerative diseases such as scrapie, bovine spongiform encephalitis, Creutzfeldt-Jakob disease and the like are characterized by the appearance and accumulation of a protease-resistant form of prion protein (referred to as AScr or PrP-27) in the central nervous system. Similarly, Alzheimer's disease, another neurdegenerative disorder, is characterized by neuritic plaques and neurofibrillary tangles. In this case, the plaque and blood vessel amyloid is formed by the deposition of fibrillar β-amyloid protein.

Alzheimer's disease (AD) is the most common type of senile dementia and is believed to be responsible for 40-60% of all cases of dementia. The incidence of AD increases with age, affecting 1 out of 10 persons older than age 65 and nearly 1 out of 2 persons older than age 85. Overall, the natural history of the disease can be characterized as an irreversibly progressive brain disorder that ultimately results in devastating memory loss, profound behavioral and personality changes, and severely damaged cognitive abilities. These impairments are related to the underlying death of brain cells and the breakdown of communication between them. In view of the large expenses for health care systems that must provide institutional and ancillary care for the AD patients, the impact of AD on society and on national economies is enormous.

Two major types of histological lesions are observed in AD brains, in association with neuronal loss (Felician and Sandson, 1999): (i) at the intracellular level, the neuronal cytoskeleton in AD patients is progressively disrupted and replaced by neurofibrillary tangles (NFTs) composed of paired helical filaments (PHF); (ii) at the extracellular level, amyloid plaques are formed by deposits of fibrillary β-amyloid (Aβ).

The microtubule-associated protein tau is a major protein component of paired helical filaments (PHF) and neurofibrillar tangles (NFTs), associated with Alzheimer's disease (Brion et al., 1985; Delacourte and Defossez, 1986; Grundke-Iqbal et al., 1986; Kosik et al., 1986; Wood et al., 1986; Kondo et al., 1988). Ten stages of tau pathology (S0-S10) were defined, according to ten brain areas that are successively affected (Delacourte et al., 1999).

Aβ is a major component of the senile plaques. Aβ is a small peptide found mainly in two sizes, consisting of 40 (Aβ₄₀) and 42 (Aβ₄₂) amino acids respectively, and in minor amounts in other sizes. Aβ is known to be metabolised from the proteolytic cleavage of APP (Saido, 2000), a large transmembrane protein with known, although not completely clear, neurotrophic functions (Seo et al., 2001). APP can be cleaved via two main routes, a major non-amyloidogenic route and a minor second, amyloidogenic route that yields Aβ as ultimate product.

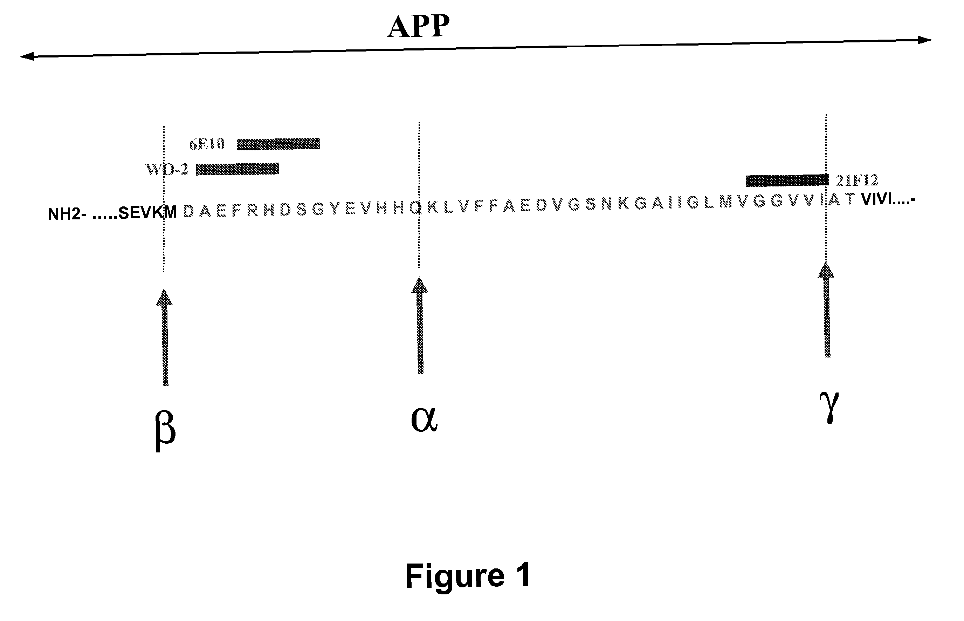

The main pathway for catabolism of APP is through cleavage by α-secretase at a single site in APP near the center of the β-amyloid peptide region (Esch et al., 1990; Sisodia, 1992). The products yielded by this route are a large N-terminal region of APP (APPsα) and a membrane associated C-terminal fragment (C83), which is subsequently hydrolysed by γ-secretase to yield the nearly unknown small p3 peptide. This is the non-amyloidogenic route because the cleavage site is located approximately in the middle of the Aβ sequence, with no possibility of Aβ formation. The second APP processing pathway is the N- and C-terminal cleavage of APP by β-and γ-secretase (Fig. 1). The resulting molecules of these two proteolytic steps are the central fragments of APP, Aβ₄₀ and Aβ₄₂, Aβ₄₀ being the more abundant of the whole Aβ formed (Conde, 2002). β-secretase cleaves at the amino terminus of the β-amyloid peptide and occurs first, followed by γ-secretase, which releases the carboxy terminus of the peptide. This statement is based upon the observation that C-terminal fragments produced by β-secretase cleavage are readily apparent in cells, whereas APP fragments corresponding to a single C-terminal γ cleavage are not (Haass et al., 1992; Seubert et al., 1992).

Molecular heterogeneity of APP processing that generates Aβ peptides results, amongst others, from different types of mutations in familial autosomal dominant Alzheimer's disease (FAD AD), located near the β or the γ cleavage sites (De Strooper and Annaert, 2000). These different pathogenic mutations can be modeled in transgenic mice (Chen et al., 2000). However, AD is essentially non-familial (non-FAD AD), this form including more than 99% of all patients, according to a large scale population study (Campion et al., 1999). An overexpression of Aβ or a change in the ratio Aβ₄₂/Aβ₄₀ is well demonstrated in FAD AD, but not in non-FAD AD, in which amyloidosis is explained by a lack of Aβ clearance or an increase of fibrillogenesis.

In the human brain, amyloidosis is observed first as diffuse aggregates of Aβ₄₂ peptides, which accumulate progressively as amyloid plaques, followed by the deposition of Aβ₄₀ peptides (Delacourte et al. 2001; 2002). A microglial cell proteolysis of Aβ₄₂ into Aβ₄₀ species has also been suggested (Fukumoto et al., 1996).

The latter peptides are also observed in large quantities in the cerebral vessel walls, to constitute amyloid angiopathy, which is found in variable amounts in AD brains (Barelli et al., 1997; Wisniewski et al., 1997). Senile plaques consist largely of insoluble Aβ surrounded by a variety of neuronal and glial processes. This amorphous, acellular material is found in the spaces between the brain's nerve cells.

Both tau pathology and Aβ aggregation should be used as neuropathological diagnostic criteria for AD (Hyman and Trojanowski, 1997). Clinical AD is diagnosed in patients with tau pathology in the frontal pole and the parietal cortex (stages 7 to 10 according to Delacourte et al., 1999) and the presence of Aβ₄₂ aggregates above 50 µg/gram of wet tissue in these corical areas. Infraclinical AD is diagnosed in non-demented patients or patients with mild cognitive impairment, with insoluble Aβ₄₂ at a concentration of 10 µg/gram of tissue in neocortical areas, such as the frontal pole or the parietal cortex, and a tau pathology in the hippocampal area. In infraclinical AD patients, tau pathology can be asymptomatic up to stage 6. Non-demented patients can be considered as "pure controls" or "normal aging" (as far as tau and APP pathologies are concerned) if tau pathology is absent in all cortical areas, including the hippocampal area and if there is no trace of Aβ₄₂ aggregates in neocortical areas. If the patients are older than 75 years, very discrete or moderate tau pathology is likely to be found in the hippocampal area (stages 1 to 3), due to aging or a pathological process that remains to be determined. But these aged non-demented patients can be considered as controls as they have no detectable Aβ₄₂ aggregates (Delacourte et al., 1999; 2001).

A very significant effort is underway to test a large number of therapeutic options for AD. These approaches include numerous agents such as acetycholinesterase inhibitors, nonsteroidal anti-inflammatory drugs (NSAIDS), estrogen, neurotrophic agents, and even vitamins (Sramek and Cutler, 2000; Thal, 2000). Three general sub-approaches have arisen with the ultimate goal of limiting the presence of Aβ, in order to slow, stop or reverse the progression of AD. These approaches aim at preventing the formation of deposition of Aβ and to clear the already formed Aβ. The first way may be undertaken by enhancing the activity of α-secretase or inhibiting β- and γ-secretases. Upregulation or inhibition of enzymes is, however, a delicate and uncertain task, certainly when their normal biological activity is not completely known. The second focus is avoiding the aggregation of the already formed Aβ into fibrils. In this approach, however, the possibility that aggregation or fibrillogenesis inhibitors may reverse the process must be considered (Klein et al., 2001). The third route, and currently the most promising strategy for the treatment of AD, is the immunization with Aβ or some suitable fragment thereof to obtain antibody-mediated clearance of Aβ. Various research teams have reported on the benefits of immunization of different lines of APP transgenic mice with Aβ. Immunization with Aβ prevented the development of β-amyloid plaque formation and removed existing plaques (Schenk et al., 1999; 2000). It further reduced the deposition of cerebral fibrillar Aβ, the cerebral Aβ burden and the cognitive dysfunction (Janus et al., 2000; Morgan et al., 2000; Weiner et al., 2000; Lemere et al., 2000a; Sigurdsson et al., 2001; Lemere et al., 2001). Even passive administration of antibodies alone was sufficient to reduce plaque pathology (Bard et al., 2000; Bacskai et al., 2001; DeMattos et al., 2001) and reverse memory impairment (Dodart et al., 2002). Anti-Aβ antibodies appear to bind to plaques and then, through what is likely an Fc-mediated phagocytosis process, the plaque material is taken up by microglia cells and hydrolyzed. Clearance of amyloid deposits in the brain of AD patients using vaccination against Aβ peptide is a novel approach that opens treatment perspectives (Schenk et al., 2001).

The above teachings, however, do not identify a specific chemical nature of the Aβ peptide to be employed in vaccination strategies. The above teachings are restricted to the use of specific naturally occurring forms of β-amyloid peptide or peptide complexes (i.e. Aβ₃₉, Aβ₄₀, Aβ₄₁, Aβ₄₂ or Aβ₄₃). However, Aβ is also a physiological product with an unknown function and vaccination with naturally occuring Aβ could generate an undesirable immune reaction. A clinical Phase II trial with AN-1792, a formulation containing a synthetic form of Aβ₄₂, had to be stopped because symptoms consistent with inflammation in the CNS were reported for 15-19 patients (Conde, 2002). Therefore, the efficiency of a therapeutic strategy relies on the precise knowledge of the chemical nature of the first amyloid deposits that seed fibrillogenesis and that are pathological, rather than physiological, in order to avoid autoimmune responses. Only a clear understanding of the precise chemical nature of the pathological Aβ species prone to aggregation and present in the very earliest deposits will enable the correct diagnosis of a person susceptible to or at risk of developing a disease associated with β-amyloid formation and/or aggregation such as AD and thus enable the subsequent prevention of Aβ aggregation or the subsequent removal of the amyloid burden by vaccination.

Since the discovery of β-amyloid (Aβ) as the major constituent of amyloid deposits in Alzheimer's disease (AD) (Glenner and Wong, 1984), amino-truncated Aβ peptides have been identified in plaques of AD patients (Masters et al., 1985). A modified Aβ peptide, starting with pyroglutamyl residue at position 3 of the Aβ sequence, was identified in Aβ deposits of mainly Down's syndrome patients by Saido et al. (1995; 1996) and Harigaya et al. (2000). N-terminal degradations were observed in Aβ peptide deposits from AD demented patients (Kalback et al., 2002). These studies, however, do not identify or differentiate between Aβ C-terminal variants (Aβ₃₉, Aβ₄₀, Aβ₄₁, Aβ₄₂ or Aβ₄₃). Furthermore, it is quite probable that these N-terminal truncations of Aβ peptides are the result of artefacts of experimental procedures used to extract the Aβ peptides of *in situ* amyloid deposits of AD patients (Masters et al., 1985; Kalback et al., 2002). They could also result from catabolism of amyloid deposits by a late stage proteolytic activity (generated by microglial cells for example) generated in AD brains (Kawarabayashi et al., 2001). N-terminal truncated Aβ peptides can also be produced by cell models transfected with a mutated amyloid precursor protein (APP) (Cescato et al., 2000) or when cells are treated with drugs such as bafilomycin A1 or ammonium chloride (Haass et al., 1995; Schrader-Fischer and Paganetti, 1996). Here again, the dramatic physiological dysfunction that is induced, triggers a proteolytic activity, by membrane associated proteases. In summary, the above data suggest that in individuals with fully developed AD, amino-truncated Aβ are components of the amyloid deposits. Their production could be a late event generated by microglial cells or by experimentally-induced proteolytic activities.

For diagnosis, prevention and treatment of amyloidosis, however, it is mandatory to know the specific chemical nature of Aβ peptides at the very early stages of amyloid deposition, before ongoing microglial or astrocytic reactions generate proteolytic activity. At present, no data are available on the specific structure of pathological Aβ peptides that are prone to aggregation and/or that are present in amyloid deposits that seed fibrillogenesis, and that are not yet subject to proteolysis mediated by microglial and astrocyte activity.

### SUMMARY OF THE INVENTION

The present invention provides Aβ peptides with a very specific chemical nature, i.e. N-terminal truncated and/or post-translationally modified Aβ peptides that were identified as the pathological Aβ peptides in amyloid dimers that seed fibrillogenesis at the very early stages of AD. Those Aβ peptides could be used for the induction of an immune response in methods for preventing and treating diseases characterized by β-amyloid formation and/or aggregation such as Alzheimer's disease. The invention thus relates to a preparation comprising a β-amyloid variant or an N-terminal fragment thereof characterized in that said β-amyloid variant or said N-terminal fragment thereof contains an N-terminal truncation and/or a post-translational modification.
In a preferred embodiment, the present invention provides a preparation comprising a N-terminal truncated and/or post-translationally modified β-amyloid variant or an N-terminal fragment thereof, further characterized in that said N-terminal truncated β-amyloid variant or said N-terminal fragment thereof starts at position 2, 3, 4, 5, 6, 7, 8, 9, or 10 of β-amyloid and that the post-translational modification is a methylation or a pyroglutamylation.
In another preferred embodiment, the present invention provides a preparation comprising an N-terminal truncated and/or post-translationally modified β-amyloid variant or an N-terminal fragment thereof, further characterized in that the pyroglutamylation is present at position 3 of an N-terminal truncated β-amyloid variant starting at position 3 of β-amyloid.
In another preferred embodiment, the invention provides a preparation comprising an N-terminal truncated and/or post-translationally modified β-amyloid variant or an N-terminal fragment thereof, further characterized in that the β-amyloid variant or the N-terminal fragment thereof comprises an amino acid sequence selected from the group consisting of SEQ ID NOs 1 to 165.
In another preferred embodiment of the invention, the present invention provides a preparation comprising an N-terminal truncated and/or post-translationally modified β-amyloid variant or an N-terminal fragments thereof, further characterized in that the N-terminal truncated and/or post-translationally modified β-amyloid variant or the N-terminal fragment thereof contains an additional modification resulting in a separated spot of the N-terminal truncated and/or post-translationally modified β-amyloid variant or the N-terminal fragment thereof on two-dimensional gel electrophoresis compared to the spot obtained with said N-terminal truncated and/or post-translationally modified β-amyloid variant or the N-terminal fragment thereof without said additional modification.

The finding of said N-terminal truncated and/or post-translationally modified Aβ variants, which are a result of an aberrant pathway of APP metabolism, supports the presence of N-terminal APP soluble fragments containing extra amino acids at the C-terminal tail, resulting from aberrant cleavage by β-like secretase (i.e. behind amino acid 1, 2, 3, 4, 5, 6, 7, 8, or 9 of Aβ). Accordingly, the invention also relates to a preparation comprising an N-terminal APP soluble fragment obtainable by secretase cleavage of APP, characterized in that the C-terminal end of said N-terminal APP soluble fragment consists of position 1, or 1 to 2, 1 to 3, 1 to 4, 1 to 5, 1 to 6, 1 to 7, 1 to 8, or 1 to 9 of β-amyloid. The invention also relates to a C-terminal fragment of such an N-terminal APP soluble fragment.
In a preferred embodiment, the present invention relates to a preparation comprising an N-terminal APP soluble fragment obtainable by secretase cleavage of APP, or a C-terminal fragment thereof, further characterized in that said N-terminal APP soluble fragment or said C-terminal fragment thereof comprises an amino acid sequence selected from the group consisting of SEQ ID NOs 1 to 6, 14 to 18, 27 to 30, 53 to 55, 66 to 67, 79, and 166 to 261.

The invention further relates to a nucleic acid preparation comprising a nucleic acid sequence capable of encoding the N-terminal truncated and/or post-translationally modified β-amyloid variant of the invention or the N-terminal fragment thereof.
The invention also relates to a nucleic acid preparation comprising a nucleic acid sequence capable of encoding the N-terminal APP soluble fragment of the invention or the C-terminal fragment thereof.

The invention further relates to a method for the preparation of an antibody that specifically recognizes an N-terminally truncated and/or post-translationally modified β-amyloid variant, comprising the following steps:
(a) Immunizing an animal with a preparation comprising an N-terminal truncated and/or post-translationally modified β-amyloid variant or an N-terminal fragment thereof or with a nucleic acid preparation encoding an N-terminal truncated and/or post-translationally modified β-amyloid variant or an N-terminal fragment thereof;
(b) Obtaining the antibodies generated by the immunization in step (a);
(c) Screening the antibodies obtained in step (b) for their specific recognition of the N-terminal truncated and/or post-translationally modified β-amyloid variant.
The invention also relates to an antibody obtainable by the above method.
The invention further also relates to a method for the preparation of an antibody that specifically recognizes an N-terminal APP soluble fragment of the invention, comprising the following steps:
(a) Immunizing an animal with a preparation of N-terminal APP soluble fragment of the invention or a C-terminal fragment thereof or with a nucleic acid preparation encoding an N-terminal APP soluble fragment of the invention or a C-terminal fragment thereof;
(b) Obtaining the antibodies generated by the immunization in step (a);
(c) Screening the antibodies obtained in step (b) for their specific recognition of the N-terminal APP soluble fragment of the invention.
The invention also relates to an antibody obtainable by the above method.

As indicated above, the present invention provides preparations and methods for preventing and treating, in a mammal, a disease characterized by β-amyloid formation and/or aggregation. In accordance, the invention also relates to a vaccine composition or a therapeutic composition comprising an N-terminal truncated and/or post-translationally modified β-amyloid variant or an N-terminal fragment thereof, comprising an antibody recognizing said N-terminal truncated and/or post-translationally modified β-amyloid variant, or comprising a nucleic acid encoding said N-terminal truncated and/or post-translationally modified β-amyloid variant or said N-terminal fragment thereof.
The invention thus also relates to a method for the prevention and/or treatment, in a mammal, of a disease associated with β-amyloid formation and/or aggregation such as Alzheimer's disease, said method comprising the administration, to said mammal, of a vaccine composition or a therapeutic composition of the invention.

Another aspect of the present invention relates to a diagnostic or theranostic kit comprising a preparation of N-terminal truncated and/or post-translationally modified β-amyloid variant of the invention or an N-terminal fragment thereof, comprising a preparation of an N-terminal APP soluble fragment of the invention or a C-terminal fragment thereof, or comprising an antibody recognizing said peptide or fragments.
The invention also relates to a method for the measurement, in a mammal, of the immune response induced by vaccination or therapeutic application with a vaccine composition or a therapeutic composition of the invention, said method comprising the following steps:
(a) Determining, in a sample obtained from said mammal, the amount of antibody specific for an N-terminal truncated and/or post-translationally modified β-amyloid variant of the invention;
(b) Comparing the amount determined in step (a) with the amount of antibody specific for said N-terminal truncated and/or post-translationally modified β-amyloid variant present in the mammal before vaccination or therapeutic application with the vaccine or therapeutic composition of the invention;
(c) Concluding, from the comparison in step (b), whether the mammal is responding to the vaccination or therapy, an increased amount of antibody specific for said N-terminal truncated and/or post-translationally modified β-amyloid variant being an indication that the mammal is responding to the vaccination or therapy.
The invention further relates to a method for determining, in a mammal, the susceptibility to a disease associated with β-amyloid formation and/or aggregation such as Alzheimer's disease, for determining, in a mammal, the risk of developing a disease associated with β-amyloid formation and/or aggregation such as Alzheimer's disease, for screening of the clearance of β-amyloid deposition in a mammal, or for predicting the level of β-amyloid burden in a mammal, said method comprising the following steps:
(a) Determining, in said mammal, the amount of N-terminal truncated and/or post-translationally modified β-amyloid variant of the invention, the amount of N-terminal APP soluble fragment of the invention, or the amount of antibody specific for said β-amyloid variant or said N-terminal APP soluble fragment of the invention;
(b) Comparing the amount determined in step (a) with the amount of said N-terminal truncated and/or post-translationally modified β-amyloid variant, said N-terminal APP soluble fragment or said antibody in a control mammal;
(c) Concluding, from the comparison in step (b), whether the mammal is susceptible to a disease associated with β-amyloid formation and/or aggregation such as Alzheimer's disease, whether the mammal is at risk of developing a disease associated with β-amyloid formation and/or aggregation such as Alzheimer's disease, whether the β-amyloid deposition in the mammal is cleared, or what the level of β-amyloid burden is in said mammal.
In a preferred embodiment, the present invention relates to a method as above, further characterized in that the amount of N-terminal truncated and/or post-translationally modified β-amyloid variant, of N-terminal APP soluble fragment or the amount of antibody specific for said β-amyloid variant or said APP soluble fragment is determined on a tissue sample taken from said mammal.
In a preferred embodiment, the present invention also relates to a method for predicting the level of β-amyloid burden in a mammal, said method comprising the following steps:
(a) Administration, to said mammal, of a vaccine composition or a therapeutic composition of the invention;
(b) Determining, in a biological fluid sample obtained from said mammal, the amount of N-terminal truncated and/or post-translationally modified β-amyloid variant of the invention;
(c) Comparing the amount determined in step (b) with the amount of said N-terminal truncated and/or post-translationally modified β-amyloid variant in a biological fluid sample obtained from a control mammal;
(d) Concluding, from the comparison in step (c), what the level of β-amyloid burden is in said mammal.

### FIGURE LEGENDS

**Figure 1****.** Partial amino acid sequence of APP770, displaying the amino acid sequence of Aβ with the α-, β-, and γ-secretase cleavage sites indicated.

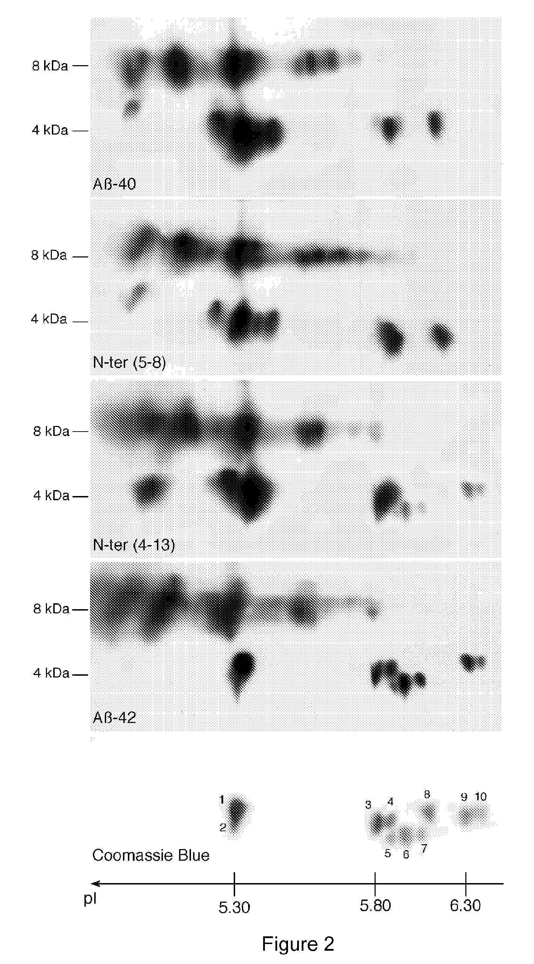

**Figure 2****. Two-dimensional electrophoretic analysis of Aβ species present in brain obtained from Alzheimer's disease patients.** Aβ aggregates solubilized with formic acid were resolved by 2-D gel electrophoresis. Aβ monomers (4 kDa) and dimers (8 kDa) were labelled with antibodies WO2 and 6E10 against the amino-terminal region of Aβ (N-ter(5-8) and N-ter(4-13) panels, respectively). The carboxy-terminal tails of Aβ₄₂ and Aβ₄₀ were detected with 21F12 and ADA40, respectively (Aβ-42 and Aβ-40 panels). The major Aβ species recovered with our extraction method were stained with Coomassie Blue, and 10 spots were subsequently analyzed by mass spectrometry (Table 3). The results presented were obtained from the AD patient showing the largest quantity of amyloid deposits. The isoelectric points (pI) and the Aβ spots used for mass spectrometric analysis are indicated. Note that dimeric species of Aβ are not stained by Coomassie Blue.

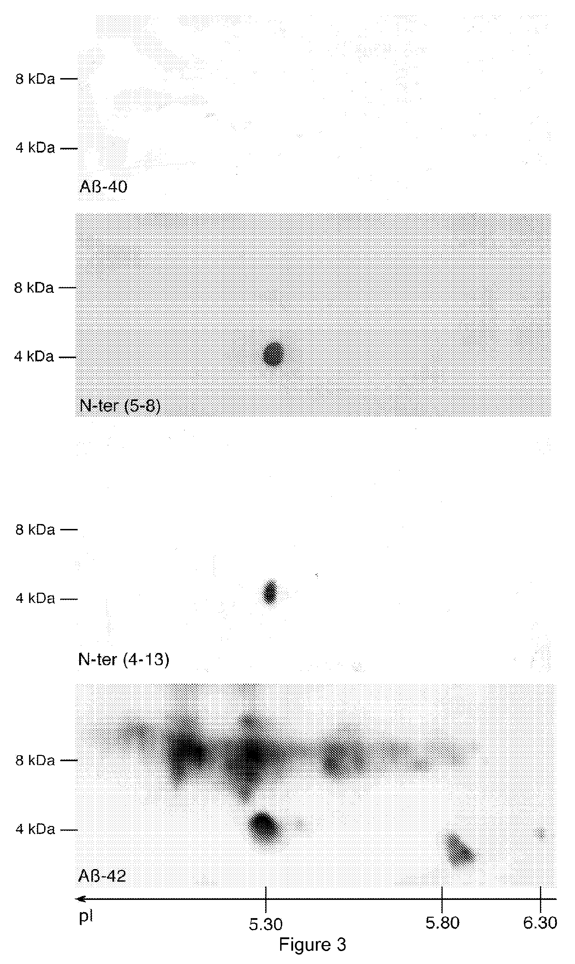

**Figure 3****. Aggregated Aβ species present in brain obtained from infraclinical AD patients.** Formic acid solubilized Aβ species derived from the brain tissue of non-demented patients were resolved by 2-D gel electrophoresis. Aβ₄₀ species are not detected with our ADA40 antiserum. 21F12 detects both Aβ₄₂ monomers (4 kDa staining) and dimers (8 kDa staining). Both WO2 (panel N-ter (5-8)) and 6E10 (panel N-ter (4-13)) antibodies stained a single Aβ peptide spot with pI 5.30.

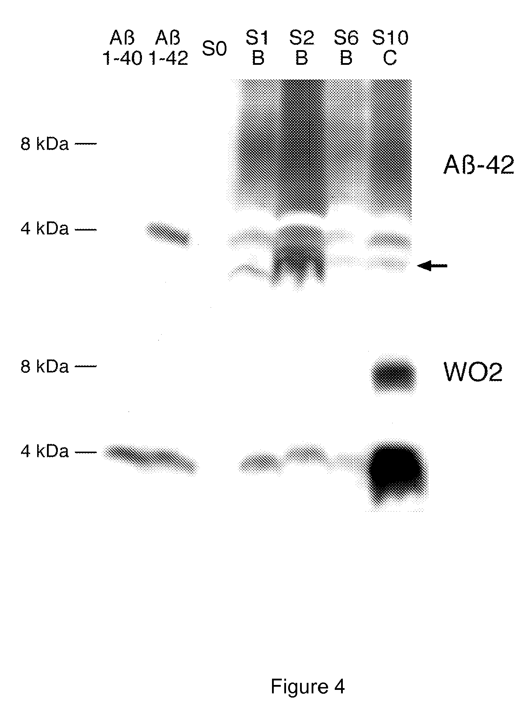

**Figure 4****. Dimers of Aβ in infraclinical AD are essentially composed of amino-truncated Aβ peptides.** The brain tissue of a control individual (S0), three non-demented cases (S1, S2, and S6) and one AD case (S10) were lysed in formic acid. According to the nomenclatures defined by Delacourte et al. (2002) and Braak and Braak (1991), the stages of tau pathology (S0 to S10) and the amyloid staging classification (B or C) are indicated at the top of the lanes, respectively. Ten ng of Aβ peptides 1-40 and 1-42 were loaded in parallel (first and second lane). Aβ x-42 was identified with 21F12 (panel Aβ-42) and the amino-terminal region with WO2 (WO2 panel). Molecular weights are indicated on the left, and an arrow indicates the amino-truncated variants labelled by 21F12. Note that the same AD case was used for both 2-D electrophoretic analysis and mass spectrometry.

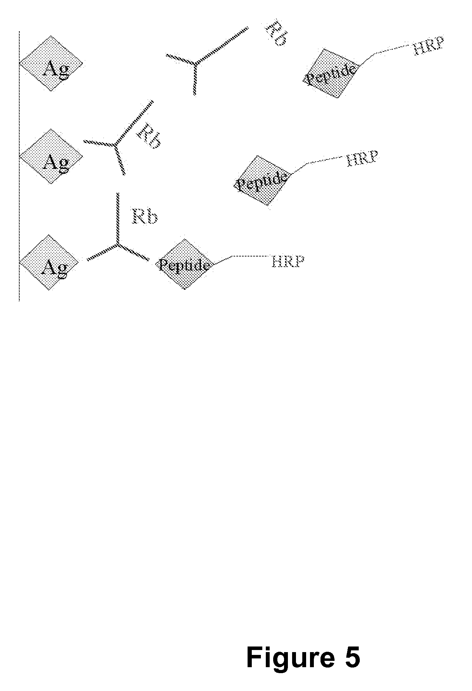

**Figure 5****.** Principle of the bridging assay as used in Example 2.

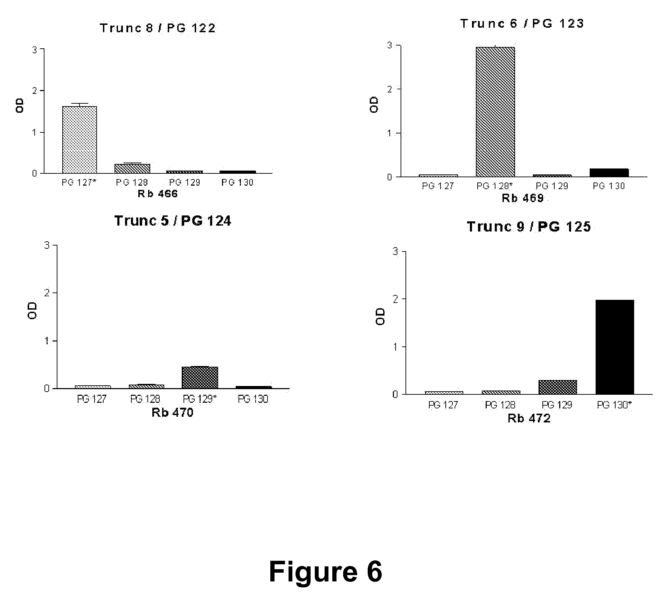

**Figure 6****.** Analysis of the specifity of the antibodies by use of the bridging assay. The different N-terminally truncated amyloid peptides were used for coating, and specific HRP-peptide conjugates were used for detection. The antibodies were generated as described in Example 2.

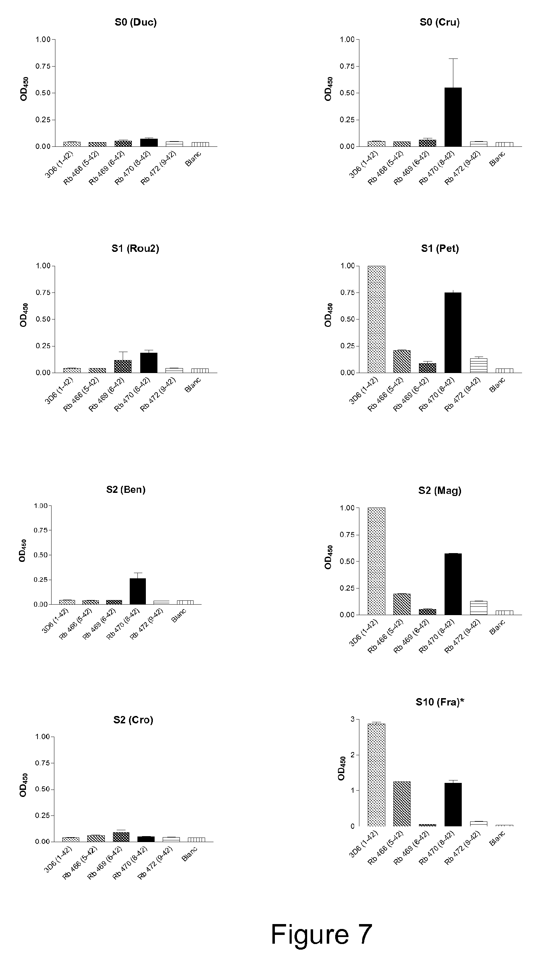

**Figure 7****. Analysis of the N-terminally truncated Aβ peptides present in human brain extract.** OD450 values are shown for a 1/20 dilution of a formic acid extract of several stages of Alzheimer pathology. Peptides were captured on a plate with 21F12 (plate from kit K-1080, Innogenetics, Ghent, Belgium) and detected with biotinylated 3D6 for 1-42, and by a bridging assay for the different antisera specific for truncated amyloid.

### DETAILED DESCRIPTION OF THE INVENTION

### N-terminal truncated and/or post-translationally modified Aβ peptides

The present invention provides compositions and methods for preventing and treating diseases characterized by β-amyloid formation and/or aggregation such as Alzheimer's disease. The methods of the invention encompass the induction of an immune response against Aβ peptides with a very specific chemical nature, i.e. N-terminal truncated and/or post-translationally modified Aβ peptides. N-terminal truncated and/or post-translationally modified Aβ variants are the aggregated species of Aβ that seed amyloid deposition at the infraclinical stages of Alzheimer pathology. They were identified, for the first time, in the brain tissue of non-demented individuals with both neurofibrillary degeneration and amyloid deposition at low levels, while they were not found in "pure control" individuals without neurofibrillary degeneration or amyloid deposition. Hence, these N-terminal truncated and/or post-translationally modified Aβ variants correspond to the infraclinical stages of AD (i.e. the earliest possible stages I and II of AD pathology, as described by Braak and Braak, 1991 or the Tau pathology stages 1 to 6 according to Delacourte et al., 1999) and form the seeds of amyloid deposition. These N-terminal truncated and/or post-translationally modified Aβ variants were observed in the brain tissue not yet affected by microglial or astrocyte reaction that generate proteolytic activities. This indicates that these N-terminal truncated and/or post-translationally modified Aβ variants are not the result of late stage proteolytic activity but that they should be in situ products, resulting from an aberrant pathway of APP metabolism. These N-terminal truncated and/or post-translationally modified Aβ variants can thus be considered as the very first pathological species related to the early events of amyloidosis.

Aβ peptide, Aβ, β-amyloid peptide, or A4 peptide are used interchangeably throughout the present invention and refer to a peptide of 39-43 amino acids (Aβ₃₉, Aβ₄₀, Aβ₄₁, Aβ₄₂ or Aβ₄₃), which is the principal component of characteristic plaques of Alzheimer's disease. Aβ is generated by processing of a larger protein APP by two enzymes, termed β- and γ-secretases (Haass et al. 1992; Seubert et al. 1992). The sequence of the Aβ₄₂ peptide is the following:
DAEFRHDSGYEVHHQKLVFFAEDVGSNKGAIIGLMVGGVVIA (SEQ ID NO 144)

Aβ₄₁, Aβ₄₀ and Aβ₃₉ differ from Aβ₄₂ by the omission of Ala (A), Ile-Ala (IA), and Val-Ile-Ala (VIA) respectively from the C-terminal end. Aβ₄₃ differs from Aβ₄₂ by the presence of a threonine residue at the C-terminus.

Diseases associated with β-amyloid formation and/or aggregation are diseases in which the β-amyloid is formed in the brain as a result of β- and γ-cleavage of APP and/or in which said β-amyloid forms oligomers. Diseases associated with β-amyloid formation and/or aggregation include but are not limited to dementia with Lewy bodies (DLB) with amyloid, Down's syndrome, Alzheimer's disease, both late and early onset.

Disaggregated or monomeric Aβ, as used in the present invention, means soluble, monomeric peptide units of Aβ. Aggregated Aβ is a mixture of oligomers in which the monomeric units are held together by non-covalent bounds. "Disaggregating" refers to solubilization of aggregated proteins typically held together by non-covalent bounds. β-amyloid deposits comprise Aβ peptides in aggregated form such as formed in the brain of AD patients. In most cases, the aggregate has a β-pleated sheet structure. The present invention demonstrates that Aβ dimers, the very first aggregates in Aβ deposition, are mainly or exclusively composed of N-terminal truncated and/or post-translationally modified Aβ variants. This supports that the basis mechanisms of β-amyloidosis are related to an aggregation of these N-terminal truncated and/or post-translationally modified Aβ variants.

The present invention thus relates to a preparation comprising a β-amyloid variant or an N-terminal fragment thereof characterized in that said β-amyloid variant or said N-terminal fragment thereof contains an N-terminal truncation and/or a post-translational modification. These β-amyloid variants and the N-terminal fragment thereof are jointly called "N-terminal truncated and/or post-translationally modified Aβ peptides."

The preparation of the invention is typically substantially pure. This means that the β-amyloid variant or the N-terminal fragment thereof is typically at least about 50% w/w (weight/weight) pure, as well as being substantially free from interfering proteins and contaminants. It is preferred that the β-amyloid variant or the N-terminal fragment thereof is at least about 80% w/w and, more preferably at least 90% or about 95% w/w pure. Using conventional protein purification techniques, homogeneous peptides of at least 99% w/w can even be obtained.

The N-terminal truncated and/or post-translationally modified β-amyloid variants that were present in brains from patients in the infraclinical stages of Alzheimer's disease typically consisted of Aβ starting at position 2, 3, 4, 5, 6, 7, 8, 9, or 10. Post-translational modifications that were characterized are methylation or pyroglutamylation. But N-terminal truncated Aβ variants without any post-translational modification have also been identified. Accordingly, the present invention relates to the preparation as described above, further characterized in that the N-terminal truncated β-amyloid variant or the N-terminal fragment thereof starts at position 2, 3, 4, 5, 6, 7, 8, 9, or 10 of β-amyloid and that the post-translational modification, if present, is a methylation or a pyroglutamylation.
In a preferred embodiment of the invention the β-amyloid variant or the N-terminal fragment thereof starts at position 2, 3, 4, 5, 8, 9, or 10 of β-amyloid. In another preferred embodiment, the β-amyloid variant or the N-terminal fragment thereof starts at position 3, 4, 5, 8, or 9 of β-amyloid. In another preferred embodiment, the β-amyloid variant or the N-terminal fragment thereof contains a methylation at any one of positions 1, 2, 4, or 6. In another preferred embodiment, the β-amyloid variant or the N-terminal fragment thereof is pyroglutamylated at position 3. Accordingly, the present invention relates to the preparation as discussed above, further characterized in that the pyroglutamylation is present at position 3 of an N-terminal truncated β-amyloid variant starting at position 3 of β-amyloid.

The N-terminal fragments of the invention typically have a sequence of at least 3, 4, 5, 6, 7, 8, 9, 10, 11, 12, 13, 14, 15, 16, 17, 18, 19, 20, or more contiguous amino acids from the N-terminal part of the N-terminal truncated and/or post-translationally modified β-amyloid variant. Accordingly, the N-terminal fragments of the invention may consist of or comprise the peptides as indicated in SEQ ID NOs 1 to 143. In other cases, the β-amyloid peptides of the invention may consist of longer polypeptides that include the β-amyloid variant together with other amino acids. Accordingly, the β-amyloid peptides of the invention may also consist of or comprise the peptides as indicated in SEQ ID NOs 144 to 165. The present invention thus relates to the above preparation, further characterized in that the β-amyloid variant or the N-terminal fragment thereof comprises an amino acid sequence selected from the group consisting of SEQ ID NOs 1 to 165.
In addition to a methylation or pyroglutamylation, the β-amyloid peptides of the present invention may comprise other modifications. Therefore, the present invention also relates to the preparation as discussed above, further characterized in that the N-terminal truncated and/or post-translationally modified β-amyloid variant or the N-terminal fragment thereof contains an additional modification resulting in a separated spot of the N-terminal truncated and/or post-translationally modified β-amyloid variant or the N-terminal fragment thereof on two-dimensional gel electrophoresis compared to the spot obtained with the N-terminal truncated and/or post-translationally modified β-amyloid variant or the N-terminal fragment thereof without said additional modification. Additional modifications include any modification which induces an apparent shift in the isoelectric point (pI) and/or molecular weight (MW) on a two-dimensional gel. They include, but are not limited, to phosphorylation, addition of isoaspartate, acetylation, glycosylation, racemization, isomerization, proteolysis, stereomerization, cyclization. In a preferred embodiment, the additional modification is an isoaspartate on position 7 of the N-terminal truncated Aβ peptide.

The demonstration, by the present inventors, that N-terminal truncated and/or post-translationally modified Aβ variants are amongst the earliest pathological antigens of Alzheimer disease demonstrates that they are the ideal target for vaccination. Indeed, N-terminal truncated and/or post-translationally modified peptides will target the immune response specifically towards these N-terminal truncated and/or post-translationally modified Aβ variants, which do not exist naturally. The resulting immune response will, consequently, selectively clear these pathological N-terminal truncated and/or post-translationally modified Aβ variants.

In order to induce an immune response, the preparation of the invention should be immunogenic. An "immunogenic preparation" is a preparation that comprises an "immunogenic agent" or "immunogen" that is capable of inducing an immunological response directed against itself upon administration to a recipient mammal, optionally in conjunction with an adjuvant. The immunogenic preparation of the present invention may comprise an N-terminal truncated and/or post-translationally modified Aβ peptide as immunogen. The term "immunological" or "immune" response refers to the development of a beneficial humoral (antibody-mediated) and/or a cellular (mediated by antigen-specific T-cells or their secretion products) response directed against the N-terminal truncated and/or post-translationally modified Aβ peptide in a recipient mammal. Such a response can be an active response induced by administration of immunogen or a passive response induced by administration of antibody or primed T-cells (see further). A cellular immune response is elicited by the presentation of polypeptide epitopes in association with Class I or Class II MHC molecules to activated antigen-specific CD4 T helper cells and/or CD8+ cytotoxic T-cells. The response may also involve activation of monocytes, macrophages, NK cells, basophils, dendritic cells, astrocytes, microglia cells, eosinophils or components of innate immunity.
Some agents for inducing an immune response contain the appropriate epitope for induction of an immune response against amyloid deposits but are in themselves too small to be immunogenic. In this situation, a peptide immunogen can be linked to a suitable carrier to help elicit an immune response. Suitable carriers include serum albumins, keyhole limphet hemocyanin, immunoglobulin molecules, thyroglobulin, ovalbumin, tetanus toxoid, or a toxoid from other pathogenic bacteria, such as diphtheria, *E. coli,* cholera, or *H. pylori,* or an attenuated toxin derivative. Other carriers for stimulating or enhancing an immune response include cytokines such as IL-1, IL-1 α and β peptides, IL-2, αTNF, IL-10, GM-CSF, and chemokines, such as MIP1α and β and RANTES. Immunogenic agents can also be linked to peptides that enhance transport across tissues, as described in WO 97/17613 and WO 97/17614. Immunogenic agents can be linked to carriers by chemical crosslinking. Techniques for linking an immunogen to a carrier include the formation of disulfide linkages using N- succinimidyl-3-(2-pyridyl-thio) propionate (SPDP) and succinimidyl 4-(N-maleimidomethyl)cyclohexane-1-carboxylate (SMCC) (if the peptide lacks a sulfhydryl group, this can be provided by addition of a cysteine residue). These reagents create a disulfide linkage between themselves and peptide cysteine residus on one protein, and an amide linkage through the ε-amino on a lysine, or other free amino group in other amino acids. A variety of such disulfide/amide-forming agents are described by Janssen et al. (1982). Other bifunctional coupling agents form a thioether rather than a disulfide linkage. Many of these thio-ether-forming agents are commercially available and include reactive esters of 6-maleimidocaproic acid, 2-bromoacetic acid, and 2-iodoacetic acid, 4-(N-maleimido-methyl)cyclohexane-1-carboxylic acid. The carboxyl groups can be activated by combining them with succinimide or 1-hydroxyl-2-nitro-4-sulfonic acid, sodium salt. Immunogenic peptides can also be expressed as fusion proteins with carriers. The immunogenic peptide can be linked at the amino terminus, the carboxyl terminus, or internally to the carrier. Optionally, multiple repeats of the immunogenic peptide can be present in the fusion protein.

### N-terminal APP soluble fragments

The finding of the N-terminal truncated and/or post-translationally modified Aβ variants as a result from an aberrant pathway of APP metabolism supports the presence of N-terminal APP soluble fragments containing extra amino acids at the C-terminal tail, resulting from truncated cleavage by β-like secretase (i.e. behind amino acid 1, 2, 3, 4, 5, 6, 7, 8, or 9 of Aβ). As discussed in the background art section, Aβ is metabolized from APP by N- and C-terminal cleavage of APP by β- and γ-secretase (Fig. 1). APP is found in three isoforms, APP695, APP751, and APP770, referring, respectively, to the 695, 751, and 770 amino acid residue long polypeptides encoded by the human APP gene (Kang et al., 1987; Ponte et al. 1988; Kitaguchi et al., 1988). In the present invention, amino acids within the human amyloid precursor protein (APP) are assigned numbers according to the sequence of the APP770 isoform (Fig. 1). β-secretase cleaves at the amino terminus of the β-amyloid peptide between positions 671 and 672 of APP. An aberrant cleavage of this β-secretase, however, more towards the Aβ sequence, will result in N-terminal APP fragments with one or more additional Aβ amino acids. Accordingly, the present invention relates to a preparation comprising an N-terminal APP soluble fragment obtainable by β-like secretase cleavage of APP, characterized in that the C-terminal end of said N-terminal APP soluble fragment consists of position 1, or 1 to 2, 1 to 3, 1 to 4, 1 to 5, 1 to 6, 1 to 7, 1 to 8, or 1 to 9 of β-amyloid. The invention also relates to a C-terminal fragment of such an N-terminal APP soluble fragment. In a specific embodiment, the invention relates to the preparation as described above, further characterized in that the N-terminal APP soluble fragment or the C-terminal fragment thereof comprises an amino acid sequence selected from the group consisting of SEQ ID NOs 1 to 6, 14 to 18, 27 to 30, 53 to 55, 66 to 67, 79, and 166 to 261.

The Aβ variants of the invention, their N-terminal fragments, the N-terminal APP soluble fragments of the invention and the C-terminal fragments thereof can be synthesized by solid phase peptide synthesis or recombinant expression, or can be obtained from natural sources. Automatic peptide synthesizers are commercially available from numerous suppliers, such as Applied Biosystems (Foster City, California, US). Recombinant expression can be in bacteria, such as *E. coli,* in yeast, in insect cells or in mammalian cells. Procedures for recombinant expression are described by Sambrook et al. (1989).

Also included in the present invention are peptides and molecules, also called peptidomimetics, which retain the essential three-dimensional shape and immunological binding capacity of the Aβ peptides and APP soluble fragments of the invention. These peptidomimetics can mimic the Aβ peptides and APP soluble fragments of the invention for inducing an immune response in a mammalian recipient or for the formation of an immunological complex with an antibody specific for said Aβ peptide or APP soluble fragment of the invention.

### Nucleic acids

The present invention also relates to a nucleic acid preparation comprising a nucleic acid sequence capable of encoding the β-amyloid variant of the invention or the N-terminal fragment thereof or capable of encoding the N-terminal APP soluble fragment of the invention or the C-terminal fragment thereof. The nucleic acids of the invention can be DNA or RNA. A DNA molecule is "capable of expressing" a polypeptide, such as the β-amyloid variant of the invention or the N-terminal fragment thereof or the N-terminal APP soluble fragment of the invention or the C-terminal fragment thereof, if it contains nucleotide sequences which contain transcriptional and translational regulatory information, and such sequences are "operably linked" to nucleotide sequences which encode the polypeptide. An operable linkage is a linkage in which the regulatory DNA sequences and the DNA sequence sought to be expressed are connected in such a way as to permit gene expression. The regulatory regions needed for gene expression, in general, include a promoter region as well as the DNA sequences which, when transcribed into RNA, will signal the initiation of protein synthesis. Such regions will normally include those 5' -noncoding sequences involved in initiation of transcription and translation. A promoter region would be operably linked to a DNA sequence if the promoter were capable of effecting transcription of that DNA sequence. The nucleic acid segment encoding the β-amyloid variant of the invention or the N-terminal fragment thereof or the N-terminal APP soluble fragment of the invention or the C-terminal fragment thereof should thus be linked to regulatory elements, such as a promoter and enhancer, that allow expression of the nucleic acid segment in the intended host. For expression in blood cells, for example, as is desirable for induction of an immune response in a mammal, promoter and enhancer elements from light or heavy chain immunoglobulin genes or the CMV major intermediate early promoter and enhancer are suitable to direct expression.
The linked regulatory elements and coding sequences are often cloned into a vector. A vector is a nucleic acid, preferably a DNA molecule, capable of autonomous replication in a cell, to which a nucleic acid segment (e. g. a gene or polynucleotide, preferably DNA) can be operatively linked so as to bring about replication of the attached segment. Vectors capable of directing the expression of nucleic acid (preferably DNA) segments (genes) encoding for one or more proteins are referred to as "expression vectors". An expression vector thus is any plasmid or virus into which a foreign nucleotide sequence (preferably DNA) may be inserted or expressed. A number of viral vector systems are available, including (but not limited to) retroviral systems (Lawrie and Tumin, 1993), adenoviral vectors (Bett et al., 1993), adeno-associated virus vectors (Zhou et al., 1994), viral vectors from the pox family including vaccinia virus and the avian pox viruses, viral vectors from the alpha virus genus such as those derived from Sindbis and Semliki Forest Viruses (Dubensky et al., 1996), and papillomaviruses (Ohe et al., 1995; WO 94/12629; Xiao and Brandsma, 1996).

### Antibodies

In another embodiment, the invention relates to a method for the preparation of an antibody that specifically recognizes the N-terminal truncated and/or post-translationally modified β-amyloid variant of the invention. Said method comprises the following steps:
(a) Immunizing an animal with an immunogenic preparation comprising an N-terminal truncated and/or post-translationally modified Aβ peptide or with a nucleic acid preparation encoding said N-terminal truncated and/or post-translationally modified Aβ peptide;
(b) Obtaining the antibodies generated by the immunization in step (a);
(c) Screening the antibodies obtained in step (b) for their specific recognition of N-terminal truncated and/or post-translationally modified β-amyloid variants.
The invention further relates to antibodies obtainable by the above method.

In a further embodiment, the present invention relates to a method for the preparation of an antibody that specifically recognizes the N-terminal APP soluble fragment of the invention. Said method comprises the following steps:
(a) Immunizing an animal with a preparation of N-terminal APP soluble fragment of the invention or C-terminal fragment thereof or with the nucleic acid preparation encoding said N-terminal APP soluble fragment of the invention or said C-terminal fragment thereof;
(b) Obtaining the antibodies generated by the immunization in step (a);
(c) Screening the antibodies obtained in step (b) for their specific recognition of N-terminal APP soluble fragment of the invention.
The invention further relates to antibodies obtainable by the above method.

The term "specific recognition", "specifically recognizing", "specifically binding with", "specifically reacting with" or "specifically forming an immunological reaction with" refers to a binding reaction by the antibody to the N-terminal truncated and/or post-translationally modified β-amyloid variant or to the N-terminal APP soluble fragment respectively, which is determinative of the presence of the N-terminal truncated and/or post-translationally modified β-amyloid variant or the N-terminal APP soluble fragment respectively in the sample tested, in the presence of a heterogeneous population of other proteins and/or other biologics. Thus, under the designated immunoassay conditions, the specified antibody preferentially binds to an N-terminal truncated and/or post-translationally modified β-amyloid variant or to an N-terminal APP soluble fragment of the invention while binding to other proteins or protein isoforms does not occur in significant amounts. In a preferred embodiment the specified antibody preferentially binds to an N-terminal truncated and/or post-translationally modified β-amyloid variant or to an N-terminal APP soluble fragment of the invention while binding to normal, non pathological Aβ or to normal N-terminal APP fragment does not occur in significant amounts.

As used herein, an "antibody" refers to a protein consisting of one or more polypeptides substantially encoded by immunoglobulin genes or fragments of immunoglobulin genes. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids, primarily responsible for antigen recognition. The terms "variable light chain (V_{L})" and "variable heavy chain (V_{H})" refer to these variable regions of the light and heavy chains respectively. Optionally, the antibody or the immunological portion of the antibody, can be chemically conjugated to, or expressed as, a fusion protein with other proteins.
Antibodies of the invention include, but are not limited to polyclonal, monoclonal, bispecific, human, humanized or chimeric antibodies, single variable fragments (ssFv), single chain fragments (scFv), Fab fragments, F(ab') fragments, fragments produced by a Fab expression library, anti-idiotypic antibodies and epitope-binding fragments of any of the above, provided that they retain the original binding properties. Also mini-antibodies and multivalent antibodies such as diabodies, triabodies, tetravalent antibodies and peptabodies can be used in a method of the invention. The preparation and use of these fragments and multivalent antibodies has been described extensively in International Patent Application WO 98/29442. The immunoglobulin molecules of the invention can be of any class (i.e. IgG, IgE, IgM, IgD and IgA) or subclass of immunoglobulin molecules.

The N-terminal truncated and/or post-translationally modified Aβ variants, the N-terminal fragments thereof, the N-terminal APP soluble fragments of the invention or the C-terminal fragments thereof can be used as an immunogen to generate the antibodies of the invention which specifically bind such an immunogen. Various host animals can be immunized for injection with said N-terminal truncated and/or post-translationally modified Aβ variants, the N-terminal fragments thereof, the N-terminal APP soluble fragments of the invention or the C-terminal fragments thereof, including, but not limited to, rabbits, mice, rats, etc. Various adjuvants may be used to enhance the immunological response, depending on the host species, including, but not limited to, complete or incomplete Freund's adjuvant, a mineral gel such as aluminum hydroxide, surface active substances such as lysolecithin, pluronic polyol, a polyanion, a peptide, an oil emulsion, keyhole limpet hemocyanin, dinitrophenol, or an adjuvant such as BCG (bacille Calmette-Guerin) or corynebacterium parvum. For the preparation of monoclonal antibodies, any technique which provides for the production of antibody molecules by continuous cell lines in culture may be used. Hyperimmunization of an appropriate donor, generally a mouse, with the antigen is undertaken. Isolation of splenic antibody producing cells is then carried out. These cells are fused to a cell characterized by immortality, such as a myeloma cell, to provide a fused cell hybrid (Hybridoma) which can be maintained in culture and which secretes the required monoclonal antibody. The cells are then cultured in bulk and the monoclonal antibodies harvested from the culture media for use. Specific techniques include but are not limited to the hybridoma technique developed by Kohler and Milstein (1975), the human B-cell hybridoma technique (Kozbor et al., 1983) or the EBV-hybridoma technique to produce human monoclonal antibodies (Cole et al., 1985). Screening for the desired antibody can be done by techniques known in the art, such as ELISA. For selection of an antibody that specifically binds an N-terminal truncated and/or post-translationally modified Aβ variant or an N-terminal APP soluble fragment of the invention, but that does not specifically bind another protein, normal Aβ peptide or normal N-terminal APP soluble fragment, can be done on the basis of positive binding to the first and the lack of binding to the second. Thus, in a particular embodiment, the present invention provides an antibody that binds with greater affinity (particularly at least 2-fold, more particularly at least 5-fold, still more particularly at least 10-fold greater affinity) to an N-terminal truncated and/or post-translationally modified Aβ variant or to an N-terminal APP soluble fragment of the invention than to another protein. In another preferred embodiment, the present invention provides an antibody that binds with greater affinity (particularly at least 2-fold, more particularly at least 5-fold, still more particularly at least 10-fold greater affinity) to an N-terminal truncated and/or post-translationally modified Aβ variant or to an N-terminal APP soluble fragment of the invention than to normal Aβ peptide or to normal N-terminal APP soluble fragment.

While various antibody fragments are defined in terms of enzymatic digestion of an intact antibody with papain, pepsin or other proteases, one of skill will appreciate that such antibody fragments as well as full size antibodies may be synthesized de novo either chemically or by utilizing recombinant DNA methodology. Thus, the term antibody, as used herein, also includes antibodies and antibody fragments either produced by the modification of whole antibodies or synthesized de novo using recombinant DNA methodologies.

The term "humanized antibody" means that at least a portion of the framework regions of an immunoglobulin is derived from human immunoglobulin sequences. The humanized versions of the mouse monoclonal antibodies can, for example, be made by means of recombinant DNA technology, departing from the mouse and/or human genomic DNA sequences coding for H and L chains or from cDNA clones coding for H and L chains. Humanized forms of mouse antibodies can be generated by linking the CDR regions of non-human antibodies to human constant regions by recombinant DNA techniques (Queen et al., 1989; WO 90/07861). Alternatively the monoclonal antibodies used in the method of the invention may be human monoclonal antibodies. Human antibodies can be obtained, for example, using phage-display methods (WO 91/17271; WO 92/01047). In these methods, libraries of phage are produced in which members display different antibodies on their outer surfaces. Antibodies are usually displayed as Fv or Fab fragments. Phage displaying antibodies with a desired specificity are selected by affinity enrichment to N-terminal truncated and/or post-translationally modified Aβ peptide or APP soluble fragment of the invention (or C-terminal fragment thereof). Human antibodies against N-terminal truncated and/or post-translationally modified Aβ peptide or against APP soluble fragment can also be produced from non-human transgenic mammals having transgenes encoding at least a segment of the human immunoglobulin locus and an inactivated endogenous immunoglobulin locus (WO93/12227; WO 91/10741). Human antibodies can be selected by competitive binding experiments, or otherwise, to have the same epitope specificity as a particular mouse antibody. Such antibodies are particularly likely to share the useful functional properties of the mouse antibodies. Human polyclonal antibodies can also be provided in the form of serum from humans immunized with an immunogenic agent. Optionally, such polyclonal antibodies can be concentrated by affinity purification using N-terminal truncated and/or post-translationally modified Aβ peptide or using N-terminal APP soluble fragment or a C-terminal fragment thereof as an affinity reagent. Monoclonal antibodies can be obtained from serum according to the technique described in WO 99/60846.

### Vaccine and therapeutic compositions

As indicated above, the present invention provides preparations and methods for preventing and treating, in a mammal, a disease characterized by β-amyloid formation and/or aggregation by induction of an immune response in said mammal. Accordingly, the present invention also provides a vaccine composition or a therapeutic composition comprising an N-terminal truncated and/or post-translationally modified Aβ peptide as referred to above, comprising an antibody or a T-cell specific for an N-terminal truncated and/or post-translationally modified Aβ peptide as referred to above, or comprising a nucleic acid encoding an N-terminal truncated and/or post-translationally modified Aβ peptide as referred to above.

As used herein, the term "preventing a disease" means inhibiting or reversing the onset of the disease, inhibiting or reversing the initial signs of the disease (i.e. formation and/or aggregation of Aβ variants), inhibiting the appearance of clinical symptoms of the disease. As used herein, the term "treating a disease" includes substantially inhibiting the disease, substantially slowing or reversing the progression of the disease, substantially ameliorating clinical symptoms of the disease or substantially preventing the appearance of clinical symptoms of the disease.
The mammal examined in the present invention may be a non-human mammal, such as (but not limited to) a cow, a pig, a sheep, a goat, a horse, a monkey, a rabbit, a hare, a dog, a cat, a mouse, a rat, an elk, a deer, or a tiger. In a preferred embodiment, the mammal is a primate. In another preferred embodiment the mammal is a human, more preferably the mammal is a human adult.
The vaccine or therapeutic compositions of the present invention induce an immune response against the specific N-terminal truncated and/or post-translationally modified Aβ peptides of the invention. The induction of an immune response is "active" when an immunogen is administered to induce antibodies or T- cells reactive against the immunogen. The induction of an immune response is "passive" when an antibody is administered that itself binds to the N-terminal truncated and/or post-translationally modified Aβ variants in the mammal.
Accordingly, the vaccine or therapeutic compositions of the invention may also comprise antibodies that specifically bind to the N-terminal truncated and/or post-translationally modified Aβ variants of the invention.
Furthermore, immune responses against the N-terminal truncated and/or post-translationally modified Aβ peptides of the invention can also be induced by administration of nucleic acids encoding said N-terminal truncated and/or post-translationally modified Aβ peptides, or encoding recombinant antibodies that specifically recognize said N-terminal truncated and/or post-translational modified Aβ peptides. Such nucleic acids can be DNA or RNA. In order to facilitate the introduction of a recombinant DNA molecule into cells of the CNS, a number of different means for gene delivery can be used in association with the recombinant DNA molecule. The term "means for gene delivery" is meant to include any technique suitable for delivery of DNA molecules across the blood brain barrier and/or for transmembrane delivery across cell membranes. Non-limiting examples of means for gene delivery are viral vectors (e.g., adeno-associated virus-based vectors, lipids/liposomes, ligands for cell surface receptors, etc). DNA encoding an immunogen, or a vector containing the same, can be packaged into liposomes. Suitable lipids and related analogs are described by US 5,208,036, 5,264,618, 5,279,833, and 5,283,185. Vectors and DNA encoding an immunogen can also be adsorbed to or associated with particulate carriers, examples of which include polymethyl methacrylate polymers and polylactides and poly(lactide-co-glycolides) (McGee et al., 1996). Gene therapy vectors or naked DNA can be delivered in vivo by administration to an individual patient, typically by systemic administration (e.g., intravenous, intraperitoneal, nasal, gastric, intradermal, intramuscular, subdermal, or intracranial infusion) or topical application (see e.g., US 5,399,346). DNA can also be administered using a gene gun (Xiao and Brandsma, 1996). The DNA encoding an immunogen is precipitated onto the surface of microscopic metal beads. The microprojectiles are accelerated with a shock wave or expanding helium gas, and penetrate tissues to a depth of several cell layers. For example, The Accel^{™} Gene Delivery Device manufactured by Agacetus, Inc. (Middleton WI, US) is suitable. Alternatively, naked DNA can pass through skin into the blood stream simply by spotting the DNA onto skin with chemical or mechanical irritation (WO 95/05853). In a further variation, vectors encoding the N-terminal truncated and/or post-translationally modified peptide, or encoding recombinant antibodies specifically reconizing said N-terminal truncated and/or post-translationally modified peptide, can be delivered to cells ex vivo, such as cells explanted from an individual patient (e.g., lymphocytes, bone marrow aspirates, tissue biopsy) or universal donor hematopoietic stem cells, followed by reimplantation of the cells into a patient, usually after selection for cells which have incorporated the vector.
In a further variation, the N-terminal truncated and/or post-translationally modified Aβ peptide can be presented as a viral or bacterial vaccine. A nucleic acid encoding the immunogenic peptide is incorporated into a genome or episome of the virus or bacteria. Optionally, the nucleic acid is incorporated in such a manner that the immunogenic peptide is expressed as a secreted protein or as a fusion protein with an outersurface protein of a virus or a transmembrane protein of a bacterium so that the peptide is displayed (Frenkel et al., 2000; 2001; WO 01/18169). Viruses or bacteria used in such methods should be nonpathogenic or attenuated. Suitable viruses include bacteriophage such as filamentous bacteriophage, adenovirus, HSV, vaccinia, and fowl pox. Fusion of an immunogenic peptide to HBsAg of HBV is particularly suitable.

The vaccine and/or therapeutic composition of the present invention may also comprise T-cells that bind to the N-terminal truncated and/or post-translationally modified Aβ peptide of the invention. For example, T-cells can be activated against said peptides by expressing a human MHC class I gene and a human β-2-microglobulin gene from an insect cell line. An empty complex is formed on the surface of the cells that can be loaded with the N-terminal truncated and/or post-translational modified Aβ peptide of the invention. T-cells contacted with the cell line become specifically activated against the peptides of the invention (US 5,314,813). Insect cell lines expressing an MHC class II antigen can similarly be used to activate CD4 T-cells.

In prophylactic applications, vaccine compositions are administered to a mammal susceptible to, or at risk of developing a disease associated with β-amyloid formation and/or aggregation in an amount sufficient to eliminate or reduce the risk or delay the ontset of said disease. In therapeutic applications, compositions are administered to a mammal suspected of, or already suffering from such a disease in an amount sufficient to cure, or at least partially arrest, the symptoms of the disease and its complications. An amount adequate to accomplish this is defined as a therapeutically- or pharmaceutically-effective dose. In both prophylactic and therapeutic regimes, agents are usually administered in several dosages until a sufficient immune response has been achieved. Typically, the immune response is monitored and repeated dosages are given if the immune response starts to fade.
Effective doses of the vaccine and therapeutic compositions of the present invention, vary depending upon many different factors, including means of administration, target site, physiological state of the mammal, whether the patient is a human or an animal, other medications administered, and whether treatment is prophylactic or therapeutic. Treatment dosages need to be titrated to optimize safety and efficacy. The amount of immunogen depends on whether adjuvant is also administered, with higher dosages being required in the absence of adjuvant. The amount of an immunogen for administration sometimes varies from 1-500 µg per mammal and more usually from 5-500 µg per injection for human administration. Occasionally, a higher dose of 1-2 mg per injection is used. Typically about 10, 20, 50 or 100 µg is used for each human injection. The timing of injections can vary significantly from once a day, to once a year, to once a decade. On any given day that a dosage of immunogen is given, the dosage is greater than 1 µg/patient and usually greater than 10 µg/patient if adjuvant is also administered. In the absence of adjuvant, the dosage is greater than 10 µg/patient and usually greater than 100 µg/patient. A typical regimen consists of an immunization followed by booster injections at 6 weekly intervals. Another regimen consists of an immunization followed by booster injections 1, 2 and 12 months later. Another regimen consists of an injection every two months for life. Alternatively, booster injections can be on an irregular basis as indicated by monitoring of immune response.
For passive immunization with an antibody, the dosage ranges from about 0.0001 to 100 mg/kg, and more usually 0.01 to 5 mg/kg of the host body weight. Doses for nucleic acids encoding immunogens range from about 10 ng to 1 g, 100 ng to 100 mg, 1 µg to 10 mg, or 30 to 300 µg DNA per patient. Doses for infectious viral vectors vary from 10 to 10⁹ or more virions per dose.
Agents for inducing an immune response can be administered by parenteral, topical, intravenous, oral, subcutaneous, intraperitoneal, intranasal, or intramuscular means for prophylactic and/or therapeutic treatment. The most typical route of administration is subcutaneous, although others can be equally effective. The next most common is intramuscular injection. This type of injection is most typically performed in the arm or leg muscles. Intravenous injections as well as intraperitoneal injections, intraarterial, intracranial, or intradermal injections are also effective in generating an immune response. In some methods, agents are injected directly into a particular tissue where deposits are accumulated. Intranasal immunization was successfully used to increase the production of anti-Aβ antibodies in wildtype mice (Lemere et al. 2000b,c).
Vaccine or therapeutic compositions of the invention can optionally comprise other agents that are at least partly effective in treatment of diseases associated with β-amyloid formation and/or aggregation. In the case of Alzheimer's and Down's syndrome, in which β-amyloid aggregation occurs in the brain, the vaccine or therapeutic composition of the invention may also comprise other agents that increase passage of the active components of the composition of the invention across the blood-brain barrier. A peptide, protegrin PG-1, belonging to the family of beta-stranded antimicrobil peptides, for example, has been successfully used to deliver therapeutic compounds into eucaryotic cells (Drin and Temsamani, 2002). Other strategies to enhance drug delivery across the blood brain barrier, especially vector-mediated strategies, have been reviewed by Temsamani et al. (2001).
Immunogenic agents of the invention, such as peptides, are sometimes administered in combination with an adjuvant. The term "adjuvant" refers to a compound that, when administered in conjunction with an antigen, augments the immune response to the antigen, but, when administered alone, does not generate an immune response to the antigen. Adjuvants can augment an immune response by several mechanisms including lymphocyte recruitment, stimulation of B and/or T cells, and stimulation of macrophages. A variety of adjuvants can be used in combination with the N-terminal truncated and/or post-translationally modified Aβ peptides of the invention in order to elicit an immune response. Preferred adjuvants augment the intrinsic response to an immunogen without causing conformational changes in the immunogen that affect the qualitative form of the response. Preferred adjuvants include alum, 3 De-O-acylated monophosphoryl lipid A (MPL) (GB 2220211). QS21 is a triterpene glycoside or saponin isolated from the bark of the Quillaja Saponaria Molina tree found in South America (Kensil et al., 1995; US 5,057,540). Other adjuvants are oil in water emulsions (such as squalene or peanut oil), optionally in combination with immune stimulants, such as monophosphoryl lipid A (Stoute et al., 1997). Another adjuvant is CpG (Davis et al., 1998). Alternatively, the N-terminal truncated and/or post-translationally modified Aβ peptide can be coupled to an adjuvant. For example, a lipopeptide version of the N-terminal truncated and/or post-translationally modified Aβ peptide can be prepared by coupling palmitic acid or other lipids directly to the N-terminal truncated and/or post-translational modified Aβ peptide as described for hepatitis D antigen vaccination (Livingston et al, 1997). However, such coupling should not substantially change the conformation of the N-terminal truncated and/or post-translational modified Aβ peptide so as to affect the nature of the immune response thereto.

A preferred class of adjuvants is aluminum salts (alum), such as aluminum hydroxide, aluminum phosphate, aluminum sulfate. Such adjuvants can be used with or without other specific immunostimulating agents such as MPL or 3-DMP, QS21, polymeric or monomeric amino acids such as polyglutamic acid or polylysine. Another class of adjuvants is oil-in-water emulsion formulations. Such adjuvants can be used with or without other specific immunostimulating agents such as muramyl peptides (e.g., N-acetylmuramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'dipalmitoyl-sn-glycero-3-hydroxyphosphoryloxy)-ethylamine (MTP-PE), N-acetylglucosaminyl-N-acetylinuramyl-L-Ala-D-isoglu-L-Ala-dipalmitoxy propylamide (DTP-DPP) theramide^{™}) (Vogel et al., 2003), or other bacterial cell wall components. Oil-in-water emulsions include (a) MF59 (WO 90/14837), containing 5% Squalene, 0.5% Tween 80, and 0.5% Span 85 (optionally containing various amounts of MTP-PE) formulated into submicron particles using a microfluidizer such as Model110Y microfluidizer (Micro fluidics, Newton, MA, US), (b) SAF, containing 10% Squalane, 0.4% Tween 80, 5% pluronic-blocked polymer L121, and thr-MDP, either microfluidized into a submicron emulsion or vortexed to generate a larger particle size emulsion, and (c) Ribi^{™} adjuvant system (RAS), (Ribi Immunochem, Hamilton, MT, US) containing 2% squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox^{™}).
Another class of preferred adjuvants is saponin adjuvants, such as Stimulon^{™} (QS21, Aquila, Worcester, MA) or particles generated therefrom, such as ISCOMs (immunostimulating complexes) and ISCOMATRIX. Other adjuvants include Complete Freund's Adjuvant (CFA) and Incomplete Freund's Adjuvant (IFA). Other adjuvants include cytokines, such as interleukins (IL-1, IL-2, and IL-12), macrophage colony stimulating factor (M-CSF), and tumor necrosis factor (TNF). Published mucosal adjuvants include bacterial enterotoxins such as cholera toxin (CT) and E. coli LT, which are ~80% homologous (Dallas and Falkow, 1980).
An adjuvant can be administered with an immunogen as a single composition, or can be administered before, concurrent with or after administration of the immunogen. Immunogen and adjuvant can be packaged and supplied in the same vial or can be packaged in separate vials and mixed before use. Immunogen and adjuvant are typically packaged with a label indicating the intended therapeutic application. If immunogen and adjuvant are packaged separately, the packaging typically includes instructions for mixing before use. The choice of an adjuvant and/or carrier depends on the stability of the vaccine containing the adjuvant, the route of administration, the dosing schedule, the efficacy of the adjuvant for the species being vaccinated, and, in humans, a pharmaceutically acceptable adjuvant is one that has been approved or is approvable for human administration by pertinent regulatory bodies. For example, Complete Freund's adjuvant is not suitable for human administration. Alum, MPL and QS21 are preferred. Optionally, two or more different adjuvants can be used simultaneously. Preferred combinations include alum with MPL, alum with QS21, MPL with QS21, and alum, QS21 and MPL together. Also, Incomplete Freund's ajuvant can be used (Jensen et al., 1998), optionally in combination with any of alum, QS21, and MPL and all combinations thereof.
The preferred form of the vaccine and/or therapeutic composition depends on the intended mode of administration and application. The compositions can also include, depending on the formulation desired, pharmaceutically-acceptable, non-toxic carriers or diluents, which are defined as vehicles commonly used to formulate pharmaceutical compositions for animal or human administration. The diluent is selected so as not to affect the biological activity of the combination. Examples of such diluents are distilled water, physiological phosphate-buffered saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.
The vaccine or pharmaceutical compositions can also include large, slowly metabolized macromolecules such as proteins, polysaccharides, polylactic acids, polyglycolic acids and copolymers (such as latex functionalized sepharose, agarose, cellulose, and the like), polymeric amino acids, amino acid copolymers, and lipid aggregates (such as oil droplets or liposomes). Additionally, these carriers can function as immunostimulating agents (i.e., adjuvants).
For parenteral administration, the compositions of the invention can be administered as injectable dosages of a solution or suspension of the substance in a physiologically acceptable diluent with a pharmaceutical carrier that can be a sterile liquid such as water, oils, saline, glycerol, or ethanol. Also encapsulation into biodegradable microparticles can be used as a parenteral delivery system (Brayden et al., 2001).
Additionally, auxiliary substances, such as wetting or emulsifying agents, surfactants, pH buffering substances and the like can be present in the vaccine and/or therapeutic compositions. Other components of pharmaceutical compositions are those of petroleum, animal, vegetable, or synthetic origin, for example, peanut oil, soybean oil, and mineral oil. In general, glycols such as propylene glycol or polyethylene glycol are preferred liquid carriers, particularly for injectable solutions.
Typically, compositions are prepared as injectables, either as liquid solutions or suspensions. Solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above (Langer, 1990; Langer et al, 1997). The compositions of this invention can be administered in the form of a depot injection or implant preparation which can be formulated in such a manner as to permit a sustained or pulsatile release of the active ingredient.
Additional formulations suitable for other modes of administration include oral, intranasal, and pulmonary formulations, suppositories, and transdermal applications.
For suppositories, binders and carriers include, for example, polyalkylene glycols or triglycerides. Such suppositories can be formed from mixtures containing the active ingredient in the range of 0.5% to 10%, preferably 1% to 2%. Oral formulations include excipients, such as pharmaceutical grades of mannitol, lactose, starch, magnesium stearate, sodium saccharine, cellulose, and magnesium carbonate. These compositions take the form of solutions, suspensions, tablets, pills, capsules, sustained release formulations, or powders and contain 10% to 95% of active ingredient, preferably 25% to 70%.
Topical application can result in transdermal or intradermal delivery. Topical administration can be facilitated by co-administration of the agent with cholera toxin or detoxified derivatives or subunits thereof or other similar bacterial toxins (Glenn et al., 1998). Co-administration can be achieved by using the components as a mixture or as linked molecules obtained by chemical crosslinking or expression as a fusion protein.
Alternatively, transdermal delivery can be achieved using a skin path or using transferosomes (Paul et al., 1995; Cevc et al., 1998).

Further techniques for formulation and administration of drugs can also be found in "Remington's Pharmaceutical Sciences".

The vaccine and therapeutic compositions of the present invention can thus be used for the prevention and/or treatment of any disease associated with β-amyloid formation and/or aggregation. In a preferred embodiment the vaccine and therapeutic compositions of the invention are used for the prevention and/or treatment of Alzheimer's disease or Down's syndrome.
The invention thus relates to an N-terminal truncated and/or post-translationally modified Aβ peptide as described above, an antibody or T-cell specific for an N-terminal truncated and/or post-translationally modified Aβ peptide as described above, or a nucleic acid encoding an N-terminal truncated and/or post-translationally modified Aβ peptide as described above for use as a prophylactic vaccine for the prevention of a disease associated with β-amyloid formation and/or aggregation. The invention thus also relates to the use of an N-terminal truncated and/or post-translationally modified Aβ peptide as discussed above, an antibody or T-cell specific for an N-terminal truncated and/or post-translationally modified Aβ peptide as discussed above, or a nucleic acid encoding an N-terminal truncated and/or post-translationally modified Aβ peptide as discussed above for the manufacture of a prophylactic vaccine for the prevention of a disease associated with β-amyloid formation and/or aggregation.
In non-demented humans, prophylaxis can begin at any age (e.g., 10, 20, 30 years). Usually, however, it is not necessary to begin prophylaxis until a patient reaches the age of 40, 50, 60, or 70 years. Prophylaxis typically encompasses multiple dosages over a period of time. The response can be monitored by assaying antibody, or activated T-cell or B-cell responses to the N-terminal truncated and/or post-translationally modified peptide over time (see further). If the response falls, a booster dosage is indicated.
The invention further relates to an N-terminal truncated and/or post-translationally modified Aβ peptide as discussed above, an antibody specific for an N-terminal truncated and/or post-translationally modified Aβ peptide as discussed above, or a nucleic acid encoding an N-terminal truncated and/or post-translationally modified Aβ peptide as discussed above for use as a therapeutic for the treatment of a disease associated with β-amyloid formation and/or aggregation. The invention thus also relates to the use of an N-terminal truncated and/or post-translationally modified Aβ peptide as discussed above, an antibody specific for an N-terminal truncated and/or post-translationally modified Aβ peptide as discussed above, or a nucleic acid encoding an N-terminal truncated and/or post-translationally modified Aβ peptide as discussed above for the manufacture of a therapeutic for the treatment of a disease associated with β-amyloid formation and/or aggregation.
Accordingly, the present invention also encompasses a method for the prevention and/or treatment, in an mammal, of a disease associated with β-amyloid formation and/or aggregation, said method comprising the administration, to said mammal, of a vaccine composition or a therapeutic composition as described above.

### Diagnostic and theranostic kits

Another aspect of the present invention relates to a diagnostic or theranostic kit comprising a preparation of an N-terminal truncated and/or post-translationally modified peptide as described above, comprising a preparation of N-terminal APP soluble fragment of the invention or C-terminal fragment thereof as described above, or comprising an antibody specifically recognizing said peptide or fragments.
In one embodiment, the kit thus contains a peptide or peptide fragment that specifically binds to antibodies recognizing N-terminal truncated and/or post-translationally modified Aβ peptide or recognizing N-terminal APP soluble fragment of the invention (or C-terminal fragment thereof), or that reacts with T-cells specific for N-terminal truncated and/or post-translationally modified Aβ peptide or specific for N-terminal APP soluble fragment of the invention (or C-terminal fragment thereof). The kit may typically also include a label (see below). For the detection of antibodies to N-terminal truncated and/or post-translationally modified Aβ peptide or N-terminal APP soluble fragment of the invention (or C-terminal fragment thereof), the label is typically in the form of labelled anti-Ig antibodies. For detection of antibodies, the N-terminal truncated and/or post-translationally modified Aβ peptide or N-terminal APP soluble fragment of the invention or C-terminal fragment thereof can be supplied prebound to a solid phase, such as to the wells of a microtiter dish. For detection of reactive T- cells, the label can be supplied as 3H-thymidine to measure a proliferative response.

A diagnostic or theranostic kit comprising N-terminal truncated and/or post-translationally modified Aβ peptide will aid in methods of detecting an immune response against N-terminal truncated and/or post-translationally modified Aβ peptide in a mammal. The immune response can be determined from the presence of antibodies or T- cells that specifically bind to the N-terminal truncated and/or post-translationally modified Aβ peptide. The methods are particularly useful for monitoring a course of treatment being administered to a mammal. The kit can be used to monitor both therapeutic treatment on symptomatic patients and prophylactic treatment on asymptomatic patients. Accordingly, the present invention relates to a preparation comprising N-terminal truncated and/or post-translationally modified Aβ peptide for use as a diagnostic or theranostic for the measurement of the immune response induced in a mammal by vaccination or therapeutic application with respectively a vaccine composition or a therapeutic composition of the invention. The invention thus also relates to the use of a preparation comprising N-terminal truncated and/or post-translationally modified Aβ peptide for the manufacture of a diagnostic or theranostic kit for the measurement of the immune response induced in a mammal by vaccination or therapeutic application with respectively a vaccine composition or a therapeutic composition of the invention.
The diagnostic or theranostic kit of the invention thus will aid in a method for the measurement, in a mammal, of the immune response induced by vaccination or therapeutic application with respectively a vaccine composition or a therapeutic composition of the invention. Said method comprises the following steps:
(a) Determining, in a sample obtained from said mammal, the amount of antibody or reactive T-cell specific for an N-terminal truncated and/or post-translationally modified Aβ peptide;
(b) Comparing the amount determined in step (a) with the amount of antibody or reactive T-cell specific for said N-terminal truncated and/or post-translationally modified Aβ peptide present in the mammal before vaccination or therapeutic application with the vaccine or therapeutic composition of the invention;
(c) Concluding, from the comparison in step (b), whether the mammal is responding to the vaccination or therapy, an increased amount of antibody or reactivated T-cell specific for said N-terminal truncated and/or post-translationally modified Aβ peptide being an indication that the mammal is responding to the vaccination or therapy.
The antibody specific for said N-terminal truncated and/or post-translationally modified Aβ peptide can be detected by an immunoassay. As used herein, an "immunoassay" is an assay that utilizes an antibody to specifically bind to the antigen (i.e. the N-terminal truncated and/or post-translationally modified Aβ peptide). The immunoassay is thus characterized by detection of specific binding of proteins to antibodies. Immunological methods include but are not limited to fluid or gel precipitation reactions, immunodiffusion (single or double), agglutination assays, immunoelectrophoresis, radioimmunoassays (RIA), enzyme-linked immunosorbent assays (ELISA), Western blots, liposome immunoassays (Monroe et al., 1986), complement-fixation assays, immunoradiometric assays, fluorescent immunoassays, protein A immunoassays, or immunoPCR. An overview of different immunoassays is given in Wild D. (2001) and Ghindilis et al. (2002).
T-cells that recognize a particular epitope can be identified by in vitro assays that measure antigen-dependent proliferation, as determined by 3 H-thymidine incorporation by primed T cells in response to an epitope (Burke et al., 1994), by antigen-dependent killing (cytotoxic T lymphocyte assay; Tigges et al. 1996) or by cytokine secretion. The presence of a cell-mediated immunological response can be determined by proliferation assays (CD4+ T cells) or CTL (cytotoxic T lymphocyte) assays (Burke et al. 1994; Tigges et al., 1996).

The method entails determining a baseline value of an immune response in a patient before administering a dosage of the vaccine or therapeutic composition, and comparing this with a value for the immune response after vaccination or therapy. A significant increase (i.e., greater than is the typical margin of experimental error in repeat measurements of the same sample, expressed as one standard deviation from the mean of such measurements) in value of the immune response signals a positive vaccination or therapy outcome (i.e., that administration of the vaccine or therapeutic composition has achieved or augmented an immune response). If the value for immune response does not change significantly, or decreases, a negative vaccination or therapy outcome is indicated. In general, patients undergoing an initial course of treatment with a vaccine or therapeutic composition are expected to show an increase in immune response with successive dosages, which eventually reaches a plateau. Administration of the vaccine or therapeutic composition is generally continued while the immune response is increasing. Attainment of the plateau is an indicator that the administration can be discontinued or reduced in dosage or frequency.
The term "sample" refers to any source of biological material, for instance body fluids, brain extract, peripheral blood, mucus, or any other sample comprising the antibodies or the reactive T-cells to the N-terminal truncated and/or post-translationally modified Aβ peptide. In a preferred embodiment, the level of said antibodies or reactive T-cells is determined in a body fluid sample of the mammal. The term "body fluid" refers to all fluids that are present in the mammalian body including but not limited to blood, lymph, urine, and cerebrospinal fluid (CSF) comprising the antibodies or reactive T-cells to be detected. The term "cerebrospinal fluid" or "CSF" is intended to include whole cerebrospinal fluid or derivatives of fractions thereof well known to those skilled in the art. Thus, a cerebrospinal fluid sample can include various fractionated forms of cerebrospinal fluid or can include various diluents added to facilitate storage or processing in a particular assay. Such diluents are well known to those skilled in the art and include various buffers, preservatives and the like. In another preferred embodiment, the level of antibody is detected in a blood sample of the mammal. The blood sample may include a plasma sample or a serum sample.

In another embodiment of the invention, the diagnostic or theranostic kit of the invention comprises a peptide or peptide fragment that specifically binds to antibodies recognizing N-terminal truncated and/or post-translationally modified Aβ variant or to N-terminal APP soluble fragment of the invention or that reacts with T-cells specific for N-terminal truncated and/or post-translationally modified Aβ variant or for N-terminal APP soluble fragment of the invention. A diagnostic or theranostic kit comprising N-terminal truncated and/or post-translationally modified Aβ peptide, N-terminal APP soluble fragment of the invention or C-terminal fragment thereof will aid in methods of detecting mammals susceptible to or at risk of developing a disease associated with β-amyloid formation and/or aggregation. The susceptibility to or the risk of developing such a disease can be determined from the presence of antibodies or T- cells that specifically bind to the N-terminal truncated and/or post-translationally modified Aβ peptide, to the N-terminal APP soluble fragment of the invention or to the C-terminal fragment thereof. Accordingly, the present invention relates to a preparation comprising an N-terminal truncated and/or post-translationally modified Aβ peptide, an N-terminal APP soluble fragment of the invention, or a C-terminal fragment thereof for use as a diagnostic or theranostic for determining, in a mammal, the susceptibility to a disease associated with β-amyloid formation and/or aggregation or for determining, in a mammal, the risk of developing a disease associated with β-amyloid formation and/or aggregation. The invention thus also relates to the use of a preparation comprising an N-terminal truncated and/or post-translationally modified Aβ peptide, an N-terminal APP soluble fragment of the invention or a C-terminal fragment thereof for the manufacture of a diagnostic or theranostic kit for determining, in a mammal, the susceptibility to a disease associated with β-amyloid formation and/or aggregation or for determining, in a mammal, the risk of developing a disease associated with β-amyloid formation and/or aggregation.
The diagnostic or theranostic kit of the invention thus will aid in a method for the measurement, in a mammal, of the susceptibility to a disease associated with β-amyloid formation and/or aggregation or of the risk of developing a disease associated with β-amyloid formation and/or aggregation. Said method comprises the following steps:
(a) Determining, in a sample obtained from said mammal, the amount of antibody or reactive T-cells specific for an N-terminal truncated and/or post-translationally modified Aβ peptide, for an N-terminal APP soluble fragment of the invention or for a C-terminal fragment thereof;
(b) Comparing the amount determined in step (a) with the amount of said antibody or reactive T-cells in a control mammal;
(c) Concluding, from the comparison in step (b), whether the mammal is susceptible to a disease associated with β-amyloid formation and/or aggregation or whether the mammal is at risk of developing a disease associated with β-amyloid formation and/or aggregation, an increased amount of antibody or reactivated T-cells specific for said N-terminal truncated and/or post-translationally modified Aβ peptide, for said N-terminal APP soluble fragment of the invention or for said C-terminal fragment thereof being an indication that the mammal is susceptible to or at risk of developing a disease associated with Aβ formation and/or aggregation.

In another embodiment, the diagnostic or theranostic kit of the invention comprises an antibody that specifically recognizes N-terminal truncated and/or post-translationally modified Aβ peptide or N-terminal APP soluble fragment of the invention or C-terminal fragment thereof. This kit can then be used to determine, in a mammal, the level of N-terminal truncated and/or post-translationally modified Aβ variant or N-terminal APP soluble fragment of the invention, indicating if the mammal is susceptible to a disease associated with β-amyloid formation and/or aggregation, if the mammal is at risk of developing a disease associated with β-amyloid formation and/or aggregation, if β-amyloid deposits have been cleared, or the level of β-amyloid burden in the mammal. Accordingly, the present invention relates to an antibody that specifically recognizes an N-terminal truncated and/or post-translationally modified Aβ variant or N-terminal APP soluble fragment of the invention for use as a diagnostic or theranostic for determining, in a mammal, susceptibility to a disease associated with β-amyloid formation and/or aggregation, for determining, in a mammal, the risk of developing a disease associated with β-amyloid formation and/or aggregation, for screening of the clearance of β-amyloid deposition in a mammal, or for predicting the level of β-amyloid burden in a mammal. The invention further relates to the use of an antibody that specifically recognizes an N-terminal truncated and/or post-translationally modified Aβ variant or N-terminal APP soluble fragment of the invention for the manufacture of a diagnostic or theranostic kit for determining, in a mammal, susceptibility to a disease associated with β-amyloid formation and/or aggregation, for determining, in a mammal, the risk of developing a disease associated with β-amyloid formation and/or aggregation, for screening of the clearance of β-amyloid deposition in a mammal, or for predicting the level of β-amyloid burden in a mammal.
A preferred kit for carrying out the method of the invention comprises:
- an antibody (primary antibody) which forms an immunological complex with the N-terminal truncated and/or post-translationally modified Aβ variant or the N-terminal APP soluble fragment of the invention to be detected;
- an antibody (secondary antibody) which specifically recognizes the N-terminal truncated and/or post-translationally modified Aβ variant or the N-terminal APP soluble fragment of the invention to be detected;
- a marker either for specific tagging or coupling with said secondary antibody;
- appropriate buffer solutions for carrying out the immunological reaction between the primary antibody and the N-terminal truncated and/or post-translationally modified Aβ variant or the N-terminal APP soluble fragment, between the secondary antibody and the primary antibody-N-terminal truncated and/or post-translationally modified Aβ variant or -N-terminal APP soluble fragment complex and/or between the bound secondary antibody and the marker;
- possibly, for standardization purposes, a purified N-terminal truncated and/or post-translationally modified Aβ peptide or a purified N-terminal APP soluble fragment (or a C-terminal fragment thereof).
The kit of the invention can be used in a method for determining, in a mammal, the susceptibility to a disease associated with β-amyloid formation and/or aggregation, for determining, in a mammal, the risk of developing a disease associated with β-amyloid formation and/or aggregation, for screening of the clearance of β-amyloid deposition in a mammal, or for predicting the level of β-amyloid burden in a mammal. Said method comprises the following steps:
(a) Determining, in said mammal, the amount of N-terminal truncated and/or post-translationally modified Aβ variant or the amount of N-terminal APP soluble fragment of the invention;
(b) Comparing the amount determined in step (a) with the amount of N-terminal truncated and/or post-translationally modified Aβ variant or N-terminal APP soluble fragment of the invention in a control mammal;
(c) Concluding, from the comparison in step (b), whether the mammal is susceptible to a disease associated with β-amyloid formation and/or aggregation, whether the mammal is at risk of developing a disease associated with β-amyloid formation and/or aggregation, whether the β-amyloid deposition in the mammal is cleared, or what the level of β-amyloid burden is in said mammal.

An increase in the level of N-terminal truncated and/or post-translationally modified Aβ variant in the brain of the tested mammal, for example, could be an indication of the mammal being susceptible to or at risk of developing a disease associated with β-amyloid formation and/or aggregation. It could also indicate that the Aβ deposition in the mammal is not yet cleared. Increased levels of N-terminal truncated and/or post-translationally modified Aβ variant in certain body fluids after vaccination or therapy, are an indication of the level of Aβ burden (DeMattos et al., 2002). N-terminal APP soluble fragment will mainly be found in certain body fluids. The presence of these N-terminal APP soluble fragments indicates an aberrant cleavage of APP, resulting in the formation ofN-terminal truncated Aβ variants and, consequently, in an increased susceptibility to or risk of developing a disease associated with β-amyloid formation and/or aggregation by the mammal.

In an embodiment of the invention, the level of N-terminal truncated and/or post-translationally modified Aβ variant or N-terminal APP soluble fragment of the invention can be determined by *in vivo* imaging. Agents or ligands (such as labeld Aβ peptides or antibodies) and methods for *in vivo* imaging of Aβ deposits have been described by Bacskai et al. (2002) and can be adapted for the specific and selective detection of N-terminal truncated and/or post-translationally modified Aβ variant or N-terminal APP soluble fragment of the invention. The level of N-terminal truncated and/or post-translationally modified Aβ variant or N-terminal APP soluble fragment of the invention can be determined *in situ* by non-invasive methods including but not limited to brain imaging methods described by Arbit et al. (1995), Tamada et al. (1995), Wakabayashi et al. (1995), Huang et al. (1996), Sandrock et al. (1996), and Mariani et al. (1997). These *in vivo* imaging methods may allow the localization and quantification of the N-terminal truncated and/or post-translationally modified Aβ variant or N-terminal APP soluble fragment of the invention, for example, by use of labeled antibodies (see below) as ligand, specifically recognizing said N-terminal truncated and/or post-translationally modified Aβ variant or N-terminal APP soluble fragment of the invention. *In vivo* multiphoton microscopy (Bacskai et al., 2001) can be used to image the presence of N-terminal truncated and/or post-translationally modified Aβ variant or N-terminal APP soluble fragment of the invention using labeled antibodies specific for the N-terminal truncated and/or post-translationally modified Aβ variant or the N-terminal APP soluble fragment of the invention. Particular useful as ligand in the above methods might be the heavy chain variable domains (VHH) produced as part of the humoral immune response of camelids. Recombinant VHH selected from 'camelised' human VH libraries could consitute excellent ligands for the *in vivo* imaging of N-terminal truncated and/or post-translationally modified Aβ variant or the N-terminal APP soluble fragment of the invention (Spinelli et al., 2000; Muyldermans, 2001; Cortez-Retamozo et al., 2002). Other agents and methods for *in vivo* detection of Aβ deposits are described by Poduslo et al. (2002), Small (2002), and Petrella et al. (2003).
In another embodiment of the invention, the level of N-terminal truncated and/or post-translationally modified Aβ variant or N-terminal APP soluble fragment of the invention can be determined *in vitro,* in a sample obtained from the mammal. Accordingly, the present invention also relates to a method for determining, in a mammal, the susceptibility to a disease associated with β-amyloid formation and/or aggregation, for determining, in a mammal, the risk of developing a disease associated with β-amyloid formation and/or aggregation, for screening of the clearance of β-amyloid deposition in a mammal, or for predicting the level of β-amyloid burden in a mammal, further characterized in that the amount of N-terminal truncated and/or post-translationally modified Aβ variant or the amount of N-terminal APP soluble fragment of the invention is determined on a sample obtained from said mammal. The invention thus comprises the following steps:
(a) Determining, in a sample obtained from said mammal, the amount of N-terminal truncated and/or post-translationally modified Aβ variant or N-terminal APP soluble fragment of the invention;
(b) Comparing the amount determined in step (a) with the amount of N-terminal truncated and/or post-translationally modified Aβ variant or N-terminal APP soluble fragment of the invention in a tissue sample obtained from a control mammal;
(c) Concluding, from the comparison in step (b), whether the mammal is susceptible to a disease associated with β-amyloid formation and/or aggregation, whether the mammal is at risk of developing a disease associated with β-amyloid formation and/or aggregation, whether the β-amyloid deposition in the mammal is cleared, or what the level of β-amyloid burden is in said mammal.
The present invention further relates to a method for predicting the level of β-amyloid burden in a mammal (DeMattos et al., 2002), said method comprising the following steps:
(a) Administration, to said mammal, of a vaccine composition or a therapeutic composition as described above;
(b) Determining, in a biological fluid sample obtained from said mammal, the amount of N-terminal truncated and/or post-translationally modified β-amyloid variant;
(c) Comparing the amount determined in step (b) with the amount of N-terminal truncated and/or post-translationally modified β-amyloid variant in a biological fluid sample obtained from a control mammal;
(d) Concluding, from the comparison in step (c), what the level of β-amyloid burden is in said mammal.

The level of N-terminal truncated and/or post-translationally modified β-amyloid variant or N-terminal APP soluble fragment of the invention can be detected by an immunoassay as discussed above. Immunoassays for detecting the N-terminal truncated and/or post-translationally modified Aβ variant or N-terminal APP soluble fragment of the invention may be either competitive or non-competitive. Non-competitive immunoassays are assays in which the amount of captured analyte (i.e. the N-terminal truncated and/or post-translationally modified Aβ variant or N-terminal APP soluble fragment of the invention) is directly measured. In competitive assays, the amount of analyte (i.e. the N-terminal truncated and/or post-translationally modified Aβ variant or N-terminal APP soluble fragment of the invention) present in the sample is measured indirectly by measuring the amount of an added (exogenous) analyte displaced (or competed away) from a capture agent (i.e. the antibody) by the analyte present in the sample. In one competition assay, a known amount of (exogenous) N-terminal truncated and/or post-translationally modified Aβ peptide or N-terminal APP soluble fragment of the invention (or a C-terminal fragment thereof) is added to the sample and the sample is then contacted with the antibody. The amount of added (exogenous) N-terminal truncated and/or post-translationally modified Aβ peptide or N-terminal APP soluble fragment of the invention (or C-terminal fragment thereof) bound to the antibody is inversely proportional to the concentration of the N-terminal truncated and/or post-translationally modified Aβ variant or N-terminal APP soluble fragment of the invention in the sample before the exogenous N-terminal truncated and/or post-translationally modified Aβ peptide or N-terminal APP soluble fragment of the invention (or C-terminal fragment thereof) is added.

In a preferred embodiment, the level of the N-terminal truncated and/or post-translationally modified Aβ variant or N-terminal APP soluble fragment of the invention is determined by an immunoassay comprising at least the following steps:
(a) Contacting the N-terminal truncated and/or post-translationally modified Aβ variant or N-terminal APP soluble fragment of the invention present in the sample with an antibody that specifically recognizes the N-terminal truncated and/or post-translationally modified Aβ variant or the N-terminal APP soluble fragment of the invention, under conditions suitable for producing an antigen-antibody complex; and
(b) Detecting the immunological binding that has occurred between the antibody and the N-terminal truncated and/or post-translationally modified Aβ variant or the N-terminal APP soluble fragment of the invention.

In one preferred "sandwich" assay, for example, the antibodies can be bound directly to a solid substrate where they are immobilized. These immobilized antibodies then capture the N-terminal truncated and/or post-translationally modified Aβ variant or N-terminal APP soluble fragment of the invention present in the sample which are subsequently detected with a second antibody. In a preferred embodiment, the N-terminal truncated and/or post-translationally modified Aβ variant or the N-terminal APP soluble fragment of the invention can be detected by a sandwich ELISA comprising the following steps:
(a) Bringing said N-terminal truncated and/or post-translationally modified Aβ variant or said N-terminal APP soluble fragment of the invention present in the sample, into contact with an antibody (primary antibody or capturing antibody) recognizing said N-terminal truncated and/or post-translationally modified Aβ variant or said N-terminal APP soluble fragment of the invention, under conditions being suitable for producing an antigen-antibody complex;
(b) Bringing the complex formed between said N-terminal truncated and/or post-translationally modified Aβ variant or said N-terminal APP soluble fragment of the invention and said primary antibody into contact with another antibody (secondary antibody or detector antibody) specifically recognizing the N-terminal truncated and/or post-translationally modified Aβ variant or the N-terminal APP soluble fragment of the invention, under conditions being suitable for producing an antigen-antibody complex;
(c) Bringing the antigen-antibody complex into contact with a marker either for specific tagging or coupling with said secondary antibody, with said marker being any possible marker known to the person skilled in the art;
(d) Possibly also, for standardization purposes, bringing the antibodies in contact with a purified N-terminal truncated and/or post-translationally modified Aβ peptide or N-terminal APP soluble fragment of the invention (or a C-terminal fragment thereof) reactive with both antibodies.
Advantageously, the secondary antibody itself carries a marker or a group for direct or indirect coupling with a marker.

The antibodies used in the diagnostic or theranostic methods of the present invention may be labeled by an appropriate label. The particular label or detectable group used in the assay is not a critical aspect of the invention, so long as it does not significantly interfere with the specific binding of the antibody used in the assay. The detectable group can be any material having a detectable physical or chemical property. Such detectable labels have been well developed in the field of immunoassays and, in general, almost any label useful in such methods can be applied to the method of the present invention. Thus, a label is any composition detectable by spectroscopic, photochemical, biochemical, immunochemical, electrical, optical, radiological or chemical means. Useful labels in the present invention include but are not limited to magnetic beads (e.g. Dynabeads^{™}), fluorescent dyes (e.g. fluorescein isothiocyanate, texas red, rhodamine), radiolables (e.g. ³H, ¹²⁵I, ³⁵S, ¹⁴C, or ³²P), enzymes (e.g. horseradish peroxidase, alkaline phosphatase and others commonly used in an ELISA), and colorimetric labels such as colloidal gold, colored glass or plastic (e.g. polystyrene, polypropylene, latex, etc.) beads.
The label may be coupled directly or indirectly to the desired component of the assay according to methods well known in the art. As indicated above, a wide variety of labels may be used, with the choice of label depending on the sensitivity required, the ease of conjugation with the compound, stability requirements, the available instrumentation and disposal provisions. Non-radioactive labels are often attached by indirect means. Generally, a ligand molecule (e.g. biotin) is covalently bound to the antibody. The ligand then binds to an anti-ligand (e.g. streptavidin) molecule, which is either inherently detectable or covalently bound to a signal system, such as a detectable enzyme, a fluorescent compound, or a chemiluminescent compound. A number of ligands and anti-ligands can be used. Where a ligand has a natural anti-ligand, for example, biotin, thyroxine, and cortisol, it can be used in conjunction with the labeled, naturally occurring anti-ligands. Alternatively, a haptenic or antigenic compound can be used in combination with an antibody. The antibodies can also be conjugated directly to signal-generating compounds, for example, by conjugation with an enzyme or fluorophore. Enzymes of interest as labels will primarily be hydrolases, particularly phosphatases, esterases and glycosidases, or oxidoreductases, particularly peroxidases. Fluorescent compounds include fluorescein and its derivatives, rhodamine and its derivatives, dansyl, umbelliferone, etc. Chemiluminescent compounds include luciferin, and 2,3-dihydrophtalazinediones, for example, luminol. A review of other labeling or signal producing systems is available in US patent No. 4,391,904.
Means for detecting labels are well known in the art. Thus, for example, where the label is a radioactive label, means for detection include a scintillation counter or photographic film as in autoradiography. Where the label is a fluorescent label, it may be detected by exciting the fluorophore with the appropriate wavelength of light and detecting the resulting fluorescence. The fluorescence may be detected visually, by means of a photographic film, by the use of electronic detectors such as charge coupled devices (CCDs) or photomultipliers and the like. Similarly, enzyme labels may be detected by providing the appropriate substrates for the enzyme and detecting the resulting reaction product. Finally simple colorimetric labels may be detected simply by observing the color associated with the label.

Some assay formats do not require the use of labeled components. For instance, agglutination assays can be used to detect the presence of the target antibodies. In this case, antigen-coated particles are agglutinated by samples comprising the target antibodies. In this format, none of the components need to be labeled and the presence of the target antibody is detected by simple visual inspection.

Throughout this specification and the claims which follow, unless the context requires otherwise, the word "comprise", and variations such as "comprises" and "comprising", will be understood to imply the inclusion of a stated integer or step or group of stated integers or steps but not to the exclusion of any other integer or step or group of integers or steps.

### EXAMPLES

### Example 1: Characterization of the Aβ peptides in fully developed Alzheimer's disease patients and in non-demented patients

### 1. Material and methods

### Patients

All of the brain autopsy materials used in the present study were from the brain bank maintained at INSERM U422 (Lille, France). Five AD cases and five non-demented cases have already been described (Delacourte et al., 2002; Delacourte et al., 1999). The five AD cases fulfilled the neuropathological diagnostic criteria of AD as established by the National Institute on Aging and the Reagan Institute Working Group on diagnostic criteria for the neuropathological assessment of Alzheimer disease (Hyman and Trojanowski, 1997). The five non-demented cases correspond to neurofibrillary stages I and II according to Braak and Braak (1991), or Tau pathology stages 1 to 6 according to Delacourte et al. (1999; 2002) and stage B for amyloid deposition, according to neuropathological staging of Braak and Braak (1991). At autopsy, one brain hemisphere was deep-frozen and used for biochemical analysis, and the other hemisphere was formalin-fixed for both neuropathological examination and histochemistry.

### Antibodies

The amino-terminal regions of Aβ peptides were analyzed with WO2 (Abeta, GmBH, Germany) and 6E10 (Senetek, MO, USA) antibodies. These recognize the amino-acid sequences 5-8 and 4-13, respectively, of Aβ. Aβ x-42 species were studied using 21F12 antibody and ADA42 antiserum. Aβ x-40 species were analyzed with antiserum ADA40 (Delacourte et al., 2002).

### Formic acid isolation of Aβ aggregates and two-dimensional gel electrophoresis

The brain tissue samples from the temporal, frontal, parietal, and occipital cortex were processed as already described (Delacourte et al., 2002). Formic acid (Prolabo, Fontenay s/Bois, France) extracted brain tissue homogenate (100 pL) was evaporated under nitrogen and dissolved in 400 µl of two-dimensional electrophoresis lysis buffer (7 M urea, 2 M thiourea, 4% Triton X-100, 20 mM DTT and 0.6% Pharmalytes^{™} pH 3-10). The sample was sonicated and an IPG strip pH 4-7 (BioRad, Marnes la Coquette, France) was equilibrated with the sample for 15 hrs (Sergeant et al., 2002). Isoelectric focusing was performed using the Protean IEF cell following the manufacturer's (BioRad, Marnes la Coquette, France) instructions. Polypeptides were resolved on Tris-Tricine gels as described earlier (Sergeant et al., 2002). The gels were transferred for immunodetection using the Multiphor transfer unit (Amersham-Pharmacia Biotech, Saclay, France), according to the manufacturer's instructions, or they were stained with Coomassie Brilliant Blue G250 (Sigma, France) for mass spectrometric analyses. Isoelectric points, molecular weights, and the amount of each Aβ peptide variant were determined using Melanie III 2-D gel analysis software (Genebio, Geneva, Switzerland).

### Mass spectrometry characterization

Coomassie Blue-stained polypeptides spots were cut into 1-mm² gel pieces and washed twice with 50% CH₃CN in 25 mM Tris-HCl pH 9. Gel pieces were dehydrated in a Speed-Vac and then in-gel digested overnight with 10 ng of Endoproteinase Lys-C (EC 3.4.21.19, Roche Molecular Biochemicals, Meylan, France) in 3 µl of Tris-HCl pH 9. The resulting digested peptides were recovered in 10 µl of 50% CH₃CN and 1% trifluoroacetic acid (TFA). Samples were then prepared by the dry-droplet method. One ml of the peptide mixture was mixed with freshly dissolved α-cyano-4-hydroxycinnaminic acid 0.5 ml (5 mg/ml in 50% CH₃CN and 0.1 % TFA), and spotted on the sample plate. The dry spot was then washed with 5 µl of 0.1%TFA. Mass spectrometry was performed with a MALDI-TOF Voyager-DE-STR (Applied Biosystems, Palo Alto, CA) set to the following parameters: positive mode, reflector, voltage 20 kV, grid 61%, delayed extraction 90 ns, low mass gate 500 amu. The laser energy required to desorb/ionize samples was kept to a low value, compatible with a good signal/noise ratio. Spectra were calibrated externally using the [M+H⁺] monoisotopic ions from trypsinized lysozyme.

### 2. Characterization of the Aβ peptides in brain obtained from fully developed Alzheimer's disease patients

We first characterized the Aβ peptides found in large amounts in fully developed Alzheimer cases, because of the low amounts of amyloid burden in the brain tissue of infraclinical cases (Delacourte et al. 2002). Aggregates consisting of amyloid-beta peptides (Aβ) in Alzheimer brains were soluble only in pure formic acid as already described (Delacourte et al., 2002; Kalback et al., 2002). Such formic acid-soluble Aβ species were resolved by two-dimensional gel (2-D) electrophoresis and characterized using a panel of specific Aβ antibodies, which revealed that both Aβ x-40 and Aβ x-42 species were present (Fig. 2; Aβ-40 and Aβ-42 panels). Further characterization was performed with brain tissue of Alzheimer's disease (AD) patients in which the total amount of formic acid soluble Aβ enabled subsequent mass spectrometry. Ten Aβ spots were resolved by 2-D electrophoresis (Fig. 2; Coomassie Blue panel), nine of which were identified by mass spectrometry (Table 3). They all corresponded to monomers of Aβ. The immunodetected dimeric species at 8 kDa was present in too low amounts for mass spectrometric analysis. The full-length Aβ peptides corresponded to spot 1 and spot 2 (Table 3). Spots 3-7 and 9-10 corresponded to amino-terminal truncated and post-translationally modified variants of Aβ (Table 3). The major truncated variants consisted of Aβ starting at amino acid positions 2 to 5 and 8 to 10. The post-translational modifications characterized were pyroglutamylation at position 3 and methylation (Table 3). Interestingly, spots 6, 7, 9, and 10 contained similar Aβ variants but were separated as two spots, suggesting that an as-yet-unidentified modification was present.

Coomassie Blue staining enabled a precise quantification of each Aβ species. The full-length Aβ peptides represented only 33% of all Aβ species. The truncated variants thus accounted for more than 65%, among which 16% and 23% corresponded to truncated species starting at residues 4, 5, 8, 9, and 10, respectively. Moreover, the 2-D pattern of Aβ₄₀ as revealed by the ADA40 antiserum completely overlapped the pattern obtained with WO2, which detects the amino-terminal region of Aβ. These results show that the identified truncated Aβ from spots 6, 7, 9, and 10 is derived from the Aβ₄₂ and not Aβ₄₀ species.

### 3. Characterization of Aβ peptides in brain obtained from non-demented patients

Subsequently, the Aβ species that aggregate in the first steps of amyloidosis were investigated in the brain tissue of non-demented patients. We studied fives cases that had traces to low amounts of Aβ. Aβ aggregates were exclusively composed of Aβ₄₂ species (Fig. 3; Aβ₄₀ and Aβ₄₂ panels), indicating a complete absence of Aβ₄₀ species at infraclinical stages of Alzheimer pathology. Antibodies against the N-terminal region of Aβ only detected a single spot corresponding to the full-length Aβ peptide (Fig. 3; N-ter (5-8) and N-ter (4-13) panels). In addition, the Aβ₄₂ specific antibody 21F12 labelled spots 4, 5, 6, and 10 (Fig. 3; Aβ₄₂ panel) as well as dimers. The Aβ₄₂ species in the brains of non-demented patients, as in Alzheimer's brains, correspond to N-terminally truncated variants starting at position 3-pyroglutamyl, 4, 5, 8, and 9. The lack of staining of Aβ dimers with N-terminal Aβ antibodies (Fig. 3 and Fig.4; N-ter (5-8) panel) demonstrates that Aβ dimers are exclusively composed of N-terminally truncated Aβ₄₂ species (Fig. 3 and Fig. 4; Aβ₄₂ panel).

These Aβ₄₂ variants do not result from treatment of brain tissue with formic acid (Delacourte et al., 2002; Kalback et al., 2002), as the treatment of synthetic Aβ peptides 1-40 and 1-42 with formic acid did not generate the truncated variants derived from human brain tissue (Delacourte et al., 2002).

### Example 2: Generation of antibodies that specifically recognize N-terminally truncated (5, 6, 8, 9) β-amyloid and their characterization on brain sample homogenates

### 1. Generation of antibodies

Peptides with different N-terminal truncations were synthesized on a Millepore 9050 synthesizer. An additional glycine residue was added as spacer, and a cysteine residue for coupling at the carboxy-terminus (Table 4) using maleimide chemistry. The peptides were conjugated to KLH (Keyhole Limpet Hemocyanin, Pierce Cat No 77606) and used as immunogen for the generation of an antiserum in rabbits (two rabbits per peptide; Table 5). Titers were determined in ELISA with a peptide conjugated to BSA (ICN, Cat No 810667) for coating and with a HRP-coupled peptide (Horse Radish Peroxidase, Boehringer, Cat No 814407, bridging principle) for detection. Titers are expressed as EC50 values and are comparable in titer to other peptide immunizations (Table 5). The selection of antisera for use in further studies was based on high titer or on specificity.

### 2. Specificity of the antibodies

The specificity of the antibodies obtained was determined by the 'bridging assay'. The principle of the bridging assay is shown in Figure 5. The different peptides conjugated to BSA were used for coating, and detection was performed using HRP-conjugated peptides (Table 4). As shown in Figure 6, the antisera were only reactive with their corresponding peptide while not being reactive with the other peptides, which have an overlapping sequence. This suggests that the antibodies are primarily directed to the N-terminus of the peptide.

### 3. Detection of N-terminally truncated peptides in human brain extract

These crude antisera were further used in a bridging assay to determine the amount of N-terminally truncated peptides in formic acid brain extracts from control cases (2), infraclinical stage 1 and 2, and end-stage AD (S10) (Table 6; Delacourte et al., 2002).

The amount of Aβ(1-42) was measured with the monoclonal antibody 3D6 (HS-format of K-1080, Innogenetics, Ghent, Belgium). An overview of the different N-terminally truncated Aβ present in brain extracts from the different AD stages is given in Table 6 and Figure 7. Three cases have high amounts of β-amyloid (1-42): S1 (Pet), S2 (Mag), and S10 (Fra). These cases also have a substantial amount of N-terminally truncated Aβ42 species. In particular, Rb 470, specific for Aβ N-terminal truncated at amino acid 8, is highly reactive. These N-terminally truncated species are also present in cases with infraclinical stages S 1 (Rou2) and S2 (Ben), in which there is no detectable β-amyloid (1-42). This suggests that truncated species of Aβ42 are the earliest biochemical markers of the disease.

### Example 3: Detection of Aβ variants in CSF obtained from control patients and AD patients

### 1. Preparation of ProteinChip^{®} arrays and SELDI-TOF analysis

CSF samples were subjected to antibody capture using a monoclonal antibody specific for the C-terminus (especially for x-42) of human Aβ peptides (4D7A3; Innogenetics Cat. no. BR032D). Affinity arrays were prepared by coupling the 4D7A3 antibody or a control mouse IgG onto a PS20 ProteinChip^{®} array (Ciphergen Cat. no. C553-0045). After pre-wetting the ProteinChip array with 5 µl PBS, a 3-µl aliquot of a 1 mg/ml antibody in PBS was incubated in a humidity chamber for 3 hours at room temperature to allow covalent binding to the array. Washing of the array was performed on spot, twice with PBS/0.1% Triton X-100 and once with PBS. Unreacted sites were then blocked by incubating 3 µl of 10 mg/ml BSA in PBS for 2 hours at room temperature. Washing off the excess of BSA occurred twice with PBS/0.5% Triton X-100 followed by three washes with PBS. The arrays were then placed in a 96-well bioprocessor where volumes of 100 µl CSF were applied. The samples were incubated overnight at 4°C with constant shaking. After discarding the CSF, the ProteinChip arrays were removed from the bioprocessor and washed on spot three times with PBS/0.1% Triton X-100 followed by three washes with PBS and two washes with 5 mM Hepes. After the arrays had dried, 0.8 µl of a 20% saturated solution of α-cyano-4-hydroxycinnamic acid (CHCA; Ciphergen Cat. no. C300-0001) in 0.5% (v/v) trifluoroacetic acid (TFA), 50% (v/v) acetonitrile (ACN) was applied to each spot. Mass analysis was performed on a ProteinChip reader (model PBS II; Ciphergen).

### 2. Analysis of CSF samples obtained from patients clinically diagnosed with different neurological disease or different stages of AD development

A study was carried out on CSF samples obtained from 161 patients with different neurological diseases. The following patient groups were distinguished based on clinical parameters (Table 7): control patients (control), patients suffering from dementia with Lewy bodies (DLB; McKeith et al., 1996), patients with mild cognitive impairment who later developed AD (MCI-AD; Petersen et al., 1999), cognitively impaired patients who did not develop AD (Cogn; Wahlund et al., 2003), patients suffering from Parkinson's disease (PD; Langston et al., 1992), and patients suffering from Alzheimer's disease (McKhann et al., 1984). Patients suffering from AD were further divided into three different groups, based on their MMSE scores (Folstein et al., 1975): Mild AD (MMSE 24-28), Mod AD (MMSE 17-23) and Sev AD (MMSE 2-16). Three CSF samples were selected from each neurological disease group (at least 200 µl available for analysis). These CSF samples were run on an immuno-chip from Ciphergen coated with 4D7A3, an antibody specific for the carboxy-terminus of Aβ42. An analysis of those Aβ42 peptides that are different from the 1-42 is shown in Table 8. The data suggest that oxidized N-terminally truncated Aβ peptides 8-42 and 5-42 are detected in the CSF and are specific for AD, even in the very early stages of AD, when no clinical symptoms of AD are yet observed.

### Example 4: Analysis of Aβ variants in model systems

The presence or absence of N-terminally truncated Aβ species is studied in the brain tissue or any tissue of any animal sources such as transgenic mice containing the human mutated amyloid precursor protein (APP) gene (Games et al., 1995; Hsiao et al., 1996, Sturchler-Pierrat et al., 1997; Moechars et al., 1999; Takeuchi et al., 2000; Kawarabayashi et al., 2001; for a more complete list of available transgenic models see http://www.alzforum.org/home.asp). As amyloid deposition in these transgenic animals is age-dependent, brains need to be examined at different stages, for instance, at very early stages (6 months), early stages (9 months), middle stages (15 months), and late stages (21 months) in the transgenic mice of Moechars et al. (1999).

The methodology used is similar to the method described earlier for human brain tissue. The tissue is homogenized in Tris HCl pH 6.8 with 2% of Triton X-100 at a ratio of 1 per 10 volumes of homogenizing buffer. The homogenate is centrifuged at 100,000 g for 1 hour at 4°C. The supernatant is collected and the pellet is homogenized in 100 µl of pure formic acid and sonicated. The formic acid is evaporated under nitrogen and homogenized in the 2-D lysis buffer. Two-dimensional gel electrophoresis and Western blotting is performed as described for the human brain tissue.

Evidence of the presence of N-terminally truncated Aβ species is shown by spots detected exactly at the same molecular weights and isoelectric points as the N-terminally truncated Aβ species characterized in the human brain. The rodent Aβ sequence is different from that of the human sequence (Sergeant et al., 2003). Accordingly, the presence of N-terminally truncated Aβ peptides from human or from rodent origin can be shown by Aβ peptide spots at molecular weights and isoelectric points close to the theoretical isoelectric points of the respective endogenous sequence of the Aβ peptides.

### TABLES

**Table 1. N-terminal truncated and/or post-translationally modified Aβ peptides comprised in the preparation of the invention.**

| **Position in βA** | **Modification** | **Sequence** | **SEQ ID NO** |
|---|---|---|---|
| 1-4 | Methyl | DAEF | 1 |
| 1-5 | Methyl | DAEFR | 2 |
| 1-6 | Methyl | DAEFRH | 3 |
| 1-7 | Methyl | DAEFRHD | 4 |
| 1-8 | Methyl | DAEFRHDS | 5 |
| 1-9 | Methyl | DAEFRHDSG | 6 |
| 1-10 | Methyl | DAEFRHDSGY | 7 |
| 1-11 | Methyl | DAEFRHDSGYE | 8 |
| 1-12 | Methyl | DAEFRHDSGYEV | 9 |
| 1-13 | Methyl | DAEFRHDSGYEVH | 10 |
| 1-14 | Methyl | DAEFRHDSGYEVHH | 11 |
| 1-15 | Methyl | DAEFRHDSGYEVHHQ | 12 |
| 1-16 | Methyl | DAEFRHDSGYEVHHQK | 13 |
| 2-5 | None/methyl | AEFR | 14 |
| 2-6 | None/methyl | AEFRH | 15 |
| 2-7 | None/methyl | AEFRHD | 16 |
| 2-8 | None/methyl | AEFRHDS | 17 |
| 2-9 | None/methyl | AEFRHDSG | 18 |
| 2-10 | None/methyl | AEFRHDSGY | 19 |
| 2-11 | None/methyl | AEFRHDSGYE | 20 |
| 2-12 | None/methyl | AEFRHDSGYEV | 21 |
| 2-13 | None/methyl | AEFRHDSGYEVH | 22 |
| 2-14 | None/methyl | AEFRHDSGYEVHH | 23 |
| 2-15 | None/methyl | AEFRHDSGYEVHHQ | 24 |
| 2-16 | None/methyl | AEFRHDSGYEVHHQK | 25 |
| 2-17 | None/methyl | AEFRHDSGYEVHHQKL | 26 |
| 3-6 | None | EFRH | 27 |
| 3-7 | None | EFRHD | 28 |
| 3-8 | None | EFRHDS | 29 |
| 3-9 | None | EFRHDSG | 30 |
| 3-10 | None | EFRHDSGY | 31 |
| 3-11 | None | EFRHDSGYE | 32 |
| 3-12 | None | EFRHDSGYEV | 33 |
| 3-13 | None | EFRHDSGYEVH | 34 |
| 3-14 | None | EFRHDSGYEVHH | 35 |
| 3-15 | None | EFRHDSGYEVHHQ | 36 |
| 3-16 | None | EFRHDSGYEVHHQK | 37 |
| 3-17 | None | EFRHDSGYEVHHQKL | 38 |
| 3-18 | None | EFRHDSGYEVHHQKLV | 39 |
| 3-6 | Pyroglutamyl | PyrE-FRH | 40 |
| 3-7 | Pyroglutamyl | PyrE-FRHD | 41 |
| 3-8 | Pyroglutamyl | PyrE-FRHDS | 42 |
| 3-9 | Pyroglutamyl | PyrE-FRHDSG | 43 |
| 3-10 | Pyroglutamyl | PyrE-FRHDSGY | 44 |
| 3-11 | Pyroglutamyl | PyrE-FRHDSGYE | 45 |
| 3-12 | Pyroglutamyl | PyrE-FRHDSGYEV | 46 |
| 3-13 | Pyroglutamyl | PyrE-FRHDSGYEVH | 47 |
| 3-14 | Pyroglutamyl | PyrE-FRHDSGYEVHH | 48 |
| 3-15 | Pyroglutamyl | PyrE-FRHDSGYEVHHQ | 49 |
| 3-16 | Pyroglutamyl | PyrE-FRHDSGYEVHHQK | 50 |
| 3-17 | Pyroglutamyl | PyrE-FRHDSGYEVHHQKL | 51 |
| 3-18 | Pyroglutamyl | PyrE-FRHDSGYEVHHQKLV | 52 |
| 4-7 | None/methyl | FRHD | 53 |
| 4-8 | None/methyl | FRHDS | 54 |
| 4-9 | None/methyl | FRHDSG | 55 |
| 4-10 | None/methyl | FRHDSGY | 56 |
| 4-11 | None/methyl | FRHDSGYE | 57 |
| 4-12 | None/methyl | FRHDSGYEV | 58 |
| 4-13 | None/methyl | FRHDSGYEVH | 59 |
| 4-14 | None/methyl | FRHDSGYEVHH | 60 |
| 4-15 | None/methyl | FRHDSGYEVHHQ | 61 |
| 4-16 | None/methyl | FRHDSGYEVHHQK | 62 |
| 4-17 | None/methyl | FRHDSGYEVHHQKL | 63 |
| 4-18 | None/methyl | FRHDSGYEVHHQKLV | 64 |
| 4-19 | None/methyl | FRHDSGYEVHHQKLVF | 65 |
| 5-8 | None/methyl | RHDS | 66 |
| 5-9 | None/methyl | RHDSG | 67 |
| 5-10 | None/methyl | RHDSGY | 68 |
| 5-11 | None/methyl | RHDSGYE | 69 |
| 5-12 | None/methyl | RHDSGYEV | 70 |
| 5-13 | None/methyl | RHDSGYEVH | 71 |
| 5-14 | None/methyl | RHDSGYEVHH | 72 |
| 5-15 | None/methyl | RHDSGYEVHHQ | 73 |
| 5-16 | None/methyl | RHDSGYEVHHQK | 74 |
| 5-17 | None/methyl | RHDSGYEVHHQKL | 75 |
| 5-18 | None/methyl | RHDSGYEVHHQKLV | 76 |
| 5-19 | None/methyl | RHDSGYEVHHQKLVF | 77 |
| 5-20 | None/methyl | RHDSGYEVHHQKLVFF | 78 |
| 6-9 | None/methyl | HDSG | 79 |
| 6-10 | None/methyl | HDSGY | 80 |
| 6-11 | None/methyl | HDSGYE | 81 |
| 6-12 | None/methyl | HDSGYEV | 82 |
| 6-13 | None/methyl | HDSGYEVH | 83 |
| 6-14 | None/methyl | HDSGYEVHH | 84 |
| 6-15 | None/methyl | HDSGYEVHHQ | 85 |
| 6-16 | None/methyl | HDSGYEVHHQK | 86 |
| 6-17 | None/methyl | HDSGYEVHHQKL | 87 |
| 6-18 | None/methyl | HDSGYEVHHQKLV | 88 |
| 6-19 | None/methyl | HDSGYEVHHQKLVF | 89 |
| 6-20 | None/methyl | HDSGYEVHHQKLVFF | 90 |
| 6-21 | None/methyl | HDSGYEVHHQKLVFFA | 91 |
| 7-10 | None/methyl | DSGY | 92 |
| 7-11 | None/methyl | DSGYE | 93 |
| 7-12 | None/methyl | DSGYEV | 94 |
| 7-13 | None/methyl | DSGYEVH | 95 |
| 7-14 | None/methyl | DSGYEVHH | 96 |
| 7-15 | None/methyl | DSGYEVHHQ | 97 |
| 7-16 | None/methyl | DSGYEVHHQK | 98 |
| 7-17 | None/methyl | DSGYEVHHQKL | 99 |
| 7-18 | None/methyl | DSGYEVHHQKLV | 100 |
| 7-19 | None/methyl | DSGYEVHHQKLVF | 101 |
| 7-20 | None/methyl | DSGYEVHHQKLVFF | 102 |
| 7-21 | None/methyl | DSGYEVHHQKLVFFA | 103 |
| 7-22 | None/methyl | DSGYEVHHQKLVFFAE | 104 |
| 8-11 | None | SGYE | 105 |
| 8-12 | None | SGYEV | 106 |
| 8-13 | None | SGYEVH | 107 |
| 8-14 | None | SGYEVHH | 108 |
| 8-15 | None | SGYEVHHQ | 109 |
| 8-16 | None | SGYEVHHQK | 110 |
| 8-17 | None | SGYEVHHQKL | 111 |
| 8-18 | None | SGYEVHHQKLV | 112 |
| 8-19 | None | SGYEVHHQKLVF | 113 |
| 8-20 | None | SGYEVHHQKLVFF | 114 |
| 8-21 | None | SGYEVHHQKLVFFA | 115 |
| 8-22 | None | SGYEVHHQKLVFFAE | 116 |
| 8-23 | None | SGYEVHHQKLVFFAED | 117 |
| 9-12 | None | GYEV | 118 |
| 9-13 | None | GYEVH | 119 |
| 9-14 | None | GYEVHH | 120 |
| 9-15 | None | GYEVHHQ | 121 |
| 9-16 | None | GYEVHHQK | 122 |
| 9-17 | None | GYEVHHQKL | 123 |
| 9-18 | None | GYEVHHQKLV | 124 |
| 9-19 | None | GYEVHHQKLVF | 125 |
| 9-20 | None | GYEVHHQKLVFF | 126 |
| 9-21 | None | GYEVHHQKLVFFA | 127 |
| 9-22 | None | GYEVHHQKLVFFAE | 128 |
| 9-23 | None | GYEVHHQKLVFFAED | 129 |
| 9-24 | None | GYEVHHQKLVFFAEDV | 130 |
| 10-13 | None | YEVH | 131 |
| 10-14 | None | YEVHH | 132 |
| 10-15 | None | YEVHHQ | 133 |
| 10-16 | None | YEVHHQK | 134 |
| 10-17 | None | YEVHHQKL | 135 |
| 10-18 | None | YEVHHQKLV | 136 |
| 10-19 | None | YEVHHQKLVF | 137 |
| 10-20 | None | YEVHHQKLVFF | 138 |
| 10-21 | None | YEVHHQKLVFFA | 139 |
| 10-22 | None | YEVHHQKLVFFAE | 140 |
| 10-23 | None | YEVHHQKLVFFAED | 141 |
| 10-24 | None | YEVHHQKLVFFAEDV | 142 |
| 10-25 | None | YEVHHQKLVFFAEDVG | 143 |
| 1-42 | Methyl | DAEFRHDSGYEVHHQLVFFAEDVGSNKG AIIGLMVGGVVIA | 144 |
| 2-42 | None/methyl | AEFRHDSGYEVHHQLVFFAEDVGSNKGA IIGLMVGGVVIA | 145 |
| 3-42 | | EFRHDSGYEVHHQLVFFAEDVGSNKGAII GLMVGGVVIA | 146 |
| 3-42 | Pyroglutamyl | PyrE-FRHDSGYEVHHQLVFFAEDVGSNK GAIIGLMVGGVVIA | 147 |
| 4-42 | None/methyl | FRHDSGYEVHHQLVFFAEDVGSNKGAIIG LMVGGVVIA | 148 |
| 5-42 | None/methyl | RHDSGYEVHHQLVFFAEDVGSNKGAIIGL MVGGVVIA | 149 |
| 6-42 | None/methyl | HDSGYEVHHQLVFFAEDVGSNKGAIIGL MVGGVVIA | 150 |
| 7-42 | None/methyl | DSGYEVHHQLVFFAEDVGSNKGAIIGLM VGGVVIA | 151 |
| 8-42 | | SGYEVHHQLVFFAEDVGSNKGAIIGLMV GGVVIA | 152 |
| 9-42 | | GYEVHHQLVFFAEDVGSNKGAIIGLMVG GVVIA | 153 |
| 10-42 | | YEVHHQLVFFAEDVGSNKGAIIGLMVGG VVIA | 154 |
| 1-40 | Methyl | DAEFRHDSGYEVHHQLVFFAEDVGSNKG AIIGLMVGGVV | 155 |
| 2-40 | None/methyl | AEFRHDSGYEVHHQLVFFAEDVGSNKGA IIGLMVGGVV | 156 |
| 3-40 | | EFRHDSGYEVHHQLVFFAEDVGSNKGAII GLMVGGVV | 157 |
| 3-40 | Pyroglutamyl | PyrE-FRHDSGYEVHHQLVFFAEDVGSNK GAIIGLMVGGVV | 158 |
| 4-40 | None/methyl | FRHDSGYEVHHQLVFFAEDVGSNKGAIIG LMVGGVV | 159 |
| 5-40 | None/methyl | RHDSGYEVHHQLVFFAEDVGSNKGAIIGL MVGGVV | 160 |
| 6-40 | None/methyl | HDSGYEVHHQLVFFAEDVGSNKGAIIGL MVGGVV | 161 |
| 7-40 | None/methyl | DSGYEVHHQLVFFAEDVGSNKGAIIGLM VGGVV | 162 |
| 8-40 | | SGYEVHHQLVFFAEDVGSNKGAIIGLMV GGVV | 163 |
| 9-40 | | GYEVHHQLVFFAEDVGSNKGAIIGLMVG GVV | 164 |
| 10-40 | | YEVHHQLVFFAEDVGSNKGAIIGLMVGG VV | 165 |

**Table 2. C-terminal fragments of the N-terminal APP soluble fragments of the invention.**

| **Positions: APP - β-amyloid** | **Sequence** | **SEQ ID NO** |
|---|---|---|
| 669-1 | VKMD | 166 |
| 668-1 | EVKMD | 167 |
| 667-1 | SEVKMD | 168 |
| 666-1 | ISEVKMD | 169 |
| 656-1 | EISEVKMD | 170 |
| 664-1 | EEISEVKMD | 171 |
| 663-1 | TEEISEVKMD | 172 |
| 662-1 | KTEEISEVKMD | 173 |
| 661-1 | IKTEEISEVKMD | 174 |
| 660-1 | NIKTEEISEVKMD | 175 |
| 659-1 | TNIKTEEISEVKMD | 176 |
| 658-1 | LTNIKTEEISEVKMD | 177 |
| 657-1 | GLTNIKTEEISEVKMD | 178 |
| 670-2 | KMDA | 179 |
| 669-2 | VKMDA | 180 |
| 668-2 | EVKMDA | 181 |
| 667-2 | SEVKMDA | 182 |
| 666-2 | ISEVKMDA | 183 |
| 665-2 | EISEVKMDA | 184 |
| 664-2 | EEISEVKMDA | 185 |
| 663-2 | TEEISEVKMDA | 186 |
| 662-2 | KTEEISEVKMDA | 187 |
| 661-2 | IKTEEISEVKMDA | 188 |
| 660-2 | NIKTEEISEVKMDA | 189 |
| 659-2 | TNIKTEEISEVKMDA | 190 |
| 658-2 | LTNIKTEEISEVKMDA | 191 |
| 671-3 | MDAE | 192 |
| 670-3 | KMDAE | 193 |
| 669-3 | VKMDAE | 194 |
| 668-3 | EVKMDAE | 195 |
| 667-3 | SEVKMDAE | 196 |
| 666-3 | ISEVKMDAE | 197 |
| 665-3 | EISEVKMDAE | 198 |
| 664-3 | EEISEVKMDAE | 199 |
| 663-3 | TEEISEVKMDAE | 200 |
| 662-3 | KTEEISEVKMDAE | 201 |
| 661-3 | IKTEEISEVKMDAE | 202 |
| 660-3 | NIKTEEISEVKMDAE | 203 |
| 659-3 | TNIKTEEISEVKMDAE | 204 |
| 672-4 | DAEF | 1 |
| 671-4 | MDAEF | 205 |
| 670-4 | KMDAEF | 206 |
| 669-4 | VKMDAEF | 207 |
| 668-4 | EVKMDAEF | 208 |
| 667-4 | SEVKMDAEF | 209 |
| 666-4 | ISEVKMDAEF | 210 |
| 665-4 | EISEVKMDAEF | 211 |
| 664-4 | EEISEVKMDAEF | 212 |
| 663-4 | TEEISEVKMDAEF | 213 |
| 662-4 | KTEEISEVKMDAEF | 214 |
| 661-4 | IKTEEISEVKMDAEF | 215 |
| 660-4 | NIKTEEISEVKMDAEF | 216 |
| 673-5 | AEFR | 14 |
| 672-5 | DAEFR | 2 |
| 671-5 | MDAEFR | 217 |
| 670-5 | KMDAEFR | 218 |
| 669-5 | VKMDAEFR | 219 |
| 668-5 | EVKMDAEFR | 220 |
| 667-5 | SEVKMDAEFR | 221 |
| 666-5 | ISEVKMDAEFR | 222 |
| 665-5 | EISEVKMDAEFR | 223 |
| 664-5 | EEISEVKMDAEFR | 224 |
| 663-5 | TEEISEVKMDAEFR | 225 |
| 662-5 | KTEEISEVKMDAEFR | 226 |
| 661-5 | IKTEEISEVKMDAEFR | 227 |
| 674-6 | EFRH | 27 |
| 673-6 | AEFRH | 15 |
| 672-6 | DAEFRH | 3 |
| 671-6 | MDAEFRH | 228 |
| 670-6 | KMDAEFRH | 229 |
| 669-6 | VKMDAEFRH | 230 |
| 668-6 | EVKMDAEFRH | 231 |
| 667-6 | SEVKMDAEFRH | 232 |
| 666-6 | ISEVKMDAEFRH | 233 |
| 665-6 | EISEVKMDAEFRH | 234 |
| 664-6 | EEISEVKMDAEFRH | 235 |
| 663-6 | TEEISEVKMDAEFRH | 236 |
| 662-6 | KTEEISEVKMDAEFRH | 237 |
| 675-7 | FRHD | 53 |
| 674-7 | EFRHD | 28 |
| 673-7 | AEFRHD | 16 |
| 672-7 | DAEFRHD | 4 |
| 671-7 | MDAEFRHD | 238 |
| 670-7 | KMDAEFRHD | 239 |
| 669-7 | VKMDAEFRHD | 240 |
| 668-7 | EVKMDAEFRHD | 241 |
| 667-7 | SEVKMDAEFRHD | 242 |
| 666-7 | ISEVKMDAEFRHD | 243 |
| 665-7 | EISEVKMDAEFRHD | 244 |
| 664-7 | EEISEVKMDAEFRHD | 245 |
| 663-7 | TEEISEVKMDAEFRHD | 246 |
| 676-8 | RHDS | 66 |
| 675-8 | FRHDS | 54 |
| 674-8 | EFRHDS | 29 |
| 673-8 | AEFRHDS | 17 |
| 672-8 | DAEFRHDS | 5 |
| 671-8 | MDAEFRHDS | 247 |
| 670-8 | KMDAEFRHDS | 248 |
| 669-8 | VKMDAEFRHDS | 249 |
| 668-8 | EVKMDAEFRHDS | 250 |
| 667-8 | SEVKMDAEFRHDS | 251 |
| 666-8 | ISEVKMDAEFRHDS | 252 |
| 665-8 | EISEVKMDAEFRHDS | 253 |
| 664-8 | EEISEVKMDAEFRHDS | 254 |
| 677-9 | HDSG | 79 |
| 676-9 | RHDSG | 67 |
| 675-9 | FRHDSG | 55 |
| 674-9 | EFRHDSG | 30 |
| 673-9 | AEFRHDSG | 18 |
| 672-9 | DAEFRHDSG | 6 |
| 671-9 | MDAEFRHDSG | 255 |
| 670-9 | KMDAEFRHDSG | 256 |
| 669-9 | VKMDAEFRHDSG | 257 |
| 668-9 | EVKMDAEFRHDSG | 258 |
| 667-9 | SEVKMDAEFRHDSG | 259 |
| 666-9 | ISEVKMDAEFRHDSG | 260 |
| 665-9 | EISEVKMDAEFRHDSG | 261 |

**Table 3. Digested peptides of Aβ variants present in Alzheimer's disease patients**

| **Spot** | **Aβ proposed identity¹** | **Theoretical mass** | **Observed mass** | **Relative amount²** | **Theoretical pI** | **Observed pI** |
|---|---|---|---|---|---|---|
| 1 | 1-16 | 1954.879 | 1954.875 | 23% | 5.31 | 5.3 |
| | 1-16+CH₃ | 1968.905 | 1968.863 | - | 5.31 | 5.3 |
| 2 | 1-16 | 1954.879 | 1954.875 | 10% | 5.31 | 5.3 |
| | 1-16+CH₃ | 1968.905 | 1968.863 | - | 5.31 | 5.3 |
| 3 | 2-16 | 1839.852 | 1839.851 | 6% | 5.78 | 5.8 |
| | 2-16+CH₃ | 1853.878 | 1853.854 | - | 5.78 | 5.8 |
| | (3-16) | 1768.815 | 1768.804 | - | 5.78 | 5.8 |
| 4 | pyrE 3-16 | 1751.784 | 1750.790 | 10% | 6.27 | 5.9 |
| | (2-16) | 1839.852 | 1839.833 | - | 5.78 | 5.9 |
| 5 | pyrE 3-16 | 1751.784 | 1750.881 | 8% | 6.27 | 6.3 |
| 6 | 8-16 | 1084.517 | 1084.557 | 10% | 5.96 | 6.0 |
| | 9-16 | 997.485 | 998.525 | - | 6.01 | 6.0 |
| 7 | 8-16 | 1084.517 | 1084.518 | 13% | 5.96 | 6.1 |
| | 9-16 | 997.485 | 997.477 | - | 6.01 | 6.1 |
| | 10-16 | 940.463 | 940.460 | - | 6.01 | 6.1 |
| 9&10 | 4-16 | 1639.772 | 1639.848 | 16% | 6.27 | 6.3 |
| | 4-16+CH₃ | 1653.798 | 1653.859 | - | 6.27 | 6.3 |
| | 5-16 | 1492.704 | 1492.770 | - | | |

| | | | | | | |
|---|---|---|---|---|---|---|
| ¹Methylated fragments are indicated with a CH₃. PyrE corresponds to a pyroglutamyl residue at the N-terminus of the identified fragment. The peptide corresponding to amino acid sequence 17-28 of Aβ was found in all spots (not shown). ²The relative amount corresponds to the quantification using Melanie III software on Coomassie stained gels. | | | | | | |

**Table 4. Overview of the peptides used for the generation of antibodies specific for N-terminally truncated Aβ and their corresponding BSA- and HRP-conjugates (Innogenetics numbering).**

| **Truncation** | **Sequence** | **Aβ** | **APP770** | **Cys-pept** | **HRP-pept** | **BSA-pept** | **SEQ ID** |
|---|---|---|---|---|---|---|---|
| **Trunc 5** | RHDSGYEV- | 5-12 | 676-683 | IGP-2121 | PG-124 | PG-129 | 70 |
| **Trunc 6** | HDSGYEVH- | 6-13 | 677-684 | IGP-2120 | PG-123 | PG-128 | 83 |
| **Trunc 8** | SGYEVHHQ- | 8-15 | 679-686 | IGP-2119 | PG-122 | PG-127 | 109 |
| **Trunc 9** | GYEVHHQK- | 9-16 | 680-687 | IGP-2122 | PG-125 | PG-130 | 122 |

**Table 5. Overview of the rabbits immunized with KLH-coupled peptides. Two rabbits were immunized with each peptide. Antisera selected for further studies are indicated.**

| **Immunogen** | **Peptide** | **Rabbit (Rb)** | **Titer** | **Selection criterum** | **Further studies** |
|---|---|---|---|---|---|
| Trunc 5 | IGP-2121 | 466 | 13145 | Highest titer | Rb466 |
| | | 467 | 45115 | | |
| Trunc 6 | IGP-2120 | 468 | 42623 | Highest titer | Rb469 |
| | | 469 | 31291 | | |
| Trunc 8 | IGP-2119 | 470 | 15235 | Specificity | Rb470 |
| | | 471 | 26627 | | |
| Trunc 9 | IGP-2122 | 472 | 22735 | Specificity | Rb472 |
| | | 473 | 45855 | | |

**Table 6. Analysis of the amount of Aβ(1-42) and N-terminally truncated Aβ in formic acid brain extracts obtained from different cases (control, infraclinical stages 1 and 2, and end-stage AD). Clinical parameters as well as the stage according to Delacourte et al. (2002) are indicated.**

| **Cases** | **Stage** | **Age** | **Clin Diagnosis** | **3D6 (1-42)(pg/ml)** | **Rb470 (8-42)(1/20)*** |
|---|---|---|---|---|---|
| Duc | S0 | 26 | Control | <125 | 0.073 |
| Cru | S0 | 43 | Control | <125 | 0.55 |
| Rou2 | S1 | 78 | Control | <125 | 0.189 |
| Pet | S1 | 83 | Control | >10000 | 0.751 |
| Cro | S2 | 72 | Control | <125 | 0.05 |
| Ben | S2 | 89 | Control | <125 | 0.263 |
| Mag | S2 | 95 | Control | >10000 | 0.574 |
| Fra | S10 | 64 | Prob AD | >10000 | 1.225 |

| | | | | | |
|---|---|---|---|---|---|
| * OD value of a 1/20 dilution of the formic acid extract after evaporation and solubilization in PBS. | | | | | |

**Table 7. Summary of the demographic and CSF data of the patient groups clinically diagnosed with different neurological diseases that were analyzed in Example 3.**

| **Group** | **n** | **Age** | **MMSE** | **Tau (pg/ml)** | **Phosphotau (pg/ml)** | **Aβ1-42 (pg/ml)** |
|---|---|---|---|---|---|---|
| | | (med; min-max) | (med; min-max) | (Avg, SD) | (Avg, SD) | (Avg, SD) |
| Control | 29 | 66 (61-80) | 30 (28-30) | 356 (152) | 57 (23) | 711 (164) |
| Mild AD | 22 | 77 (68-87) | 26.5 (24-28) | 701 (216) | 98 (42) | 382 (80) |
| Mod AD | 22 | 78.5 (49-89) | 20.5 (17-23) | 748 (234) | 95 (36) | 361 (115) |
| Sev AD | 22 | 76.5 (61-84) | 13.5 (2-16) | 819 (359) | 105 (46) | 367 (61) |
| DLB | 12 | 77 (65-87) | 23 (17-27) | 388 (134) | 57 (15) | 466 (34) |
| MCI-AD | 14 | 78 (65-78) | 29 (28-30) | 654 (191) | 97 (30) | 503 (54) |
| Cogn | 25 | 63 (39-92) | 30 (25-30) | 257 (126) | 44 (18) | 614 (163) |
| PD | 15 | 71 (59-82) | 29 (25-30) | 306 (65) | 54 (10) | 671 (142) |

**Table 8. Molecular mass of the peaks observed on a PS-20 chip from Ciphergen coated with the carboxy-terminal 42 specific antibody 4D7A3. Experiments were done in duplicate on 100 µl of CSF.**

| **Aβ sequence** | | **11-42** | **10-42ox** | **8-42ox** | **5-42ox** |
|---|---|---|---|---|---|
| **Expected Mr** | | **3335.92** | **3515.1** | **3659.23** | **4067.64** |
| **Group** | Nr | | | | |
| **Control** | 150 | -/- | 3515.9/- | -/- | -/- |
| | 148 | -/3335.7 | 3514.6/3514.9 | 3653.2/- | -/- |
| | 147 | -/- | -/- | -/- | -/- |
| | | | | | |
| **Cogn** | 87 | -/- | 3515.1/3516.1 | -/- | -/- |
| | 78 | 3337.2/- | 3518.6/3519.4 | -/- | -/- |
| | 69 | -/- | 3516.8/3516.6 | -/- | -/- |
| | | | | | |
| **MCI-AD** | 111 | -/- | 3515.6/- | 3652.4/- | -/- |
| | 110 | -/- | 3515.3/- | 3651.9/- | -/- |
| | 112 | -/- | -/- | -/- | 4073.2/4071.8 |
| | | | | | |
| **Mild AD** | 54 | -/- | -/- | -/- | -/- |
| | 57 | -/- | -/- | 3652.6/3651.8 | 4072.2/- |
| | 64 | -/- | -/- | 3652.5/3654.5 | -/- |
| | | | | | |
| **Mod AD** | 40 | -/- | -/- | 3653.4/3652.8 | -/4074.4 |
| | 47 | -/- | 3516.5/3515.5 | 3654.7/- | -/- |
| | 15 | -/- | -/- | 3652.8/3653.7 | -/4071.6 |
| | | | | | |
| **Sev AD** | 31 | -/- | 3516.3/3523.5 | 3652.3/3652.7 | -/4072.4 |
| | 32 | -/- | -/3515.5 | -/3652.1 | -/4071.9 |
| | 22 | -/- | 3516.0/3523.2 | 3653.8/3653.3 | -/4072.4 |
| | | | | | |
| **DLB** | 94 | -/- | -/- | -/- | -/- |
| | 101 | -/- | -/- | -/- | -/- |
| | 103 | -/- | 3517.9/- | -/- | -/- |

### REFERENCES

Arbit E., Cheung N.K., Yeh S.D., Daghighian F., Shang J.J., Cordon-Cardo C., Pentlow K., Canete A., Finn R., Larson S.M. (1995) Quantitative studies of monoclonal antibody targeting to disialogangliosid GD2 in human brain tumors. Eur. J. Nucl. Med. 22: 419-426.
Bacskai B.J., Kajdasz S.T., Christie R.H., Carter C., Games D., Seubert P., Schenk D., Hyman B.T. (2001) Imaging of amyloid-beta deposits in brains of living mice permits direct observation of clearance of plaques with immunotherapy. Nature Med. 7: 369-372.
Bacskai B.J., Klunk W.E., Mathis C.A., Hyman B.T. (2002) Imaging amyloid-beta deposits in vivo. J. Cereb. Blood Flow Metab.22: 1035-1041.
Bard F., Cannon C., Barbour R., Burke R.L., Games D., Grajeda H., Guido T., Hu K., Huan J., Johnson-Wood K., Khan K., Kholodenko D., Lee M., Lieberburg I., Motter R., Nguyen M., Soriano F., Vasquez N., Weiss K., Welsch B., Seubert P., Schenk D., Yednock T. (2000) Peripherally administered antibodies against amyloid beta-peptide enter the central nervous system and reduce pathology in a mouse model of Alzheimer disease. Nature Med. 6: 916-919.
Barelli H., Lebeau A., Vizzavona J., Delaere P., Chevallier N., Drouot C., Marambaud P., Ancolio K., Buxbaum J.D., Khorkova O., Heroux J., Sahasrabudhe S., Martinez J., Warter J.M., Mohr M., Checler F. (1997) Characterization of new polyclonal antibodies specific for 40 and 42 amino acid-long amyloid beta peptides: their use to examine the cell biology of presenilins and the immunohistochemistry of sporadic Alzheimer's disease and cerebral amyloid angiopathy cases. Mol. Med. 3: 695-707.
Bett A.J., Prevec L., Graham F.L. (1993) Packaging capacity and stability of human adenovirus type 5 vectors. J. Viro1.67: 5911-5921.
Braak H., Braak E. (1991) Neuropathological stageing of Alzheimer-related changes. Acta Neuropathol. 82: 239-259.
Brayden D.J., Templeton L., McClean S., Barbour R., Huang J., Nguyen M., Ahern D., Motter R., Johnson-Wood K., Vasquez N., Schenk D., Seubert P. (2001) Encapsulation in biodegradable microparticles enhances serum antibody response to parenterally-delivered β-amyloid in mice. Vaccine 19: 4185-4193.
Brion J., Passareiro J., Nunez J., Flament-Durand J. (1985) Mise en evidence immunologique de la proteine tau au niveau des lesions de degenerescence neurofibrillaire de la maladie d'Alzheimer. Arch. Biol. 95: 229-235.
Burke R.L., Goldbeck C., Ng P., Stanberry L., Ott G., Van Nest G. (1994) The influence of adjuvant on the therapeutic efficacy of a recombinant genital herpes vaccine. J. Infect. Dis. 170: 1110-1119.
Campion D., Dumanchin C., Hannequin D., Duboid B., Belliard S., Puel M., Thomas-Anterion C., Michon A., Martin C., Charbonnier F., Raux G., Camuzat A., Penet C., Mesnage V., Martinez M., Clerget-Darpoux F., Brice A., Frebourg T. (1999) Early-onset autosomal dominant Alzheimer disease: prevalence, genetic heterogeneity, and mutation spectrum. Am. J. Hum. Genet. 65: 664-670.
Cescato R., Dumermuth E., Spiess M., Paganetti P.A. (2000) Increased generation of alternatively cleaved beta-amyloid peptides in cells expressing mutants of the amyloid precursor protein defective in endocytosis. J. Neurochem. 74: 1131-1139.
Cevc G., Gebauer D., Stieber J., Schatzlein A., Blume G. (1998) Ultraflexible vesicles, Transfersomes, have an extremely low pore penetration resistance and transport therapeutic amounts of insulin across the intact mammalian skin. Biochim Biophys Acta 1368: 201-215.
Chen G.Q., Chen K.S., Knox J., Inglis J., Bernard A., Martin S.J., Justice A., McConlogue L., Games D., Freedman S.B., Morris R.G.M. (2000) A learning deficit related to age and beta-amyloid plaques in a mouse model of Alzheimer's disease. Nature 408: 975-979.
Cole et al. (1985) In: Monoclonal Antibodies and Cancer Therapy. Alan R. Liss, Inc. pp. 77-96.
Conde S. (2002) β-amyloid peptide as a target for treatment of Alzheimer's disease. Expert Opin. Ther. Patents 12: 503-512.
Cortez-Retamozo V., Lauwereys M., Hassanzadeh Gh. G., Gobert M., Conrath K., Muyldermans S., De Baetselier P., Revets H. (2002) Efficient tumor targeting by single-domain antibody fragments of camels. Int. J. Cancer 98: 456-462.
Dallas W.S., Falkow S. (1980) Amino acid sequence homology between cholera toxin and Escherichia coli heat-labile toxin. Nature 288: 499-501.
Davis H.L., Weeratna R., Waldschmidt T.J., Tygrett L., Schorr J., Krieg A.M., Weeranta R. (1998) CpG DNA is a potent enhancer of specific immunity in mice immunized with recombinant hepatitis B surface antigen. J. Immunol. 162: 3103.
Delacourte A., Défossez A. (1986) Alzheimer's disease: Tau proteins, the promoting factors of microtubule assembly, are major components of paired helical filaments. J. Neurol. Sci. 76: 173-180.
Delacourte A., Sergeant N., Champain D., Wattez A., Maurage C.-A., Lebert F., Pasquier F., David J.-P. (1999) The biochemical pathway of neurofibrillary degeneration in aging and Alzheimer's disease. Neurology, 52: 1158-1165.
Delacourte A., Sergeant N., Champain D., Wattez A., Maurage C.-A., Lebert F., Pasquier F., David J.-P. (2001) The Biochemical Spreading of Tau and Amyloid β - Precursor Protein Pathologies in Aging and Sporadic Alzheier's Disease. Brain Aging 1: 33-42.
Delacourte A., Sergeant N., Champain D., Wattez A., Maurage C.A., Lebert F., Pasquier F., David J.P. (2002) Nonoverlapping but synergetic tau and APP pathologies in sporadic Alzheimer's disease. Neurology 59: 398-407.
DeMattos R.B., Bales K.R., Cummins D.J., Dodart J.-C., Paul S.M., Holtzman D.M. (2001) Peripheral anti-Aβ antibody alters CNS and plasma Aβ clearance and decreases brain Aβ burden in a mouse model of Alzheimer's disease. Proc. Natl. Acad. Sci. U.S.A. 98: 8850-8855.
DeMattos R.B., Bales K.R., Cummins D.J., Paul S.M., Holtzman D.M. (2002) Brain to Plasma Amyloid-β Efflux: a Measure of Brain Amyloid Burden in a Mouse Model of Alzheimer's Disease. Science 295: 2264-2267.
De Strooper B., Annaert W. (2000) Proteolytic processing and cell biological functions of the amyloid precursor protein. J. Cell Sci. 113: 1857-1870.
Dodart J.-C., Bales K.R., Gannon K.S., Greene S.J., DeMattos R.B., Mathis C., DeLong C.A., Wu S., Wu X., Holtzman D.M., Paul S.M. (2002) Immunization reverses memory deficits without reducing brain Aβ burden in Alzheimer's disease model. Neuroscience 5: 452-457.
Drin G., Temsamani J. (2002) Translocation of protegrin I through phospholipid membranes: role of peptide folding. Biochim. Biophys. Acta 1559: 160-170.
Dubensky T.W. Jr, Driver D.A., Polo J.M., Belli B.A., Latham E.M., Ibanez C.E., Chada S., Brumm D., Banks T.A., Mento S.J., Jolly D.J., Chang S.M. (1996) Sindbis virus DNA-based expression vectors: utility for in vitro and in vivo gene transfer. J. Virol. 70: 508-519.
Duyckaerts C., Colle M.A., Hauw J.J. (1999) A sketch of Alzheimer's disease histopathology. In: Alzheimer's disease and related disorders. Etiology, pathogenesis and therapeutics. Iqbal K., Swaab D.F., Winlad B., Wisniewski H.M. (Eds.). John Wiley and Sons, Sussex, UK, pp. 137-152.
Esch F.S., Keim P.S., Beattie E.C., Blacher R.W., Culwell A.R., Oltersdorf T., McClure D., Ward P.J. (1990) Cleavage of amyloid beta peptide during constitutive processing of its precursor. Science 248: 1122-1124.
Felician O., Sandson T.A. (1999) The neurobiology and pharmacotherapy of Alzheimer's disease. J. Neuropsychiatry Clin. Neurosci. 11: 19-31.
Folstein M.F., Folstein S.E., McHugh P.R. (1975) Mini-Mental State. A practical method for grading the cognitive state of patients for the clinician. J. Psychiatr. Res. 12: 189-198.
Frenkel D., Katz O., Solomon B. (2000) Immunization against Alzheimer's β-amyloid plaques via EFRH phage administration. Proc. Natl. Acad. Sci. 97: 11455-11459.
Frenkel D., Kariv N., Solomon B. (2001) Generation of auto-antibodies towards Alzheimer's disease vaccination. Vaccine 19: 2615-2619.
Fukumoto H., Asami-Odaka A., Suzuki N., Iwatsubo T. (1996) Association of A beta 40-positive senile plaques with microglial cells in the brains of patients with Alzheimer's disease and in non-demented aged individuals. Neurodegeneration 5: 13-17.
Games D., Adams D., Alessandrini R., Barbour R., Berthelette P., Blackwell C., Carr T., Clemens J., Donaldson T., Gillespie F., et al. (1995) Alzheimer-type neuropathology in transgenic mice overexpressing V717F beta-amyloid precursor protein. Nature. 373: 523-527.
Ghindilis A.L., Pavlov A.R., Atanassov P.B. (eds.) (2002) Immunoassay Methods and Protocols. Humana Press, Totowa, NJ, US.
Glenn G.M., Rao M., Matyas G.R., Alving C.R. (1998) Skin immunization made possible by cholera toxin. Nature 391: 851.
Glenner G.G., Wong C.W. (1984) Alzheimer's disease: initial report of the purification and characterization of a novel cerebrovascular amyloid protein. Biochem. Biophys. Res. Commun. 120: 885-890.
Grundke-Iqbal I., Iqbal K., Tung Y., Quinlan M., Wisniewski H., Binder L. (1986) Abnormal phosphorylation of the microtubule-associated protein (tau) in Alzheimer's cytoskeletal pathology. Proc. Natl. Acad. Sci. (USA) 83: 4913-4917.
Haass C., Schlossmacher M.G., Hung A.Y., Vigo-Pelfrey C., Mellon A., Ostaszewski B.L., Lieberburg I., Koo E.H., Schenk D., Teplow D.B., et al. (1992) Amyloid beta-peptide is produced by cultured cells during normal metabolism. Nature 359: 322-325.
Haass C., Capell A., Citron M., Teplow D.B., Selkoe D.J. (1995) The vacuolar H(+)-ATPase inhibitor bafilomycin A1 differentially affects proteolytic processing of mutant and wild-type beta-amyloid precursor protein. J. Biol. Chem. 270: 6186-6192.
Harigaya Y., Saido T.C., Eckman C.B., Prada C.-M., Shoji M., Younkin S.G. (2000) Amyloid β Protein Starting Pyroglutamate at Position 3 Is a Major Component of the Amyloid Deposits in the Alzheimer's Disease Brain. Biochemical and Biophysical Research Communications 276: 422-427.
Hsiao K., Chapman P., Nilsen S., Eckman C., Harigaya Y., Younkin S., Yang F., Cole G. (1996) Correlative memory deficits, Abeta elevation, and amyloid plaques in transgenic mice. Science 274: 99-102.
Huang Q., He G., Lan Q., Li X., Qian Z. Chen J. Lu Z., Du Z. (1996) Target imaging diagnosis of human brain glioma. Clinical analysis of 40 cases. Clin. Med. J. 109: 93-96.
Hyman B.T., Trojanowski J.Q. (1997) Consensus recommendations for the postmortem diagnosis of Alzheimer disease from the National Institute on Aging and the Reagan Institute Working Group on diagnostic criteria for the neuropathological assessment of Alzheimer disease. J. Neuropathol. Exp. Neurol. 56: 1095-1097.
Jansen F.K., Blythman H.E., Carriere D., Casellas P., Gros O., Gros P., Laurent J.C., Paolucci F., Pau B., Poncelet P., Richer G., Vidal H., Voisin G.A. (1982) Immunotoxins: hybrid molecules combining high specificity and potent cytotoxicity. Immunol. Rev. 62: 185-216.
Janus C., Pearson J., McLaurin J., Mathews P.M., Jiang Y., Schmidt S.D., Chrishti M.A., Home P., Heslin D., French J., Mount H.T.J., Nixon R.A., Mercken M., Bergeron C., Fraser P.E., George-Hyslop P., Westaway D. (2000) Aβ peptide immmunization reduces behavioural impairment and plaques in a model of Alzheimer's disease. Nature 408: 979-982.
Jensen F.C., Savary J.R., Diveley J.P., Chang J.C. (1998) Adjuvant activity of incomplete Freund's adjuvant. Adv. Drug Deliv. Rev. 32: 173-186.
Kalback W., Watson M.D., Kokjohn, T.A., Kuo Y.-M., Weiss N., Luehrs D.C., Lopez J., Brune D., Sisodia S.S., Staugenbiel M., Emmerling M., Roher A.E. (2002) APP transgenic mice Tg2576 accumulate Abeta peptides that are distinct from the chemically modified and insoluble peptides deposited in Alzheimer's disease senile plaques. Biochemistry 41: 922-928.
Kang J., Lemaire H.G., Unterbeck A., Salbaum J.M., Masters C.L., Grzeschik K.H., Multhaup G., Beyreuther K., Muller-Hill B. (1987) The precursor of Alzheimer's disease amyloid A4 protein resembles a cell-surface receptor. Nature 325: 733-736.
Kawarabayashi T., Younkin L.H., Saido T.C., Shoji M., Ashe K.H., Younkin S.G. (2001) Age-Dependent Changes in Brain, CSF, and Plasma Amyloid β Protein in the Tg2576 Transgenic Mouse Model of Alzheimer's Disease. J. Neuroscience 21: 372-381.
Kensil et al. (1995) In: Vaccine Design: The Subunit and Adjuvant Approach. Powell and Newman (eds.), Plenum Press, NY.
Kitaguchi N., Takahashi Y., Tokushima Y., Shiojiri S., Ito H. (1988) Novel precursor of Alzheimer's disease amyloid protein shows protease inhibitory activity. Nature 331: 530-532.
Klein W.L., Krafft G.A., Finch C.E. (2001) Targeting small Aβ oligomers: the solution to an Alzheimer's disease conundrum? Trends Neurosci. 24: 219-224.
Kohler G., Milstein C. (1975) Continuous cultures of fused cells secreting antibody of predefined specificity. Nature 256: 495-497.
Kondo J., Honda T., Mori H., Hamada Y., Miura R., Ogawara M., Ihara Y. (1988) The carboxyl third of tau is tightly bound to paired helical filaments. Neuron 1: 827-834.
Kosik K.S., Joachim C.L., Selkoe D.J. (1986) Microtubule-associated protein tau is a major antigenic component of paired helical filaments in Alzheimer's disease. Proc. Natl. Acad. Sci. (USA) 83: 4044-4048.
Kozbor D., Dexter D., Roder J.C. (1983) A comparative analysis of the phenotypic characteristics of available fusion partners for the construction of human hybridomas. Hybridoma 2:7-16.
Langer R. (1990) New methods of drug delivery. 249: 1527-1533.
Langer R., Cleland J.L., Hanes J. (1997) New advances in microsphere-based single-dose vaccines. Adv. Drug Deliv. Rev. 28: 97-119.
Langston J.W., Widner H., Goetz C.G., Brooks D., Fahn S., Freeman T., Watts R. (1992) Core assessment program for intracerebral transplantations (CAPIT). Mov. Disord .7: 2-13.
Lawrie and Tumin (1993) Cur. Opin. Genet. Develop. 3: 102-109.
Lemere C.A., Maron R., Spooner E.T., Grenfell T.J., Mori C., Desai R., Hancock W.W., Weiner H.L., Selkoe D.J. (2000a) Nasal Aβ treatment induces anti-Aβ antibody production and decreases cerebral amyloid burden in PD-APP mice. Ann. N.Y. Acad. Sci. 920: 328-331.
Lemere C.A., Maron R., Spooner E.T., Grenfell T.J., Mori C., Desai R., Weiner H.L., Selkoe D.J. (2000b) Nasal Aβ immunization reduces amyloid-β protein burden in PDAPP mice [abstract 567] Neurobiol. Aging 21: S126.
Lemere C.A., Spooner E.T., Desai R., Grenfell T.J., Mori C., Vekrellis K., Selkoe D.J. (2000c) Effects of Aβ immunization in mice and of the priming of microglia with Aβ antibodies [abstract 397.3] Soc. Neurosci. Abstr. 26: 1060.
Lemere C.A., Maron R., Selkoe D.J., Weiner H.L. (2001) Nasal Vaccination with β-Amyloid Peptide for the Treatment of Alzheimer's Disease. DNA and Cell Biology 20: 705-711.
Lewczuk P., Esselmann H., Meyer M., Wollscheid V., Neumann M., Otto M., Maler J.M., Ruther E., Kornhuber J., Wiltfang J. (2003) The amyloid-beta (Abeta) peptide pattern in cerebrospinal fluid in Alzheimer's disease: evidence of a novel carboxyterminally elongated Abeta peptide. Rapid. Commun. Mass. Spectrom.17: 1291-1296.
Livingston B.D., Crimi C., Grey H., Ishioka G., Chisari F.V., Fikes J., Grey H., Chesnut R.W., Sette A. (1997) The hepatitis B virus-specific CTL responses induced in humans by lipopeptide vaccination are comparable to those elicited by acute viral infection. J. Immunol. 159: 1383-1392.
Mariani G., Lasku A., Pau A., Villa G., Motta C., Calcagno G., Taddei G.Z., Castellani P., Syrigos K., Dorcaratto A., Epenetos A.A., Zardi L., Viale G.A. (1997) A pilot pharmacokinetic and immunoscintigraphic study with the technetium-99m labeled monoclonal antibody BC-1 directed against oncofetal fibronectin in patients with brain tumours. Cancer 15: 2484-2489.
Masters C.L., Simms G., Weinman N.A., Multhaup G., McDonald B.L., Beyreuther K. (1985) Amyloid plaque core protein in Alzheimer disease and Down syndrome. Proc. Natl. Acad. Sci. U S A 82: 4245-4249.
McGee J.P., Singh M., Li X.M., Qiu H., O'Hagan D.T. (1997) The encapsulation of a model protein in poly (D, L lactide-co-glycolide) microparticles of various sizes: an evaluation of process reproducibility. J. Microencapsul. 14: 197-210.
McKeith I.G., Galasko D., Kosaka K., Perry E.K., Dickson D.W., Hansen L.A., Salmon D.P., Lowe J., Mirra S.S., Byrne E.J., Lennox G., Quinn N.P., Edwardson J.A., Ince P.G., Bergeron C., Bums A., Miller B.L., Loverstone S., Collerton D., Jansen E.N.H., Ballard C., d Vos R.A.I., Wilcock G.K., Jellinger K.A., Perry R.H. (1996) Consensus guidelines for the clinical and pathologic diagnosis of dementia with Lewy bodies (DLB): Report of the consortium on DLB international workshop. Neurology 47: 1113-1124.
McKhann G., Drachman D.A., Folstein M.F., Katzman R., Price D.L., Stadlan E. (1984) Clinical diagnosis of Alzheimer's disease: report of the NINCDS-ADRDA Work Group under the auspices of the Department of Health and Human Services Task Force on Alzheimer's disease. Neurology 34: 939-944.
Moechars D., Dewachter I., Lorent K., Reverse D., Baekelandt V., Naidu A., Tesseur I., Spittaels K., Haute C.V., Checler F., Godaux E., Cordell B., Van Leuven F. (1999) Early phenotypic changes in transgenic mice that overexpress different mutants of amyloid precursor protein in brain. J. Biol. Chem. 274: 6483-6492.
Monroe et al. (1986) Amer. Clin. Prod. Rev. 5: 34-41.
Morgan D., Diamond D.M., Gottschall P.E., Ugen K.E., Dickey C., Hardy J., Duff K., Jantzen P., DiCarlo G., Wilcock D., Connor K., Hatcher J., Hope C., Gordon M., Arendash G.W. (2000) Aβ peptide vaccination prevents memory loss in an animal mo Muyldermans S. (2001) Single domain camel antibodies: current status. J. Biotechnol. 74: 277-302.
Ohe Y., Zhao D., Saijo N., Podack E.R. (1995) Construction of a novel bovine papillomavirus vector without detectable transforming activity suitable for gene transfer. Hum. Gene Ther.6: 325-333.
Paul A., Cevc G., Bachhawat B.K. (1995) Transdermal immunization with large proteins by means of ultradeformable drug carriers. Eur. J. Immunol. 25: 3521-3524.
Petersen R.C., Stevens J.C., Ganguli M., Tangalos E.G., Cummings J.L., DeKosky S.T. (2001) Practice parameter: Early detection of dementia: Mild cognitive impairment (an evidence-based review). Report of the Quality Standards Subcommittee of the American Academy of Neurology. Neurology 56: 1133-1142.
Ponte P., Gonzalez-DeWhitt P., Schilling J., Miller J., Hsu D., Greenberg B., Davis K., Wallace W., Lieberburg I., Fuller F. (1988) A new A4 amyloid mRNA contains a domain homologous to serine proteinase inhibitors. Nature 331: 525-527.
Queen C., Schneider W.P., Selick H.E., Payne P.W., Landolfi N.F., Duncan J.F., Avdalovic N.M., Levitt M., Junghans R.P., Waldmann T.A. (1989) A humanized antibody that binds to the interleukin 2 receptor. Proc. Natl. Acad. Sci. U S A 86: 10029-10033.
Remington's Pharmaceutical Sciences. (1995) Mack Publishing Co., Easton, PA, US.
Saido T.C., Iwatsubo T., Mann DM.A., Shimada H., Ihara Y., Kawashima S. (1995) Dominant and differential deposition of distinct beta-amyloid peptide species, A beta N3(pE), in senile plaques. Neuron 14: 457-466.
Saido T.C., Yamao-Harigaya W., Iwatsubo T., Kawashima S. (1996) Amino- and carboxy-terminal heterogeneity of β-amyloid peptides deposited in human brain. Neuroscience Letters 215: 173-176.
Saido T.C. (2000) Degradation of amyloid-β peptide: a key to Alzheimer pathogenesis, prevention and therapy. Neurosci. News 3: 52-62.
Sambrook J., Fritsch E., Maniatis T. (1989) Molecular Cloning: A Laboratory Manual, 2nd Edition, Cold Spring Harbour Laboratory Press, Cold Spring Harbor, NY, USA.
Sandrock D., Verheggen R., Helwig A.T., Munz D.L., Markakis E., Emrich D. (1996) Immunoscintigraphy for the detection of brain abscesses. Nucl. Med. Commun. 17: 311-316.
Schenk D.B., Barbour R., Dunn W., Gordon G., Grajeda H., Guido T., Hu K., Huan J., Johnson-Wood K., Khan K., Kholodenko D., Lee M., Liao Z., Lieberburg I., Motter R., Mutter L., Soriano F., Shopp G., Vasquez N., Vandevert C., Walker S., Wogulis M., Yednock T., Games D., Seubert P. (1999) Immunization with amyloid-β attenuates Alzheimer-disease-like pathology in the PDAPP mouse. Nature 400: 173-177.
Schenk D.B., Seubert P., Lieberburg I., Wallace J. (2000) Peptide immunization. A possible new treatment for Alzheimer's disease. Arch. Neurol. 57: 934-936.
Schenk D., Seubert P., Ciccarelli R.B. (2001) Immunotherapy with beta-amyloid for Alzheimer's disease: a new frontier. DNA Cell Biol. 20: 679-681.
Schrader-Fischer G., Paganetti P.A. (1996) Effect of alkalizing agents on the processing of the beta-amyloid precursor protein. Brain Res.716: 91-100.
Seo J., Kim S., Kim H., Park C.H., Jeong S., Lee J., Choi S.H., Chang K., Rah J., Koo J., Kim E., Suh Y. (2001) Effects of nicotine on APP secretion and Abeta- or CT(105)-induced toxicity. Biol. Psychiatry 49: 240-247.
Sergeant N., David J.P., Champain D., Ghestem A., Wattez A., Delacourte A. (2002) Progressive decrease of amyloid precursor protein carboxy terminal fragments (APP-CTFs), associated with tau pathology stages, in Alzheimer's disease. J. Neurochem. 81: 663-672.
Sergeant N., Bombois S., Ghestem A., Drobecq H., Kostanjevecki V., Missiaen C., Wattez A., David J.P., Vanmechelen E., Sergheraert C., Delacourte A. (2003) Truncated beta-amyloid peptide species in pre-clinical Alzheimer's disease as new targets for the vaccination approach. J. Neurochem. 85: 1581-1591.
Seubert P., Vigo-Pelfrey C., Esch F., Lee M., Dovey H., Davis D., Sinha S., Schlossmacher M., Whaley J., Swindlehurst C., et al. (1992) Isolation and quantification of soluble Alzheimer's beta-peptide from biological fluids. Nature 359: 325-327.
Sigurdsson E.M., Scholtzova H., Mehta P.D., Frangione B., Wisniewski T. (2001) Immunization with a non-toxic/non-fibrillar amyloid-β homologous peptide reduces Alzheimer's disease-associated pathology in transgenic mice. Am. J. Pathol. 159: 439-447.
Sisodia S.S. (1992) Beta-amyloid precursor protein cleavage by a membrane-bound protease. Proc. Natl. Acad. Sci. USA 89: 6075-6079.
Spinelli S., Frenken L.G., Hermans P., Verrips T., Brown K., Tegoni M., Cambillau C. (2000) Camelid heavy-chain variable domains provide efficient combining sites to haptens. Biochemistry.39: 1217-1222.
Sramek J.J., Cutler N.R. (2000) Ongoing trials in Alzheimer's disease. Expert Opin. Investig. Drugs 9: 899-915.
Stoute J.A., Slaoui M., Heppner D.G., Momin P., Kester K.E., Desmons P., Wellde B.T., Garcon N., Krzych U., Marchand M. (1997) A preliminary evaluation of a recombinant circumsporozoite protein vaccine against Plasmodium falciparum malaria. RTS,S Malaria Vaccine Evaluation Group. N. Engl. J. Med. 336: 86-91.
Sturchler-Pierrat C., Abramowski D., Duke M., Wiederhold K.H., Mistl C., Rothacher S., Ledermann B., Burki K., Frey P., Paganetti P.A., Waridel C., Calhoun M.E., Jucker M., Probst A., Staufenbiel M., Sommer B. (1997) Two amyloid precursor protein transgenic mouse models with Alzheimer disease-like pathology. Proc. Natl. Acad. Sci. USA 94: 13287-13292.
Takeuchi A., Irizarry M.C., Duff K., Saido T.C., Hsiao Ashe K., Hasegawa M., Mann D.M., Hyman B.T., Iwatsubo T. (2000) Age-related amyloid beta deposition in transgenic mice overexpressing both Alzheimer mutant presenilin 1 and amyloid beta precursor protein Swedish mutant is not associated with global neuronal loss. Am. J. Pathol. 157: 331-339.
Tamada K., Fujinaga S., Watanabe R., Yamashita R., Takeuchi Y., Osano M. (1995) Specific deposition of passively transferred monoclonal antibodies against herpes simplex virus type 1 in rat brain infected with the virus. Microbiol-Immunol. 39: 861-871.
Temsamani J., Rousselle C., Rees A.R., Scherrmann J.M. (2001) Vector-mediated drug delivery to the brain. Expert Opin. Biol. Ther.1: 773-782.
Thal L.J. (2000) Trials to slow progression and prevent disease onset. J. Neural. Transm. Suppl. 59: 243-249.
Tigges M.A., Leng S., Johnson D.C., Burke R.L. (1996) Human herpes simplex virus (HSV)-specific CD8+ CTL clones recognize HSV-2-infected fibroblasts after treatment with IFN-γ or when virion host shutoff functions are disabled. J. Immunol. 156: 3901-3910.
Vogel F.R., Powell M.F., Alving C.R. (2003) A Compendium of Vaccine Adjuvants and Excipients (2nd Edition) http://vrc.nih.gov/daids/vaccine/pdf/compendium.pdf.
Wahlund L.-O., Pihlstrand E., Eriksdotter Jönhagen M. (2003) Mild cognitive impairment: experience from a memory clinic. Acta Neurol. Scand. 107 (Suppl. 179): 21-24.
Wakabayashi T., Yoshida J., Okada H., Sugita K., Itoh K., Tadokoro M., Ohshima M. (1995) Radioimaging of human glioma by indium-11 labelled G-22 anti-glioma monoclonal antibody. Noshuyo-Byori 12: 105-110.
Weiner H.L., Lemere C.A., Maron R., Spooner E.T., Grenfell T.J., Mori C., Issazadeh S., Hancock W.W., Selkoe D.J. (2000) Nasal administration of amyloid-beta peptide decreases cerebral amyloid burden in a mouse model of Alzheimer's disease. Ann. Neurol. 48: 567-579.
Wild D. (ed.) (2001) The Immunoassay Handbook 2nd edition. Nature Pr., London, UK.
Wisnieuwski H.M., Vorbrodt A.W., Wegiel J. (1997) Amyloid angiopathy and blood-brain barrier changes in Alzheimer's disease. Ann. N.Y. Acad. Sci. 826: 161-172.
Wood J., Mirra S., Pollock N., Binder L. (1986) Neurofibrillary tangles of Alzheimer's disease share antigenic determinants with the axonal mirotubule-associated protein tau. Proc. Natl. Acad. Sci. (USA) 83: 4040-4043.
Xiao W., Brandsma J.L. (1996) High efficiency, long-term clinical expression of cottontail rabbit papillomavirus (CRPV) DNA in rabbit skin following particle-mediated DNA transfer. Nucleic Acids Res. 24: 2620-2622.
Zhou S.Z., Cooper S., Kang L.Y., Ruggieri L., Heimfeld S., Srivastava A., Broxmeyer H.E. (1994) Adeno-associated virus 2-mediated high efficiency gene transfer into immature and mature subsets of hematopoietic progenitor cells in human umbilical cord blood. J. Exp. Med. 179: 1867-1875.

## Claims

1. A method for determining, in a mammal, the susceptibility to a disease associated with β-amyloid formation and/or aggregation such as Alzheimer's disease, or for determining, in a mammal, the risk of developing a disease associated with β-amyloid formation and/or aggregation such as Alzheimer's disease, or for screening of the clearance of β-amyloid deposition in a mammal, or for predicting the level of β-amyloid burden in a mammal, said method comprising the following steps:
(a) determining, in said mammal, the amount of a N-terminal truncated and/or post-translational modified β-amyloid 42 variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or the amount of antibody specific for said β-amyloid variant;
(b) comparing the amount determined in step (a) with the amount of said β-amyloid variant, or said antibody in a control mammal;
(c) concluding, from the comparison in step (b), whether the mammal is susceptible to a disease associated with β-amyloid formation and/or aggregation such as Alzheimer's disease, whether the mammal is at risk of developing a disease associated with β-amyloid formation and/or aggregation such as Alzheimer's disease, whether the β-amyloid deposition in the mammal is cleared, or what the level of β-amyloid burden is in said mammal.

2. The method according to claim 1, for determining, in a mammal, the susceptibility to a disease associated with β-amyloid formation and/or aggregation such as Alzheimer's disease, or for determining, in a mammal, the risk of developing a disease associated with β-amyloid formation and/or aggregation such as Alzheimer's disease, or for screening of the clearance of β-amyloid deposition in a mammal, or for predicting the level of β-amyloid burden in a mammal, said method comprising the following steps:
(a) determining, in said mammal, the amount of a N-terminal fragment of said β-amyloid variant, or the amount of antibody specific for said N-terminal fragment of a β-amyloid variant;
(b) comparing the amount determined in step (a) with the amount of said N-terminal fragment, or said antibody specific for said N-terminal fragment in a control mammal;
(c) concluding, from the comparison in step (b), whether the mammal is susceptible to a disease associated with β-amyloid formation and/or aggregation such as Alzheimer's disease, whether the mammal is at risk of developing a disease associated with β-amyloid formation and/or aggregation such as Alzheimer's disease, whether the β-amyloid deposition in the mammal is cleared, or what the level of β-amyloid burden is in said mammal.

3. The method according to claim 1, for predicting the level of β-amyloid burden in a mammal, said method comprising the following steps:
(a) administering to said mammal of a vaccine composition or a therapeutic composition comprising a N-terminal truncated and/or post-translational modified β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or comprising an antibody that specifically recognizes a N-terminal truncated and/or post-translational modified Aβ variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or comprising a nucleic acid preparation encoding an N-terminal truncated and/or post-translational modified Aβ variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42);
(b) determining, in a biological fluid sample obtained from said mammal, the amount of N-terminal truncated and/or post-translational modified β-amyloid variant;
(c) comparing the amount determined in step (b) with the amount of N-terminal truncated and/or post-translational modified β-amyloid variant in a biological fluid sample obtained from a control mammal;
(d) concluding, from the comparison in step (c) what the level of β-amyloid burden is in said mammal.

4. The method according to claim 3, for predicting the level of β-amyloid burden in a mammal, said method comprising the following steps:
(a) administering to said mammal of a vaccine composition or a therapeutic composition comprising a N-terminal fragment of said β-amyloid variant, or comprising an antibody that specifically recognizes a N-terminal fragment of said β-amyloid variant, or comprising a nucleic acid preparation encoding a N-terminal fragment of said β-amyloid variant;
(b) determining, in a biological fluid sample obtained from said mammal, the amount of N-terminal fragment of said β-amyloid variant;
(c) comparing the amount determined in step (b) with the amount of said N-terminal fragment in a biological fluid sample obtained from a control mammal;
(d) concluding, from the comparison in step (c) what the level of β-amyloid burden is in said mammal.

5. The method according to any of claims 1 to 4, further **characterized in that** the post-translational modification is methylation or pyroglutamylation.

6. A method according to claim 1, for determining, in a mammal, the susceptibility to a disease associated with β-amyloid formation and/or aggregation or for determining, in a mammal, the risk of developing a disease associated with β-amyloid formation and/or aggregation comprising the following steps:
(a) determining, in a sample obtained from said mammal, the amount of antibody or reactive T-cells specific for an N-terminal truncated and/or post-translationally modified β-amyloid variant;
(b) comparing the amount determined in step (a) with the amount of said antibody or reactive T-cells in a control mammal;
(c) concluding, from the comparison in step (b), whether the mammal is susceptible to a disease associated with β-amyloid formation and/or aggregation or whether the mammal is at risk of developing a disease associated with β-amyloid formation and/or aggregation, an increased amount of antibody or reactivated T-cells specific for said N-terminal truncated and/or post-translationally modified β-amyloid variant, being an indication that the mammal is susceptible to or at risk of developing a disease associated with Aβ formation and/or aggregation.

7. The method according to claim 6, for determining, in a mammal, the susceptibility to a disease associated with β-amyloid formation and/or aggregation or for determining, in a mammal, the risk of developing a disease associated with β-amyloid formation and/or aggregation comprising the following steps:
(d) determining, in a sample obtained from said mammal, the amount of antibody or reactive T-cells specific for an N-terminal fragment of β-amyloid variant;
(e) comparing the amount determined in step (a) with the amount of said antibody or reactive T-cells in a control mammal;
(f) concluding, from the comparison in step (b), whether the mammal is susceptible to a disease associated with β-amyloid formation and/or aggregation or whether the mammal is at risk of developing a disease associated with β-amyloid formation and/or aggregation, an increased amount of antibody or reactivated T-cells specific for said N-terminal fragment, being an indication that the mammal is susceptible to or at risk of developing a disease associated with Aβ formation and/or aggregation.

8. The method according to any of claims 1 to 7 further **characterized in that** the sample is a brain extract sample or a body fluid sample.

9. The method according to claim 8, further **characterized in that** the body fluid sample is a blood sample or a CSF sample.

10. The method according to any of claims 1 to 9, further **characterized in that** the disease associated with β-amyloid formation and/or aggregation is AD.

11. A diagnostic or theranostic kit comprising a preparation of a N-terminal truncated and/or post-translationally modified β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or comprising an antibody specifically recognizing a N-terminal truncated and/or post-translationally modified β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42).

12. The diagnostic or theranostic kit according to claim 11, comprising a preparation of a N-terminal fragment of said β-amyloid variant, or comprising an antibody specifically recognizing a N-terminal fragment of said β-amyloid variant.

13. The kit according to claim 11, further **characterized in that** it comprises:
- an antibody (primary antibody) which forms an immunological complex with the N-terminal truncated and/or post-translationally modified β-amyloid variant to be detected;
- an antibody (secondary antibody) which specifically recognizes the N-terminal truncated and/or post-translationally modified β-amyloid variant to be detected;
- a marker either for specific tagging or coupling with said secondary antibody;
- appropriate buffer solutions for carrying out the immunological reaction between the primary antibody and the N-terminal truncated and/or post-translationally modified β-amyloid variant, between the secondary antibody and the primary antibody-N-terminal truncated and/or post-translationally modified β-amyloid variant;
- possibly, for standardization purposes, a purified N-terminal truncated and/or post-translationally modified β-amyloid variant.

14. The kit according to claim 12, further **characterized in that** it comprises:
- an antibody (primary antibody) which forms an immunological complex with the N-terminal fragment of said β-amyloid variant to be detected;
- an antibody (secondary antibody) which specifically recognizes the N-terminal fragment of said β-amyloid variant to be detected;
- a marker either for specific tagging or coupling with said secondary antibody;
- appropriate buffer solutions for carrying out the immunological reaction between the primary antibody and the N-terminal fragment of said β-amyloid variant, between the secondary antibody and the primary antibody-N-terminal fragment complex and/or between the bound secondary antibody and the marker;
- possibly, for standardization purposes, a purified N-terminal fragment of said β-amyloid variant.

15. A method for the preparation of an antibody that specifically recognizes an N-terminal truncated and/or post-translationally modified β-amyloid variant, comprising the following steps:
(a) immunizing an animal with a N-terminal β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or with a nucleic acid preparation comprising a nucleic acid sequence capable of encoding the β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42);
(b) obtaining the antibodies generated by the immunization in step (a);
(c) screening the antibodies obtained in step (b) for their specific recognition of N-terminal truncated and/or post-translationally modified β-amyloid variants.

16. The method according to claim 15, for the preparation of an antibody that specifically recognizes a N-terminal fragment of said β-amyloid variant, comprising the following steps:
(a) immunizing an animal with said N-terminal fragment or with a nucleic acid comprising a nucleic acid sequence capable of encoding said N-terminal fragment of said β-amyloid variant;
(b) obtaining the antibodies generated by the immunization in step (a);
(c) screening the antibodies obtained in step (b) for their specific recognition of N-terminal fragment of said β-amyloid variant.

17. An antibody obtainable by the method according to claim 15 or 16.

18. Use of a N-terminal β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or an antibody that specifically recognizes an N-terminal truncated and/or post-translationally modified β-amyloid variant, or a nucleic acid preparation comprising a nucleic acid sequence capable of encoding the β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), for the manufacture of a prophylactic vaccine for the prevention of a disease associated with β-amyloid formation and/or aggregation such as Alzheimer's disease.

19. Use according to claim 18, of a N-terminal fragment of said β-amyloid variant, or an antibody that specifically recognizes a N-terminal fragment of said β-amyloid variant, or a nucleic acid preparation comprising a nucleic acid sequence capable of encoding said β-amyloid variant fragment, for the manufacture of a prophylactic vaccine for the prevention of a disease associated with β-amyloid formation and/or aggregation such as Alzheimer's disease.

20. Use of a N-terminal β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or an antibody that specifically recognizes an N-terminal truncated and/or post-translationally modified β-amyloid variant, or a nucleic acid preparation comprising a nucleic acid sequence capable of encoding the β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), for the manufacture of a therapeutic composition for the treatment of a disease associated with β-amyloid formation and/or aggregation such as Alzheimer's disease.

21. Use according to claim 20, of a N-terminal fragment of said β-amyloid variant, or an antibody that specifically recognizes a N-terminal fragment of said β-amyloid variant, or a nucleic acid preparation comprising a nucleic acid sequence capable of encoding said N-terminal fragment of said β-amyloid variant, for the manufacture of a therapeutic composition for the treatment of a disease associated with β-amyloid formation and/or aggregation such as Alzheimer's disease.

22. A method for the measurement, in a mammal, of the immune response induced by vaccination or therapeutic application with a N-terminal β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or an antibody that specifically recognizes an N-terminal truncated and/or post-translationally modified β-amyloid variant, or a nucleic acid preparation comprising a nucleic acid sequence capable of encoding the β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), said method comprising the following steps:
(a) determining, in a sample obtained from said mammal, the amount of antibody specific for a N-terminal β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42),
(b) comparing the amount determined in step (a) with the amount of antibody specific for said β-amyloid variant present in the mammal before vaccination or therapeutic application with the N-terminal β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42), or an antibody that specifically recognizes an N-terminal truncated and/or post-translationally modified β-amyloid variant, or a nucleic acid preparation comprising a nucleic acid sequence capable of encoding the β-amyloid variant selected from the group consisting of Aβ (2-42), Aβ (3-42), Aβ (4-42), Aβ (5-42), Aβ (6-42), Aβ (7-42), Aβ (8-42), Aβ (9-42), Aβ (10-42);
(c) concluding, from the comparison in step (b), whether the mammal is responding to the vaccination or therapy, an increased amount of antibody specific for said β-amyloid variant being an indication that the mammal is responding to the vaccination or therapy.

23. The method according claim 22, for the measurement, in a mammal, of the immune response induced by vaccination or therapeutic application with a N-terminal fragment of said β-amyloid variant, or an antibody according to claim 18, or nucleic acid preparation according to claim 16, said method comprising the following steps:
(a) determining, in a sample obtained from said mammal, the amount of antibody specific for a N-terminal fragment of said β-amyloid variant,
(b) comparing the amount determined in step (a) with the amount of antibody specific for said N-terminal fragment of said β-amyloid variant present in the mammal before vaccination or therapeutic application with the N-terminal fragment of said β-amyloid variant, or an antibody that specifically recognizes an N-terminal fragment of said β-amyloid variant, or a nucleic acid preparation comprising a nucleic acid sequence capable of encoding the N-terminal fragment of said β-amyloid variant;
(c) concluding, from the comparison in step (b), whether the mammal is responding to the vaccination or therapy, an increased amount of antibody specific for said N-terminal fragment of said β-amyloid variant being an indication that the mammal is responding to the vaccination or therapy.


SEQUENCE LISTING

<110>  Innogenetics N.V.

<120>  Prevention, treatment and diagnosis of diseases associated with beta-
amyloid formation and/or aggregation

<130>  EP 140

<140>  02447147.6
<141>  2002-07-24

<160>  261

<170>  PatentIn version 3.1

<210>  1
<211>  4
<212>  PRT
<213>  homo sapiens

<400>  1

Asp Ala Glu Phe
1


<210>  2
<211>  5
<212>  PRT
<213>  homo sapiens

<400>  2

Asp Ala Glu Phe Arg
1               5


<210>  3
<211>  6
<212>  PRT
<213>  homo sapiens

<400>  3

Asp Ala Glu Phe Arg His
1               5


<210>  4
<211>  7
<212>  PRT
<213>  homo sapiens

<400>  4

Asp Ala Glu Phe Arg His Asp
1               5


<210>  5

<211>  8
<212>  PRT
<213>  homo sapiens

<400>  5

Asp Ala Glu Phe Arg His Asp Ser
1               5


<210>  6
<211>  9
<212>  PRT
<213>  homo sapiens

<400>  6

Asp Ala Glu Phe Arg His Asp Ser Gly
1               5


<210>  7
<211>  10
<212>  PRT
<213>  homo sapiens

<400>  7

Asp Ala Glu Phe Arg His Asp Ser Gly Tyr
1               5                   10


<210>  8
<211>  11
<212>  PRT
<213>  homo sapiens

<400>  8

Asp Ala Glu Phe Arg His Asp Ser Gly Tyr Glu
1               5                   10


<210>  9
<211>  12
<212>  PRT
<213>  homo sapiens

<400>  9

Asp Ala Glu Phe Arg His Asp Ser Gly Tyr Glu Val
1               5                   10


<210>  10
<211>  13
<212>  PRT
<213>  homo sapiens

<400>  10

Asp Ala Glu Phe Arg His Asp Ser Gly Tyr Glu Val His
1               5                   10

<210>  11
<211>  14
<212>  PRT
<213>  homo sapiens

<400>  11

Asp Ala Glu Phe Arg His Asp Ser Gly Tyr Glu Val His His
1               5                   10

<210>  12
<211>  15
<212>  PRT
<213>  homo sapiens

<400>  12

Asp Ala Glu Phe Arg His Asp Ser Gly Tyr Glu Val His His Gln
1               5                   10                  15

<210>  13
<211>  16
<212>  PRT
<213>  homo sapiens

<400>  13

Asp Ala Glu Phe Arg His Asp Ser Gly Tyr Glu Val His His Gln Lys
1               5                   10                  15

<210>  14
<211>  4
<212>  PRT
<213>  homo sapiens

<400>  14

Ala Glu Phe Arg
1

<210>  15
<211>  5
<212>  PRT
<213>  homo sapiens

<400>  15

Ala Glu Phe Arg His
1               5

<210>  16
<211>  6

<212>  PRT
<213>  homo sapiens

<400>  16

Ala Glu Phe Arg His Asp
1               5


<210>  17
<211>  7
<212>  PRT
<213>  homo sapiens

<400>  17

Ala Glu Phe Arg His Asp Ser
1               5


<210>  18
<211>  8
<212>  PRT
<213>  homo sapiens

<400>  18

Ala Glu Phe Arg His Asp Ser Gly
1               5


<210>  19
<211>  9
<212>  PRT
<213>  homo sapiens

<400>  19

Ala Glu Phe Arg His Asp Ser Gly Tyr
1               5


<210>  20
<211>  10
<212>  PRT
<213>  homo sapiens

<400>  20

Ala Glu Phe Arg His Asp Ser Gly Tyr Glu
1               5                   10


<210>  21
<211>  11
<212>  PRT
<213>  homo sapiens

<400>  21

Ala Glu Phe Arg His Asp Ser Gly Tyr Glu Val

1               5                   10


<210>  22
<211>  12
<212>  PRT
<213>  homo sapiens

<400>  22

Ala Glu Phe Arg His Asp Ser Gly Tyr Glu Val His
1               5                   10


<210>  23
<211>  13
<212>  PRT
<213>  homo sapiens

<400>  23

Ala Glu Phe Arg His Asp Ser Gly Tyr Glu Val His His
1               5                   10


<210>  24
<211>  14
<212>  PRT
<213>  homo sapiens

<400>  24

Ala Glu Phe Arg His Asp Ser Gly Tyr Glu Val His His Gln
1               5                   10


<210>  25
<211>  15
<212>  PRT
<213>  homo sapiens

<400>  25

Ala Glu Phe Arg His Asp Ser Gly Tyr Glu Val His His Gln Lys
1               5                   10                  15


<210>  26
<211>  16
<212>  PRT
<213>  homo sapiens

<400>  26

Ala Glu Phe Arg His Asp Ser Gly Tyr Glu Val His His Gln Lys Leu
1               5                   10                  15


<210>  27
<211>  4
<212>  PRT

<213>  homo sapiens

<400>  27

Glu Phe Arg His
1


<210>  28
<211>  5
<212>  PRT
<213>  homo sapiens

<400>  28

Glu Phe Arg His Asp
1               5


<210>  29
<211>  6
<212>  PRT
<213>  homo sapiens

<400>  29

Glu Phe Arg His Asp Ser
1               5


<210>  30
<211>  7
<212>  PRT
<213>  homo sapiens

<400>  30

Glu Phe Arg His Asp Ser Gly
1               5


<210>  31
<211>  8
<212>  PRT
<213>  homo sapiens

<400>  31

Glu Phe Arg His Asp Ser Gly Tyr
1               5


<210>  32
<211>  9
<212>  PRT
<213>  homo sapiens

<400>  32

Glu Phe Arg His Asp Ser Gly Tyr Glu
1               5

<210>  33
<211>  10
<212>  PRT
<213>  homo sapiens

<400>  33

Glu Phe Arg His Asp Ser Gly Tyr Glu Val
1               5                   10


<210>  34
<211>  11
<212>  PRT
<213>  homo sapiens

<400>  34

Glu Phe Arg His Asp Ser Gly Tyr Glu Val His
1               5                   10


<210>  35
<211>  12
<212>  PRT
<213>  homo sapiens

<400>  35

Glu Phe Arg His Asp Ser Gly Tyr Glu Val His His
1               5                   10


<210>  36
<211>  13
<212>  PRT
<213>  homo sapiens

<400>  36

Glu Phe Arg His Asp Ser Gly Tyr Glu Val His His Gln
1               5                   10


<210>  37
<211>  14
<212>  PRT
<213>  homo sapiens

<400>  37

Glu Phe Arg His Asp Ser Gly Tyr Glu Val His His Gln Lys
1               5                   10


<210>  38
<211>  15
<212>  PRT
<213>  homo sapiens

<400>  38

Glu Phe Arg His Asp Ser Gly Tyr Glu Val His His Gln Lys Leu
1               5                   10                  15


<210>  39
<211>  16
<212>  PRT
<213>  homo sapiens

<400>  39

Glu Phe Arg His Asp Ser Gly Tyr Glu Val His His Gln Lys Leu Val
1               5                   10                  15


<210>  40
<211>  4
<212>  PRT
<213>  homo sapiens

<220>
<221>  MISC_FEATURE
<222>  (1)..(1)
<223>  x represents PyrE


<400>  40

Xaa Phe Arg His
1


<210>  41
<211>  6
<212>  PRT
<213>  homo sapiens

<220>
<221>  MISC_FEATURE
<222>  (1)..(1)
<223>  x represents PyrE


<400>  41

Xaa Glu Phe Arg His Asp
1               5


<210>  42
<211>  7
<212>  PRT
<213>  homo sapiens

<220>
<221>  MISC_FEATURE
<222>  (1)..(1)
<223>  x represents PyrE

<400>  42

Xaa Glu Phe Arg His Asp Ser
1               5


<210>  43
<211>  8
<212>  PRT
<213>  homo sapiens

<220>
<221>  MISC_FEATURE
<222>  (1)..(1)
<223>  x represents PyrE


<400>  43

Xaa Glu Phe Arg His Asp Ser Gly
1               5


<210>  44
<211>  9
<212>  PRT
<213>  homo sapiens

<220>
<221>  MISC_FEATURE
<222>  (1)..(1)
<223>  x represents PyrE


<400>  44

Xaa Glu Phe Arg His Asp Ser Gly Tyr
1               5


<210>  45
<211>  10
<212>  PRT
<213>  homo sapiens

<220>
<221>  MISC_FEATURE
<222>  (1)..(1)
<223>  x represents PyrE


<400>  45

Xaa Glu Phe Arg His Asp Ser Gly Tyr Glu
1               5                   10


<210>  46
<211>  11

<212>  PRT
<213>  homo sapiens

<220>
<221>  MISC_FEATURE
<222>  (1)..(1)
<223>  x represents PyrE


<400>  46

Xaa Glu Phe Arg His Asp Ser Gly Tyr Glu Val
1               5                   10


<210>  47
<211>  12
<212>  PRT
<213>  homo sapiens

<220>
<221>  MISC_FEATURE
<222>  (1)..(1)
<223>  x represents PyrE


<400>  47

Xaa Glu Phe Arg His Asp Ser Gly Tyr Glu Val His
1               5                   10


<210>  48
<211>  13
<212>  PRT
<213>  homo sapiens

<220>
<221>  MISC_FEATURE
<222>  (1)..(1)
<223>  x represents PyrE


<400>  48

Xaa Glu Phe Arg His Asp Ser Gly Tyr Glu Val His His
1               5                   10


<210>  49
<211>  14
<212>  PRT
<213>  homo sapiens

<220>
<221>  MISC_FEATURE
<222>  (1)..(1)
<223>  x represents PyrE


<400>  49

Xaa Glu Phe Arg His Asp Ser Gly Tyr Glu Val His His Gln
1               5                   10

<210>  50
<211>  15
<212>  PRT
<213>  homo sapiens

<220>
<221>  MISC_FEATURE
<222>  (1)..(1)
<223>  x represents PyrE

<400>  50

Xaa Glu Phe Arg His Asp Ser Gly Tyr Glu Val His His Gln Lys
1               5                   10                  15

<210>  51
<211>  16
<212>  PRT
<213>  homo sapiens

<220>
<221>  MISC_FEATURE
<222>  (1)..(1)
<223>  x represents PyrE

<400>  51

Xaa Glu Phe Arg His Asp Ser Gly Tyr Glu Val His His Gln Lys Leu
1               5                   10                  15

<210>  52
<211>  17
<212>  PRT
<213>  homo sapiens

<220>
<221>  MISC_FEATURE
<222>  (1)..(1)
<223>  x represents PyrE

<400>  52

Xaa Glu Phe Arg His Asp Ser Gly Tyr Glu Val His His Gln Lys Leu
1               5                   10                  15

Val

<210>  53

<211>  4
<212>  PRT
<213>  homo sapiens

<400>  53

Phe Arg His Asp
1


<210>  54
<211>  5
<212>  PRT
<213>  homo sapiens

<400>  54

Phe Arg His Asp Ser
1               5


<210>  55
<211>  6
<212>  PRT
<213>  homo sapiens

<400>  55

Phe Arg His Asp Ser Gly
1               5


<210>  56
<211>  7
<212>  PRT
<213>  homo sapiens

<400>  56

Phe Arg His Asp Ser Gly Tyr
1               5


<210>  57
<211>  8
<212>  PRT
<213>  homo sapiens

<400>  57

Phe Arg His Asp Ser Gly Tyr Glu
1               5


<210>  58
<211>  9
<212>  PRT
<213>  homo sapiens

<400>  58

Phe Arg His Asp Ser Gly Tyr Glu Val
1               5

<210>  59
<211>  10
<212>  PRT
<213>  homo sapiens

<400>  59

Phe Arg His Asp Ser Gly Tyr Glu Val His
1               5                   10

<210>  60
<211>  11
<212>  PRT
<213>  homo sapiens

<400>  60

Phe Arg His Asp Ser Gly Tyr Glu Val His His
1               5                   10

<210>  61
<211>  12
<212>  PRT
<213>  homo sapiens

<400>  61

Phe Arg His Asp Ser Gly Tyr Glu Val His His Gln
1               5                   10

<210>  62
<211>  13
<212>  PRT
<213>  homo sapiens

<400>  62

Phe Arg His Asp Ser Gly Tyr Glu Val His His Gln Lys
1               5                   10

<210>  63
<211>  14
<212>  PRT
<213>  homo sapiens

<400>  63

Phe Arg His Asp Ser Gly Tyr Glu Val His His Gln Lys Leu
1               5                   10

<210>  64
<211>  15

<212>  PRT
<213>  homo sapiens

<400>  64

Phe Arg His Asp Ser Gly Tyr Glu Val His His Gln Lys Leu Val
1               5                   10                  15


<210>  65
<211>  16
<212>  PRT
<213>  homo sapiens

<400>  65

Phe Arg His Asp Ser Gly Tyr Glu Val His His Gln Lys Leu Val Phe
1               5                   10                  15


<210>  66
<211>  4
<212>  PRT
<213>  homo sapiens

<400>  66

Arg His Asp Ser
1


<210>  67
<211>  5
<212>  PRT
<213>  homo sapiens

<400>  67

Arg His Asp Ser Gly
1               5


<210>  68
<211>  6
<212>  PRT
<213>  homo sapiens

<400>  68

Arg His Asp Ser Gly Tyr
1               5


<210>  69
<211>  7
<212>  PRT
<213>  homo sapiens

<400>  69

Arg His Asp Ser Gly Tyr Glu

1               5


<210>  70
<211>  8
<212>  PRT
<213>  homo sapiens

<400>  70

Arg His Asp Ser Gly Tyr Glu Val
1               5


<210>  71
<211>  9
<212>  PRT
<213>  homo sapiens

<400>  71

Arg His Asp Ser Gly Tyr Glu Val His
1               5


<210>  72
<211>  10
<212>  PRT
<213>  homo sapiens

<400>  72

Arg His Asp Ser Gly Tyr Glu Val His His
1               5                   10


<210>  73
<211>  11
<212>  PRT
<213>  homo sapiens

<400>  73

Arg His Asp Ser Gly Tyr Glu Val His His Gln
1               5                   10


<210>  74
<211>  12
<212>  PRT
<213>  homo sapiens

<400>  74

Arg His Asp Ser Gly Tyr Glu Val His His Gln Lys
1               5                   10


<210>  75
<211>  13
<212>  PRT

<213>  homo sapiens

<400>  75

Arg His Asp Ser Gly Tyr Glu Val His His Gln Lys Leu
1               5                   10


<210>  76
<211>  14
<212>  PRT
<213>  homo sapiens

<400>  76

Arg His Asp Ser Gly Tyr Glu Val His His Gln Lys Leu Val
1               5                   10


<210>  77
<211>  15
<212>  PRT
<213>  homo sapiens

<400>  77

Arg His Asp Ser Gly Tyr Glu Val His His Gln Lys Leu Val Phe
1               5                   10                  15


<210>  78
<211>  16
<212>  PRT
<213>  homo sapiens

<400>  78

Arg His Asp Ser Gly Tyr Glu Val His His Gln Lys Leu Val Phe Phe
1               5                   10                  15


<210>  79
<211>  4
<212>  PRT
<213>  homo sapiens

<400>  79

His Asp Ser Gly
1


<210>  80
<211>  5
<212>  PRT
<213>  homo sapiens

<400>  80

His Asp Ser Gly Tyr
1               5

<210>  81
<211>  6
<212>  PRT
<213>  homo sapiens

<400>  81

His Asp Ser Gly Tyr Glu
1               5


<210>  82
<211>  7
<212>  PRT
<213>  homo sapiens

<400>  82

His Asp Ser Gly Tyr Glu Val
1               5


<210>  83
<211>  8
<212>  PRT
<213>  homo sapiens

<400>  83

His Asp Ser Gly Tyr Glu Val His
1               5


<210>  84
<211>  9
<212>  PRT
<213>  homo sapiens

<400>  84

His Asp Ser Gly Tyr Glu Val His His
1               5


<210>  85
<211>  10
<212>  PRT
<213>  homo sapiens

<400>  85

His Asp Ser Gly Tyr Glu Val His His Gln
1               5                   10


<210>  86
<211>  11
<212>  PRT
<213>  homo sapiens

<400>  86

His Asp Ser Gly Tyr Glu Val His His Gln Lys
1               5                   10


<210>  87
<211>  12
<212>  PRT
<213>  homo sapiens

<400>  87

His Asp Ser Gly Tyr Glu Val His His Gln Lys Leu
1               5                   10


<210>  88
<211>  13
<212>  PRT
<213>  homo sapiens

<400>  88

His Asp Ser Gly Tyr Glu Val His His Gln Lys Leu Val
1               5                   10


<210>  89
<211>  14
<212>  PRT
<213>  homo sapiens

<400>  89

His Asp Ser Gly Tyr Glu Val His His Gln Lys Leu Val Phe
1               5                   10


<210>  90
<211>  15
<212>  PRT
<213>  homo sapiens

<400>  90

His Asp Ser Gly Tyr Glu Val His His Gln Lys Leu Val Phe Phe
1               5                   10                  15


<210>  91
<211>  16
<212>  PRT
<213>  homo sapiens

<400>  91

His Asp Ser Gly Tyr Glu Val His His Gln Lys Leu Val Phe Phe Ala
1               5                   10                  15

<210>  92
<211>  4
<212>  PRT
<213>  homo sapiens

<400>  92

Asp Ser Gly Tyr
1


<210>  93
<211>  5
<212>  PRT
<213>  homo sapiens

<400>  93

Asp Ser Gly Tyr Glu
1               5


<210>  94
<211>  6
<212>  PRT
<213>  homo sapiens

<400>  94

Asp Ser Gly Tyr Glu Val
1               5


<210>  95
<211>  7
<212>  PRT
<213>  homo sapiens

<400>  95

Asp Ser Gly Tyr Glu Val His
1               5


<210>  96
<211>  8
<212>  PRT
<213>  homo sapiens

<400>  96

Asp Ser Gly Tyr Glu Val His His
1               5


<210>  97
<211>  9
<212>  PRT
<213>  homo sapiens

<400>  97

Asp Ser Gly Tyr Glu Val His His Gln
1               5


<210>  98
<211>  10
<212>  PRT
<213>  homo sapiens

<400>  98

Asp Ser Gly Tyr Glu Val His His Gln Lys
1               5                   10


<210>  99
<211>  11
<212>  PRT
<213>  homo sapiens

<400>  99

Asp Ser Gly Tyr Glu Val His His Gln Lys Leu
1               5                   10


<210>  100
<211>  12
<212>  PRT
<213>  homo sapiens

<400>  100

Asp Ser Gly Tyr Glu Val His His Gln Lys Leu Val
1               5                   10


<210>  101
<211>  13
<212>  PRT
<213>  homo sapiens

<400>  101

Asp Ser Gly Tyr Glu Val His His Gln Lys Leu Val Phe
1               5                   10


<210>  102
<211>  14
<212>  PRT
<213>  homo sapiens

<400>  102

Asp Ser Gly Tyr Glu Val His His Gln Lys Leu Val Phe Phe
1               5                   10

<210>  103
<211>  15
<212>  PRT
<213>  homo sapiens

<400>  103

Asp Ser Gly Tyr Glu Val His His Gln Lys Leu Val Phe Phe Ala
1               5                   10                  15


<210>  104
<211>  16
<212>  PRT
<213>  homo sapiens

<400>  104

Asp Ser Gly Tyr Glu Val His His Gln Lys Leu Val Phe Phe Ala Glu
1               5                   10                  15


<210>  105
<211>  4
<212>  PRT
<213>  homo sapiens

<400>  105

Ser Gly Tyr Glu
1


<210>  106
<211>  5
<212>  PRT
<213>  homo sapiens

<400>  106

Ser Gly Tyr Glu Val
1               5


<210>  107
<211>  6
<212>  PRT
<213>  homo sapiens

<400>  107

Ser Gly Tyr Glu Val His
1               5


<210>  108
<211>  7
<212>  PRT
<213>  homo sapiens

<400>  108

Ser Gly Tyr Glu Val His His
1               5

<210>  109
<211>  8
<212>  PRT
<213>  homo sapiens

<400>  109

Ser Gly Tyr Glu Val His His Gln
1               5

<210>  110
<211>  9
<212>  PRT
<213>  homo sapiens

<400>  110

Ser Gly Tyr Glu Val His His Gln Lys
1               5

<210>  111
<211>  10
<212>  PRT
<213>  homo sapiens

<400>  111

Ser Gly Tyr Glu Val His His Gln Lys Leu
1               5                   10

<210>  112
<211>  11
<212>  PRT
<213>  homo sapiens

<400>  112

Ser Gly Tyr Glu Val His His Gln Lys Leu Val
1               5                   10

<210>  113
<211>  12
<212>  PRT
<213>  homo sapiens

<400>  113

Ser Gly Tyr Glu Val His His Gln Lys Leu Val Phe
1               5                   10

<210>  114

<211>  13
<212>  PRT
<213>  homo sapiens

<400>  114

Ser Gly Tyr Glu Val His His Gln Lys Leu Val Phe Phe
1               5                   10


<210>  115
<211>  14
<212>  PRT
<213>  homo sapiens

<400>  115

Ser Gly Tyr Glu Val His His Gln Lys Leu Val Phe Phe Ala
1               5                   10


<210>  116
<211>  15
<212>  PRT
<213>  homo sapiens

<400>  116

Ser Gly Tyr Glu Val His His Gln Lys Leu Val Phe Phe Ala Glu
1               5                   10                  15


<210>  117
<211>  16
<212>  PRT
<213>  homo sapiens

<400>  117

Ser Gly Tyr Glu Val His His Gln Lys Leu Val Phe Phe Ala Glu Asp
1               5                   10                  15


<210>  118
<211>  4
<212>  PRT
<213>  homo sapiens

<400>  118

Gly Tyr Glu Val
1


<210>  119
<211>  5
<212>  PRT
<213>  homo sapiens

<400>  119

Gly Tyr Glu Val His
1               5


<210>  120
<211>  6
<212>  PRT
<213>  homo sapiens

<400>  120

Gly Tyr Glu Val His His
1               5


<210>  121
<211>  7
<212>  PRT
<213>  homo sapiens

<400>  121

Gly Tyr Glu Val His His Gln
1               5


<210>  122
<211>  8
<212>  PRT
<213>  homo sapiens

<400>  122

Gly Tyr Glu Val His His Gln Lys
1               5


<210>  123
<211>  9
<212>  PRT
<213>  homo sapiens

<400>  123

Gly Tyr Glu Val His His Gln Lys Leu
1               5


<210>  124
<211>  10
<212>  PRT
<213>  homo sapiens

<400>  124

Gly Tyr Glu Val His His Gln Lys Leu Val
1               5                   10


<210>  125
<211>  11

<212>  PRT
<213>  homo sapiens

<400>  125

Gly Tyr Glu Val His His Gln Lys Leu Val Phe
1               5                   10


<210>  126
<211>  12
<212>  PRT
<213>  homo sapiens

<400>  126

Gly Tyr Glu Val His His Gln Lys Leu Val Phe Phe
1               5                   10


<210>  127
<211>  13
<212>  PRT
<213>  homo sapiens

<400>  127

Gly Tyr Glu Val His His Gln Lys Leu Val Phe Phe Ala
1               5                   10


<210>  128
<211>  14
<212>  PRT
<213>  homo sapiens

<400>  128

Gly Tyr Glu Val His His Gln Lys Leu Val Phe Phe Ala Glu
1               5                   10


<210>  129
<211>  15
<212>  PRT
<213>  homo sapiens

<400>  129

Gly Tyr Glu Val His His Gln Lys Leu Val Phe Phe Ala Glu Asp
1               5                   10                  15


<210>  130
<211>  16
<212>  PRT
<213>  homo sapiens

<400>  130

Gly Tyr Glu Val His His Gln Lys Leu Val Phe Phe Ala Glu Asp Val

1               5                   10                  15


<210>  131
<211>  4
<212>  PRT
<213>  homo sapiens

<400>  131

Tyr Glu Val His
1


<210>  132
<211>  5
<212>  PRT
<213>  homo sapiens

<400>  132

Tyr Glu Val His His
1               5


<210>  133
<211>  6
<212>  PRT
<213>  homo sapiens

<400>  133

Tyr Glu Val His His Gln
1               5


<210>  134
<211>  7
<212>  PRT
<213>  homo sapiens

<400>  134

Tyr Glu Val His His Gln Lys
1               5


<210>  135
<211>  8
<212>  PRT
<213>  homo sapiens

<400>  135

Tyr Glu Val His His Gln Lys Leu
1               5


<210>  136
<211>  9
<212>  PRT

<213>  homo sapiens

<400>  136

Tyr Glu Val His His Gln Lys Leu Val
1               5


<210>  137
<211>  10
<212>  PRT
<213>  homo sapiens

<400>  137

Tyr Glu Val His His Gln Lys Leu Val Phe
1               5                   10


<210>  138
<211>  11
<212>  PRT
<213>  homo sapiens

<400>  138

Tyr Glu Val His His Gln Lys Leu Val Phe Phe
1               5                   10


<210>  139
<211>  12
<212>  PRT
<213>  homo sapiens

<400>  139

Tyr Glu Val His His Gln Lys Leu Val Phe Phe Ala
1               5                   10


<210>  140
<211>  13
<212>  PRT
<213>  homo sapiens

<400>  140

Tyr Glu Val His His Gln Lys Leu Val Phe Phe Ala Glu
1               5                   10


<210>  141
<211>  14
<212>  PRT
<213>  homo sapiens

<400>  141

Tyr Glu Val His His Gln Lys Leu Val Phe Phe Ala Glu Asp
1               5                   10

<210>  142
<211>  15
<212>  PRT
<213>  homo sapiens

<400>  142

Tyr Glu Val His His Gln Lys Leu Val Phe Phe Ala Glu Asp Val
1               5                   10                  15


<210>  143
<211>  16
<212>  PRT
<213>  homo sapiens

<400>  143

Tyr Glu Val His His Gln Lys Leu Val Phe Phe Ala Glu Asp Val Gly
1               5                   10                  15


<210>  144
<211>  41
<212>  PRT
<213>  homo sapiens

<400>  144

Asp Ala Glu Phe Arg His Asp Ser Gly Tyr Glu Val His His Gln Leu
1               5                   10                  15


Val Phe Phe Ala Glu Asp Val Gly Ser Asn Lys Gly Ala Ile Ile Gly
            20                  25                  30


Leu Met Val Gly Gly Val Val Ile Ala
        35                  40


<210>  145
<211>  40
<212>  PRT
<213>  homo sapiens

<400>  145

Ala Glu Phe Arg His Asp Ser Gly Tyr Glu Val His His Gln Leu Val
1               5                   10                  15


Phe Phe Ala Glu Asp Val Gly Ser Asn Lys Gly Ala Ile Ile Gly Leu
            20                  25                  30


Met Val Gly Gly Val Val Ile Ala
        35                  40

<210>  146
<211>  39
<212>  PRT
<213>  homo sapiens

<400>  146

Glu Phe Arg His Asp Ser Gly Tyr Glu Val His His Gln Leu Val Phe
1               5                   10                  15

Phe Ala Glu Asp Val Gly Ser Asn Lys Gly Ala Ile Ile Gly Leu Met
            20                  25                  30

Val Gly Gly Val Val Ile Ala
        35

<210>  147
<211>  39
<212>  PRT
<213>  homo sapiens

<220>
<221>  MISC_FEATURE
<222>  (1)..(1)
<223>  x represents PyrE

<400>  147

Xaa Phe Arg His Asp Ser Gly Tyr Glu Val His His Gln Leu Val Phe
1               5                   10                  15

Phe Ala Glu Asp Val Gly Ser Asn Lys Gly Ala Ile Ile Gly Leu Met
            20                  25                  30

Val Gly Gly Val Val Ile Ala
        35

<210>  148
<211>  38
<212>  PRT
<213>  homo sapiens

<400>  148

Phe Arg His Asp Ser Gly Tyr Glu Val His His Gln Leu Val Phe Phe
1               5                   10                  15

Ala Glu Asp Val Gly Ser Asn Lys Gly Ala Ile Ile Gly Leu Met Val
            20                  25                  30

Gly Gly Val Val Ile Ala

        35

<210>  149
<211>  37
<212>  PRT
<213>  homo sapiens

<400>  149

Arg His Asp Ser Gly Tyr Glu Val His His Gln Leu Val Phe Phe Ala
1               5                   10                  15

Glu Asp Val Gly Ser Asn Lys Gly Ala Ile Ile Gly Leu Met Val Gly
            20                  25                  30

Gly Val Val Ile Ala
        35

<210>  150
<211>  36
<212>  PRT
<213>  homo sapiens

<400>  150

His Asp Ser Gly Tyr Glu Val His His Gln Leu Val Phe Phe Ala Glu
1               5                   10                  15

Asp Val Gly Ser Asn Lys Gly Ala Ile Ile Gly Leu Met Val Gly Gly
            20                  25                  30

Val Val Ile Ala
        35

<210>  151
<211>  35
<212>  PRT
<213>  homo sapiens

<400>  151

Asp Ser Gly Tyr Glu Val His His Gln Leu Val Phe Phe Ala Glu Asp
1               5                   10                  15

Val Gly Ser Asn Lys Gly Ala Ile Ile Gly Leu Met Val Gly Gly Val
            20                  25                  30

Val Ile Ala
        35

<210>  152

<211>  34
<212>  PRT
<213>  homo sapiens

<400>  152

Ser Gly Tyr Glu Val His His Gln Leu Val Phe Phe Ala Glu Asp Val
1               5                   10                  15

Gly Ser Asn Lys Gly Ala Ile Ile Gly Leu Met Val Gly Gly Val Val
            20                  25                  30

Ile Ala

<210>  153
<211>  33
<212>  PRT
<213>  homo sapiens

<400>  153

Gly Tyr Glu Val His His Gln Leu Val Phe Phe Ala Glu Asp Val Gly
1               5                   10                  15

Ser Asn Lys Gly Ala Ile Ile Gly Leu Met Val Gly Gly Val Val Ile
            20                  25                  30

Ala

<210>  154
<211>  32
<212>  PRT
<213>  homo sapiens

<400>  154

Tyr Glu Val His His Gln Leu Val Phe Phe Ala Glu Asp Val Gly Ser
1               5                   10                  15

Asn Lys Gly Ala Ile Ile Gly Leu Met Val Gly Gly Val Val Ile Ala
            20                  25                  30

<210>  155
<211>  39
<212>  PRT
<213>  homo sapiens

<400>  155

Asp Ala Glu Phe Arg His Asp Ser Gly Tyr Glu Val His His Gln Leu
1               5                   10                  15

Val Phe Phe Ala Glu Asp Val Gly Ser Asn Lys Gly Ala Ile Ile Gly
            20                  25                  30

Leu Met Val Gly Gly Val Val
        35

<210>  156
<211>  38
<212>  PRT
<213>  homo sapiens

<400>  156

Ala Glu Phe Arg His Asp Ser Gly Tyr Glu Val His His Gln Leu Val
1               5                   10                  15

Phe Phe Ala Glu Asp Val Gly Ser Asn Lys Gly Ala Ile Ile Gly Leu
            20                  25                  30

Met Val Gly Gly Val Val
        35

<210>  157
<211>  37
<212>  PRT
<213>  homo sapiens

<400>  157

Glu Phe Arg His Asp Ser Gly Tyr Glu Val His His Gln Leu Val Phe
1               5                   10                  15

Phe Ala Glu Asp Val Gly Ser Asn Lys Gly Ala Ile Ile Gly Leu Met
            20                  25                  30

Val Gly Gly Val Val
        35

<210>  158
<211>  37
<212>  PRT
<213>  homo sapiens

<220>
<221>  MISC_FEATURE
<222>  (1)..(1)
<223>  x represents PyrE

<400>  158

Xaa Phe Arg His Asp Ser Gly Tyr Glu Val His His Gln Leu Val Phe
1               5                   10                  15

Phe Ala Glu Asp Val Gly Ser Asn Lys Gly Ala Ile Ile Gly Leu Met
            20                  25                  30

Val Gly Gly Val Val
        35

<210>  159
<211>  36
<212>  PRT
<213>  homo sapiens

<400>  159

Phe Arg His Asp Ser Gly Tyr Glu Val His His Gln Leu Val Phe Phe
1               5                   10                  15

Ala Glu Asp Val Gly Ser Asn Lys Gly Ala Ile Ile Gly Leu Met Val
            20                  25                  30

Gly Gly Val Val
        35

<210>  160
<211>  35
<212>  PRT
<213>  homo sapiens

<400>  160

Arg His Asp Ser Gly Tyr Glu Val His His Gln Leu Val Phe Phe Ala
1               5                   10                  15

Glu Asp Val Gly Ser Asn Lys Gly Ala Ile Ile Gly Leu Met Val Gly
            20                  25                  30

Gly Val Val
        35

<210>  161
<211>  34
<212>  PRT
<213>  homo sapiens

<400>  161

His Asp Ser Gly Tyr Glu Val His His Gln Leu Val Phe Phe Ala Glu
1               5                   10                  15

Asp Val Gly Ser Asn Lys Gly Ala Ile Ile Gly Leu Met Val Gly Gly
            20                  25                  30

Val Val

<210>  162
<211>  33
<212>  PRT
<213>  homo sapiens

<400>  162

Asp Ser Gly Tyr Glu Val His His Gln Leu Val Phe Phe Ala Glu Asp
1               5                   10                  15

Val Gly Ser Asn Lys Gly Ala Ile Ile Gly Leu Met Val Gly Gly Val
            20                  25                  30

Val

<210>  163
<211>  32
<212>  PRT
<213>  homo sapiens

<400>  163

Ser Gly Tyr Glu Val His His Gln Leu Val Phe Phe Ala Glu Asp Val
1               5                   10                  15

Gly Ser Asn Lys Gly Ala Ile Ile Gly Leu Met Val Gly Gly Val Val
            20                  25                  30

<210>  164
<211>  31
<212>  PRT
<213>  homo sapiens

<400>  164

Gly Tyr Glu Val His His Gln Leu Val Phe Phe Ala Glu Asp Val Gly
1               5                   10                  15

Ser Asn Lys Gly Ala Ile Ile Gly Leu Met Val Gly Gly Val Val
            20                  25                  30

<210>  165
<211>  30
<212>  PRT
<213>  homo sapiens

<400>  165

Tyr Glu Val His His Gln Leu Val Phe Phe Ala Glu Asp Val Gly Ser
1               5                   10                  15

Asn Lys Gly Ala Ile Ile Gly Leu Met Val Gly Gly Val Val
            20                  25                  30

<210>  166
<211>  4
<212>  PRT
<213>  homo sapiens

<400>  166

Val Lys Met Asp
1

<210>  167
<211>  5
<212>  PRT
<213>  homo sapiens

<400>  167

Glu Val Lys Met Asp
1               5

<210>  168
<211>  6
<212>  PRT
<213>  homo sapiens

<400>  168

Ser Glu Val Lys Met Asp
1               5

<210>  169
<211>  7
<212>  PRT
<213>  homo sapiens

<400>  169

Ile Ser Glu Val Lys Met Asp
1               5

<210>  170
<211>  8
<212>  PRT
<213>  homo sapiens

<400>  170

Glu Ile Ser Glu Val Lys Met Asp
1               5

<210>  171
<211>  9
<212>  PRT
<213>  homo sapiens

<400>  171

Glu Glu Ile Ser Glu Val Lys Met Asp
1               5

<210>  172
<211>  10
<212>  PRT
<213>  homo sapiens

<400>  172

Thr Glu Glu Ile Ser Glu Val Lys Met Asp
1               5                   10

<210>  173
<211>  11
<212>  PRT
<213>  homo sapiens

<400>  173

Lys Thr Glu Glu Ile Ser Glu Val Lys Met Asp
1               5                   10

<210>  174
<211>  12
<212>  PRT
<213>  homo sapiens

<400>  174

Ile Lys Thr Glu Glu Ile Ser Glu Val Lys Met Asp
1               5                   10

<210>  175
<211>  13
<212>  PRT
<213>  homo sapiens

<400>  175

Asn Ile Lys Thr Glu Glu Ile Ser Glu Val Lys Met Asp
1               5                   10

<210>  176

<211>  14
<212>  PRT
<213>  homo sapiens

<400>  176

Thr Asn Ile Lys Thr Glu Glu Ile Ser Glu Val Lys Met Asp
1               5                   10


<210>  177
<211>  15
<212>  PRT
<213>  homo sapiens

<400>  177

Leu Thr Asn Ile Lys Thr Glu Glu Ile Ser Glu Val Lys Met Asp
1               5                   10                  15


<210>  178
<211>  16
<212>  PRT
<213>  homo sapiens

<400>  178

Gly Leu Thr Asn Ile Lys Thr Glu Glu Ile Ser Glu Val Lys Met Asp
1               5                   10                  15


<210>  179
<211>  4
<212>  PRT
<213>  homo sapiens

<400>  179

Lys Met Asp Ala
1


<210>  180
<211>  5
<212>  PRT
<213>  homo sapiens

<400>  180

Val Lys Met Asp Ala
1               5


<210>  181
<211>  6
<212>  PRT
<213>  homo sapiens

<400>  181

Glu Val Lys Met Asp Ala
1               5


<210>  182
<211>  7
<212>  PRT
<213>  homo sapiens

<400>  182

Ser Glu Val Lys Met Asp Ala
1               5


<210>  183
<211>  8
<212>  PRT
<213>  homo sapiens

<400>  183

Ile Ser Glu Val Lys Met Asp Ala
1               5


<210>  184
<211>  9
<212>  PRT
<213>  homo sapiens

<400>  184

Glu Ile Ser Glu Val Lys Met Asp Ala
1               5


<210>  185
<211>  10
<212>  PRT
<213>  homo sapiens

<400>  185

Glu Glu Ile Ser Glu Val Lys Met Asp Ala
1               5                   10


<210>  186
<211>  11
<212>  PRT
<213>  homo sapiens

<400>  186

Thr Glu Glu Ile Ser Glu Val Lys Met Asp Ala
1               5                   10


<210>  187
<211>  12

<212>  PRT
<213>  homo sapiens

<400>  187

Lys Thr Glu Glu Ile Ser Glu Val Lys Met Asp Ala
1               5                   10


<210>  188
<211>  13
<212>  PRT
<213>  homo sapiens

<400>  188

Ile Lys Thr Glu Glu Ile Ser Glu Val Lys Met Asp Ala
1               5                   10


<210>  189
<211>  14
<212>  PRT
<213>  homo sapiens

<400>  189

Asn Ile Lys Thr Glu Glu Ile Ser Glu Val Lys Met Asp Ala
1               5                   10


<210>  190
<211>  15
<212>  PRT
<213>  homo sapiens

<400>  190

Thr Asn Ile Lys Thr Glu Glu Ile Ser Glu Val Lys Met Asp Ala
1               5                   10                  15


<210>  191
<211>  16
<212>  PRT
<213>  homo sapiens

<400>  191

Leu Thr Asn Ile Lys Thr Glu Glu Ile Ser Glu Val Lys Met Asp Ala
1               5                   10                  15


<210>  192
<211>  4
<212>  PRT
<213>  homo sapiens

<400>  192

Met Asp Ala Glu

1

<210>  193
<211>  5
<212>  PRT
<213>  homo sapiens

<400>  193

Lys Met Asp Ala Glu
1               5

<210>  194
<211>  6
<212>  PRT
<213>  homo sapiens

<400>  194

Val Lys Met Asp Ala Glu
1               5

<210>  195
<211>  7
<212>  PRT
<213>  homo sapiens

<400>  195

Glu Val Lys Met Asp Ala Glu
1               5

<210>  196
<211>  8
<212>  PRT
<213>  homo sapiens

<400>  196

Ser Glu Val Lys Met Asp Ala Glu
1               5

<210>  197
<211>  9
<212>  PRT
<213>  homo sapiens

<400>  197

Ile Ser Glu Val Lys Met Asp Ala Glu
1               5

<210>  198
<211>  10
<212>  PRT

<213>  homo sapiens

<400>  198

Glu Ile Ser Glu Val Lys Met Asp Ala Glu
1               5                   10


<210>  199
<211>  11
<212>  PRT
<213>  homo sapiens

<400>  199

Glu Glu Ile Ser Glu Val Lys Met Asp Ala Glu
1               5                   10


<210>  200
<211>  12
<212>  PRT
<213>  homo sapiens

<400>  200

Thr Glu Glu Ile Ser Glu Val Lys Met Asp Ala Glu
1               5                   10


<210>  201
<211>  13
<212>  PRT
<213>  homo sapiens

<400>  201

Lys Thr Glu Glu Ile Ser Glu Val Lys Met Asp Ala Glu
1               5                   10


<210>  202
<211>  14
<212>  PRT
<213>  homo sapiens

<400>  202

Ile Lys Thr Glu Glu Ile Ser Glu Val Lys Met Asp Ala Glu
1               5                   10


<210>  203
<211>  15
<212>  PRT
<213>  homo sapiens

<400>  203

Asn Ile Lys Thr Glu Glu Ile Ser Glu Val Lys Met Asp Ala Glu
1               5                   10                  15

<210>  204
<211>  16
<212>  PRT
<213>  homo sapiens

<400>  204

Thr Asn Ile Lys Thr Glu Glu Ile Ser Glu Val Lys Met Asp Ala Glu
1               5                   10                  15


<210>  205
<211>  5
<212>  PRT
<213>  homo sapiens

<400>  205

Met Asp Ala Glu Phe
1               5


<210>  206
<211>  6
<212>  PRT
<213>  homo sapiens

<400>  206

Lys Met Asp Ala Glu Phe
1               5


<210>  207
<211>  7
<212>  PRT
<213>  homo sapiens

<400>  207

Val Lys Met Asp Ala Glu Phe
1               5


<210>  208
<211>  8
<212>  PRT
<213>  homo sapiens

<400>  208

Glu Val Lys Met Asp Ala Glu Phe
1               5


<210>  209
<211>  9
<212>  PRT
<213>  homo sapiens

<400>  209

Ser Glu Val Lys Met Asp Ala Glu Phe
1               5


<210>  210
<211>  10
<212>  PRT
<213>  homo sapiens

<400>  210

Ile Ser Glu Val Lys Met Asp Ala Glu Phe
1               5                   10


<210>  211
<211>  11
<212>  PRT
<213>  homo sapiens

<400>  211

Glu Ile Ser Glu Val Lys Met Asp Ala Glu Phe
1               5                   10


<210>  212
<211>  12
<212>  PRT
<213>  homo sapiens

<400>  212

Glu Glu Ile Ser Glu Val Lys Met Asp Ala Glu Phe
1               5                   10


<210>  213
<211>  13
<212>  PRT
<213>  homo sapiens

<400>  213

Thr Glu Glu Ile Ser Glu Val Lys Met Asp Ala Glu Phe
1               5                   10


<210>  214
<211>  14
<212>  PRT
<213>  homo sapiens

<400>  214

Lys Thr Glu Glu Ile Ser Glu Val Lys Met Asp Ala Glu Phe
1               5                   10

<210>  215
<211>  15
<212>  PRT
<213>  homo sapiens

<400>  215

Ile Lys Thr Glu Glu Ile Ser Glu Val Lys Met Asp Ala Glu Phe
1               5                   10                  15


<210>  216
<211>  16
<212>  PRT
<213>  homo sapiens

<400>  216

Asn Ile Lys Thr Glu Glu Ile Ser Glu Val Lys Met Asp Ala Glu Phe
1               5                   10                  15


<210>  217
<211>  6
<212>  PRT
<213>  homo sapiens

<400>  217

Met Asp Ala Glu Phe Arg
1               5


<210>  218
<211>  7
<212>  PRT
<213>  homo sapiens

<400>  218

Lys Met Asp Ala Glu Phe Arg
1               5


<210>  219
<211>  8
<212>  PRT
<213>  homo sapiens

<400>  219

Val Lys Met Asp Ala Glu Phe Arg
1               5


<210>  220
<211>  9
<212>  PRT
<213>  homo sapiens

<400>  220

Glu Val Lys Met Asp Ala Glu Phe Arg
1               5


<210>  221
<211>  10
<212>  PRT
<213>  homo sapiens

<400>  221

Ser Glu Val Lys Met Asp Ala Glu Phe Arg
1               5                   10


<210>  222
<211>  11
<212>  PRT
<213>  homo sapiens

<400>  222

Ile Ser Glu Val Lys Met Asp Ala Glu Phe Arg
1               5                   10


<210>  223
<211>  12
<212>  PRT
<213>  homo sapiens

<400>  223

Glu Ile Ser Glu Val Lys Met Asp Ala Glu Phe Arg
1               5                   10


<210>  224
<211>  13
<212>  PRT
<213>  homo sapiens

<400>  224

Glu Glu Ile Ser Glu Val Lys Met Asp Ala Glu Phe Arg
1               5                   10


<210>  225
<211>  14
<212>  PRT
<213>  homo sapiens

<400>  225

Thr Glu Glu Ile Ser Glu Val Lys Met Asp Ala Glu Phe Arg
1               5                   10

<210>  226
<211>  15
<212>  PRT
<213>  homo sapiens

<400>  226

Lys Thr Glu Glu Ile Ser Glu Val Lys Met Asp Ala Glu Phe Arg
1               5                   10                  15


<210>  227
<211>  16
<212>  PRT
<213>  homo sapiens

<400>  227

Ile Lys Thr Glu Glu Ile Ser Glu Val Lys Met Asp Ala Glu Phe Arg
1               5                   10                  15


<210>  228
<211>  7
<212>  PRT
<213>  homo sapiens

<400>  228

Met Asp Ala Glu Phe Arg His
1               5


<210>  229
<211>  8
<212>  PRT
<213>  homo sapiens

<400>  229

Lys Met Asp Ala Glu Phe Arg His
1               5


<210>  230
<211>  9
<212>  PRT
<213>  homo sapiens

<400>  230

Val Lys Met Asp Ala Glu Phe Arg His
1               5


<210>  231
<211>  10
<212>  PRT
<213>  homo sapiens

<400>  231

Glu Val Lys Met Asp Ala Glu Phe Arg His
1               5                   10


<210>  232
<211>  11
<212>  PRT
<213>  homo sapiens

<400>  232

Ser Glu Val Lys Met Asp Ala Glu Phe Arg His
1               5                   10


<210>  233
<211>  12
<212>  PRT
<213>  homo sapiens

<400>  233

Ile Ser Glu Val Lys Met Asp Ala Glu Phe Arg His
1               5                   10


<210>  234
<211>  13
<212>  PRT
<213>  homo sapiens

<400>  234

Glu Ile Ser Glu Val Lys Met Asp Ala Glu Phe Arg His
1               5                   10


<210>  235
<211>  14
<212>  PRT
<213>  homo sapiens

<400>  235

Glu Glu Ile Ser Glu Val Lys Met Asp Ala Glu Phe Arg His
1               5                   10


<210>  236
<211>  15
<212>  PRT
<213>  homo sapiens

<400>  236

Thr Glu Glu Ile Ser Glu Val Lys Met Asp Ala Glu Phe Arg His
1               5                   10                  15


<210>  237

<211>  16
<212>  PRT
<213>  homo sapiens

<400>  237

Lys Thr Glu Glu Ile Ser Glu Val Lys Met Asp Ala Glu Phe Arg His
1               5                   10                  15


<210>  238
<211>  8
<212>  PRT
<213>  homo sapiens

<400>  238

Met Asp Ala Glu Phe Arg His Asp
1               5


<210>  239
<211>  9
<212>  PRT
<213>  homo sapiens

<400>  239

Lys Met Asp Ala Glu Phe Arg His Asp
1               5


<210>  240
<211>  10
<212>  PRT
<213>  homo sapiens

<400>  240

Val Lys Met Asp Ala Glu Phe Arg His Asp
1               5                   10


<210>  241
<211>  11
<212>  PRT
<213>  homo sapiens

<400>  241

Glu Val Lys Met Asp Ala Glu Phe Arg His Asp
1               5                   10


<210>  242
<211>  12
<212>  PRT
<213>  homo sapiens

<400>  242

Ser Glu Val Lys Met Asp Ala Glu Phe Arg His Asp
1               5                   10


<210>  243
<211>  13
<212>  PRT
<213>  homo sapiens

<400>  243

Ile Ser Glu Val Lys Met Asp Ala Glu Phe Arg His Asp
1               5                   10


<210>  244
<211>  14
<212>  PRT
<213>  homo sapiens

<400>  244

Glu Ile Ser Glu Val Lys Met Asp Ala Glu Phe Arg His Asp
1               5                   10


<210>  245
<211>  15
<212>  PRT
<213>  homo sapiens

<400>  245

Glu Glu Ile Ser Glu Val Lys Met Asp Ala Glu Phe Arg His Asp
1               5                   10                  15


<210>  246
<211>  16
<212>  PRT
<213>  homo sapiens

<400>  246

Thr Glu Glu Ile Ser Glu Val Lys Met Asp Ala Glu Phe Arg His Asp
1               5                   10                  15


<210>  247
<211>  9
<212>  PRT
<213>  homo sapiens

<400>  247

Met Asp Ala Glu Phe Arg His Asp Ser
1               5


<210>  248
<211>  10

<212>  PRT
<213>  homo sapiens

<400>  248

Lys Met Asp Ala Glu Phe Arg His Asp Ser
1               5                   10


<210>  249
<211>  11
<212>  PRT
<213>  homo sapiens

<400>  249

Val Lys Met Asp Ala Glu Phe Arg His Asp Ser
1               5                   10


<210>  250
<211>  12
<212>  PRT
<213>  homo sapiens

<400>  250

Glu Val Lys Met Asp Ala Glu Phe Arg His Asp Ser
1               5                   10


<210>  251
<211>  13
<212>  PRT
<213>  homo sapiens

<400>  251

Ser Glu Val Lys Met Asp Ala Glu Phe Arg His Asp Ser
1               5                   10


<210>  252
<211>  14
<212>  PRT
<213>  homo sapiens

<400>  252

Ile Ser Glu Val Lys Met Asp Ala Glu Phe Arg His Asp Ser
1               5                   10


<210>  253
<211>  15
<212>  PRT
<213>  homo sapiens

<400>  253

Glu Ile Ser Glu Val Lys Met Asp Ala Glu Phe Arg His Asp Ser

1               5                   10                  15


<210>  254
<211>  16
<212>  PRT
<213>  homo sapiens

<400>  254

Glu Glu Ile Ser Glu Val Lys Met Asp Ala Glu Phe Arg His Asp Ser
1               5                   10                  15


<210>  255
<211>  10
<212>  PRT
<213>  homo sapiens

<400>  255

Met Asp Ala Glu Phe Arg His Asp Ser Gly
1               5                   10


<210>  256
<211>  11
<212>  PRT
<213>  homo sapiens

<400>  256

Lys Met Asp Ala Glu Phe Arg His Asp Ser Gly
1               5                   10


<210>  257
<211>  12
<212>  PRT
<213>  homo sapiens

<400>  257

Val Lys Met Asp Ala Glu Phe Arg His Asp Ser Gly
1               5                   10


<210>  258
<211>  13
<212>  PRT
<213>  Homo sapiens

<400>  258

Glu Val Lys Met Asp Ala Glu Phe Arg His Asp Ser Gly
1               5                   10


<210>  259
<211>  14
<212>  PRT

<213>  Homo sapiens

<400>  259

Ser Glu Val Lys Met Asp Ala Glu Phe Arg His Asp Ser Gly
1               5                   10


<210>  260
<211>  15
<212>  PRT
<213>  Homo sapiens

<400>  260

Ile Ser Glu Val Lys Met Asp Ala Glu Phe Arg His Asp Ser Gly
1               5                   10                  15


<210>  261
<211>  16
<212>  PRT
<213>  Homo sapiens

<400>  261

Glu Ile Ser Glu Val Lys Met Asp Ala Glu Phe Arg His Asp Ser Gly
1               5                   10                  15